# EUROPÄISCHE PATENTSCHRIFT

(11) **EP 2 061 881 B1**
(45) Veröffentlichungstag und Bekanntmachung des Hinweises auf die Patenterteilung: **09.09.2015**
(21) Anmeldenummer: 07821334.5
(22) Anmeldetag: 15.10.2007
(51) Int. Cl.: C12N 9/12, C12N 15/54, C12P 13/04, C12P 13/08, C12R 1/15, C12N 15/63, C12N 15/69, C12N 1/21, A23L 1/227

(54) **ALLELE DES REL-GENS AUS CORYNEFORMEN BAKTERIEN**
ALLELES OF THE REL GENE OF CORYNEFORM BACTERIA
ALLÈLES DU GÈNE REL DE BACTÉRIES CORYNÉFORMES

(30) Priorität: 17.10.2006 DE 102006048882
(43) Veröffentlichungstag der Anmeldung: 27.05.2009
(73) Patentinhaber: Evonik Degussa GmbH, 45128 Essen (DE)
(72) Erfinder: BATHE, Brigitte, 33154 Salzkotten (DE); KREUTZER, Caroline, 33813 Oerlinghausen (DE); THIERBACH, Georg, 33613 Bielefeld (DE)
(86) Internationale Anmeldenummer: PCT/EP2007/060967
(87) Internationale Veröffentlichungsnummer: WO 2008/046810

(56) Entgegenhaltungen:
- EP-A- 1 486 570
- WO-A-02/07183
- WO-A2-02/077183
- DATABASE UniProt [Online] 2. August 2005 (2005-08-02), "GTP pyrophosphokinase (EC <A HREF="http://srs.ebi.ac.uk/srsbin/cgi-bin/ wgetz?[enzyme-ECNumber:2.7.6.5]+-e">2.7.6. 5</A>)." XP002476693 gefunden im EBI accession no. UNIPROT:Q4JVE5 Database accession no. Q4JVE5 & TAUCH ANDREAS ET AL: "Complete genome sequence and analysis of the multiresistant nosocomial pathogen Corynebacterium jeikeium K411, a lipid-requiring bacterium of the human skin flora." JOURNAL OF BACTERIOLOGY JUL 2005, Bd. 187, Nr. 13, Juli 2005 (2005-07), Seiten 4671-4682, ISSN: 0021-9193
- WENDRICH T M ET AL: "Cloning and characterization of a relA/spoT homologue from Bacillus subtilis." MOLECULAR MICROBIOLOGY OCT 1997, Bd. 26, Nr. 1, Oktober 1997 (1997-10), Seiten 65-79, XP000918432 ISSN: 0950-382X
- WEHMEIER L ET AL: "The role of the Corynebacterium glutamicum rel gene in (p)ppGpp metabolism." MICROBIOLOGY (READING, ENGLAND) JUL 1998, Bd. 144 ( Pt 7), Juli 1998 (1998-07), Seiten 1853-1862, XP002174033 ISSN: 1350-0872

### SEQUENCE LISTING

<110> Evonik Degussa GmbH
<120> Allele des rel-Gens aus coryneformen Bakterien
<130> 200600725
<160> 22
<170> PatentIn version 3.3
<210> 1
   <211> 2283
   <212> DNA
   <213> Corynebacterium glutamicum
<220>
   <221> CDS
   <222> (1)..(2280)
   <223> rel-Wildtyp-Gen
<220>
   <221> misc_feature
   <222> (785)..(785)
   <223> Guanin
<400> 1
   atg agt ctg gag cgc aac aca caa aaa tct tcc atg ggt gtg cga agc       48
   Met Ser Leu Glu Arg Asn Thr Gln Lys Ser Ser Met Gly Val Arg Ser
   1               5                   10                  15
   atg tca gcc agg ctt gcc cgc agc ctc aca gga aac cgc gtt cgc acc       96
   Met Ser Ala Arg Leu Ala Arg Ser Leu Thr Gly Asn Arg Val Arg Thr
               20                  25                  30
   aac cct gtg ctg gat ccg ctg ctg agc atc cac cgg caa ttt cac cca      144
   Asn Pro Val Leu Asp Pro Leu Leu Ser Ile His Arg Gln Phe His Pro
           35                  40                  45
   cgc gcc gac gta caa gtg ttg gaa cgt gca tat gac acc gcg gaa cgt      192
   Arg Ala Asp Val Gln Val Leu Glu Arg Ala Tyr Asp Thr Ala Glu Arg
       50                  55                  60
   ctt cat gat ggt gtg att cga aaa tcg ggc gat ccg tat att acc cac      240
   Leu His Asp Gly Val Ile Arg Lys Ser Gly Asp Pro Tyr Ile Thr His
   65                  70                  75                  80
   ccg ttg gct gtc gcc acc atc gcc gcg gaa atc ggc atg gac acc acc      288
   Pro Leu Ala Val Ala Thr Ile Ala Ala Glu Ile Gly Met Asp Thr Thr
                   85                  90                  95
   acg ctc gtc gca gcc ttg ttg cat gac acg gtg gaa gac acc gac tac      336
   Thr Leu Val Ala Ala Leu Leu His Asp Thr Val Glu Asp Thr Asp Tyr
               100                 105                 110
   tct ttg gac gat ctc acc cga gat ttc gga gaa gaa gtt gcc agg ctt      384
   Ser Leu Asp Asp Leu Thr Arg Asp Phe Gly Glu Glu Val Ala Arg Leu
           115                 120                 125
   gtc gac ggt gtc acc aag ctc gac aaa gtc gca cta ggt gct gcc gcg      432
   Val Asp Gly Val Thr Lys Leu Asp Lys Val Ala Leu Gly Ala Ala Ala
   130 135 140
   gag gcc gaa acg att cgc aaa atg atc gtc gcc atg agc cag gac ccc 480
   Glu Ala Glu Thr Ile Arg Lys Met Ile Val Ala Met Ser Gln Asp Pro
   145 150 155 160
   cgc gtg ctg gtg att aaa gtg gcc gac cgt ttg cac aat atg cgc acc 528
   Arg Val Leu Val Ile Lys Val Ala Asp Arg Leu His Asn Met Arg Thr
   165 170 175
   atg cgg ttc ctg ccg ccg gaa aag caa gct aaa aaa gca cgc caa acc 576
   Met Arg Phe Leu Pro Pro Glu Lys Gln Ala Lys Lys Ala Arg Gln Thr
   180 185 190
   ctt gaa gtg att gct cct ttg gca cac cgc ctg ggc atg gcc agc gtg 624
   Leu Glu Val Ile Ala Pro Leu Ala His Arg Leu Gly Met Ala Ser Val
   195 200 205
   aaa tgg gaa ttg gaa gat cta tcc ttt gcc att ttg tac ccc aag aag 672
   Lys Trp Glu Leu Glu Asp Leu Ser Phe Ala Ile Leu Tyr Pro Lys Lys
   210 215 220
   tac gaa gag atc gtg cgt ctt gtt gcc gac cgc gcg ccc tct aga gac 720
   Tyr Glu Glu Ile Val Arg Leu Val Ala Asp Arg Ala Pro Ser Arg Asp
   225 230 235 240
   cgg tac ctc aaa gaa att att gat caa gtc acc ggt ggc ttg cgc gaa 768
   Arg Tyr Leu Lys Glu Ile Ile Asp Gln Val Thr Gly Gly Leu Arg Glu
   245 250 255
   aac aac atc gcg gca gga gtg ctt ggt cga cca aag cac tac tgg tct 816
   Asn Asn Ile Ala Ala Gly Val Leu Gly Arg Pro Lys His Tyr Trp Ser
   260 265 270
   atc tat caa aag atg atc gtt cgc ggt cgt gat ttt gac gat att ttt 864
   Ile Tyr Gln Lys Met Ile Val Arg Gly Arg Asp Phe Asp Asp Ile Phe
   275 280 285
   gat ctt gtt ggc atc cgc atc ctg gta gac aac gtg aac aac tgt tac 912
   Asp Leu Val Gly Ile Arg Ile Leu Val Asp Asn Val Asn Asn Cys Tyr
   290 295 300
   gcc gcc atc ggt gtc gtg cac tcc ctg ttc aat gct ctg cct ggc cga 960
   Ala Ala Ile Gly Val Val His Ser Leu Phe Asn Ala Leu Pro Gly Arg
   305 310 315 320
   ttc aaa gac tat att tca gcc ccg cgc ttc ggt gtc tac caa tcc ctg 1008
   Phe Lys Asp Tyr Ile Ser Ala Pro Arg Phe Gly Val Tyr Gln Ser Leu
   325 330 335
   cac acc acc gtg atg gga cct ggc ggt aag cct ctg gaa gtt cag gca 1056
   His Thr Thr Val Met Gly Pro Gly Gly Lys Pro Leu Glu Val Gln Ala
   340 345 350
   cgt acc cac gac atg cac tac aac gcc gaa ttc ggc att gca gcg cac 1104
   Arg Thr His Asp Met His Tyr Asn Ala Glu Phe Gly Ile Ala Ala His
   355 360 365
   tgg cga tac aaa gaa acc aaa ggc agc cac agt ggc gag caa gcc gaa 1152
   Trp Arg Tyr Lys Glu Thr Lys Gly Ser His Ser Gly Glu Gln Ala Glu
   370 375 380
   gtg gat caa atg gcg tgg atg cgc caa ctt ctg gac tgg caa aaa gaa      1200
   Val Asp Gln Met Ala Trp Met Arg Gln Leu Leu Asp Trp Gln Lys Glu
   385                 390                 395                 400
   gca gcc gac ccc aac gag ttc ctg gac agc ctg cgc tac gat ctg act      1248
   Ala Ala Asp Pro Asn Glu Phe Leu Asp Ser Leu Arg Tyr Asp Leu Thr
                   405                 410                 415
   tcc aag cag atc ttc gtg ttc aca ccc aaa ggt gat gtg gtc aac ctg      1296
   Ser Lys Gln Ile Phe Val Phe Thr Pro Lys Gly Asp Val Val Asn Leu
               420                 425                 430
   ccg gtg aac tcc acc ccg gtg gac ttc gcc tac gcg gtg cac acc gaa      1344
   Pro Val Asn Ser Thr Pro Val Asp Phe Ala Tyr Ala Val His Thr Glu
           435                 440                 445
   gtg ggg cac cgc tgc atc ggc gcc aaa atc aac ggc aaa ctg gtc gct      1392
   Val Gly His Arg Cys Ile Gly Ala Lys Ile Asn Gly Lys Leu Val Ala
       450                 455                 460
   ttg gaa acg aaa ctc aaa tcc ggc gat cgt gtt gaa gtc ttt acc tcc      1440
   Leu Glu Thr Lys Leu Lys Ser Gly Asp Arg Val Glu Val Phe Thr Ser
   465                 470                 475                 480
   aag gac caa aac gct ggc cca agt agg gga tgg caa gaa ttt gtt gtc      1488
   Lys Asp Gln Asn Ala Gly Pro Ser Arg Gly Trp Gln Glu Phe Val Val
                   485                 490                 495
   tca cct cgt gca aag gcc aag att cgc cag tgg ttt gcc aag gaa cga      1536
   Ser Pro Arg Ala Lys Ala Lys Ile Arg Gln Trp Phe Ala Lys Glu Arg
               500                 505                 510
   cgc gaa gaa tac cta gaa gcc gga cgc gat gcg ctg gca gca gtt att      1584
   Arg Glu Glu Tyr Leu Glu Ala Gly Arg Asp Ala Leu Ala Ala Val Ile
           515                 520                 525
   cag cgt ggc ggc ctg cca atg cac cgc ttg ttc acc gcg tcc tcc atg      1632
   Gln Arg Gly Gly Leu Pro Met His Arg Leu Phe Thr Ala Ser Ser Met
       530                 535                 540
   aag acg gtg gca aca gag ctg cac tac cca gat gta gat gcg ctc tac      1680
   Lys Thr Val Ala Thr Glu Leu His Tyr Pro Asp Val Asp Ala Leu Tyr
   545                 550                 555                 560
   aca gcc atc ggc tcc ggt tct gta tct gcg caa cac gta gtc aac cgt      1728
   Thr Ala Ile Gly Ser Gly Ser Val Ser Ala Gln His Val Val Asn Arg
                   565                 570                 575
   ctc atg gct atc ttt ggt gac gaa gaa gat gcc gaa gac gca ttg gtt      1776
   Leu Met Ala Ile Phe Gly Asp Glu Glu Asp Ala Glu Asp Ala Leu Val
               580                 585                 590
   gca cgc acc cca ttc agc gag ctg gtc aac tcc cgt gcc acc acg gaa      1824
   Ala Arg Thr Pro Phe Ser Glu Leu Val Asn Ser Arg Ala Thr Thr Glu
           595                 600                 605
   agc agc acc ggc atc ctg gtc gaa ggc agc cca gat gtc atg gct aag      1872
   Ser Ser Thr Gly Ile Leu Val Glu Gly Ser Pro Asp Val Met Ala Lys
       610                 615                 620
   ctc gct aaa tgc tgt atg cca gtg cca gga gat gaa atc ttt gga ttc      1920
   Leu Ala Lys Cys Cys Met Pro Val Pro Gly Asp Glu Ile Phe Gly Phe
   625                 630                 635                 640
   gtc acc cgt ggt ggc ggt gtc tcc gta cac cga aca gac tgc acg aat      1968
   Val Thr Arg Gly Gly Gly Val Ser Val His Arg Thr Asp Cys Thr Asn
                   645                 650                 655
   gtg gaa aag ctc aaa gaa gag cca gaa cgc att gtc tcc gtc tcc tgg      2016
   Val Glu Lys Leu Lys Glu Glu Pro Glu Arg Ile Val Ser Val Ser Trp
               660                 665                 670
   gct tcg gaa ggt caa ggt tca gta ttc tct gcc aca ctg cag ctt gaa      2064
   Ala Ser Glu Gly Gln Gly Ser Val Phe Ser Ala Thr Leu Gln Leu Glu
           675                 680                 685
   gca ctt gat cgc gca ggc ctg ctc ttt gag ctc acc cgc gta atc aac      2112
   Ala Leu Asp Arg Ala Gly Leu Leu Phe Glu Leu Thr Arg Val Ile Asn
       690                 695                 700
   gaa caa aag gtc tcc gtt acc gca atg aac tcc cat tgc tca gaa gac      2160
   Glu Gln Lys Val Ser Val Thr Ala Met Asn Ser His Cys Ser Glu Asp
   705                 710                 715                 720
   cgc gta gcc acc gtg cgc ttc acc ttt gcg gtc tct gac acc aag cag      2208
   Arg Val Ala Thr Val Arg Phe Thr Phe Ala Val Ser Asp Thr Lys Gln
                   725                 730                 735
   ttg gga tcc ctg atg aca cag ctg cgc aat gcc gaa gga gtg ttt gat      2256
   Leu Gly Ser Leu Met Thr Gln Leu Arg Asn Ala Glu Gly Val Phe Asp
               740                 745                 750
   gtc tac cga gtg acc tcg ggt ggc tag                                  2283
   Val Tyr Arg Val Thr Ser Gly Gly
           755                 760
<210> 2
   <211> 760
   <212> PRT
   <213> Corynebacterium glutamicum
<400> 2
   Met Ser Leu Glu Arg Asn Thr Gln Lys Ser Ser Met Gly Val Arg Ser
   1               5                   10                  15
   Met Ser Ala Arg Leu Ala Arg Ser Leu Thr Gly Asn Arg Val Arg Thr
               20                  25                  30
   Asn Pro Val Leu Asp Pro Leu Leu Ser Ile His Arg Gln Phe His Pro
           35                  40                  45
   Arg Ala Asp Val Gln Val Leu Glu Arg Ala Tyr Asp Thr Ala Glu Arg
       50                  55                  60
   Leu His Asp Gly Val Ile Arg Lys Ser Gly Asp Pro Tyr Ile Thr His
   65                  70                  75                  80
   Pro Leu Ala Val Ala Thr Ile Ala Ala Glu Ile Gly Met Asp Thr Thr
                   85                  90                  95
   Thr Leu Val Ala Ala Leu Leu His Asp Thr Val Glu Asp Thr Asp Tyr
               100                 105                 110
   Ser Leu Asp Asp Leu Thr Arg Asp Phe Gly Glu Glu Val Ala Arg Leu
           115                 120                 125
   Val Asp Gly Val Thr Lys Leu Asp Lys Val Ala Leu Gly Ala Ala Ala
       130                 135                 140
   Glu Ala Glu Thr Ile Arg Lys Met Ile Val Ala Met Ser Gln Asp Pro
   145                 150                 155                 160
   Arg Val Leu Val Ile Lys Val Ala Asp Arg Leu His Asn Met Arg Thr
                   165                 170                 175
   Met Arg Phe Leu Pro Pro Glu Lys Gln Ala Lys Lys Ala Arg Gln Thr
               180                 185                 190
   Leu Glu Val Ile Ala Pro Leu Ala His Arg Leu Gly Met Ala Ser Val
           195                 200                 205
   Lys Trp Glu Leu Glu Asp Leu Ser Phe Ala Ile Leu Tyr Pro Lys Lys
       210                 215                 220
   Tyr Glu Glu Ile Val Arg Leu Val Ala Asp Arg Ala Pro Ser Arg Asp
   225                 230                 235                 240
   Arg Tyr Leu Lys Glu Ile Ile Asp Gln Val Thr Gly Gly Leu Arg Glu
                   245                 250                 255
   Asn Asn Ile Ala Ala Gly Val Leu Gly Arg Pro Lys His Tyr Trp Ser
               260                 265                 270
   Ile Tyr Gln Lys Met Ile Val Arg Gly Arg Asp Phe Asp Asp Ile Phe
           275                 280                 285
   Asp Leu Val Gly Ile Arg Ile Leu Val Asp Asn Val Asn Asn Cys Tyr
       290                 295                 300
   Ala Ala Ile Gly Val Val His Ser Leu Phe Asn Ala Leu Pro Gly Arg
   305                 310                 315                 320
   Phe Lys Asp Tyr Ile Ser Ala Pro Arg Phe Gly Val Tyr Gln Ser Leu
                   325                 330                 335
   His Thr Thr Val Met Gly Pro Gly Gly Lys Pro Leu Glu Val Gln Ala
   340 345 350
   Arg Thr His Asp Met His Tyr Asn Ala Glu Phe Gly Ile Ala Ala His
   355 360 365
   Trp Arg Tyr Lys Glu Thr Lys Gly Ser His Ser Gly Glu Gln Ala Glu
   370 375 380
   Val Asp Gln Met Ala Trp Met Arg Gln Leu Leu Asp Trp Gln Lys Glu
   385 390 395 400
   Ala Ala Asp Pro Asn Glu Phe Leu Asp Ser Leu Arg Tyr Asp Leu Thr
   405 410 415
   Ser Lys Gln Ile Phe Val Phe Thr Pro Lys Gly Asp Val Val Asn Leu
   420 425 430
   Pro Val Asn Ser Thr Pro Val Asp Phe Ala Tyr Ala Val His Thr Glu
   435 440 445
   Val Gly His Arg Cys Ile Gly Ala Lys Ile Asn Gly Lys Leu Val Ala
   450 455 460
   Leu Glu Thr Lys Leu Lys Ser Gly Asp Arg Val Glu Val Phe Thr Ser
   465 470 475 480
   Lys Asp Gln Asn Ala Gly Pro Ser Arg Gly Trp Gln Glu Phe Val Val
   485 490 495
   Ser Pro Arg Ala Lys Ala Lys Ile Arg Gln Trp Phe Ala Lys Glu Arg
   500 505 510
   Arg Glu Glu Tyr Leu Glu Ala Gly Arg Asp Ala Leu Ala Ala Val Ile
   515 520 525
   Gln Arg Gly Gly Leu Pro Met His Arg Leu Phe Thr Ala Ser Ser Met
   530 535 540
   Lys Thr Val Ala Thr Glu Leu His Tyr Pro Asp Val Asp Ala Leu Tyr
   545 550 555 560
   Thr Ala Ile Gly Ser Gly Ser Val Ser Ala Gln His Val Val Asn Arg
   565 570 575
   Leu Met Ala Ile Phe Gly Asp Glu Glu Asp Ala Glu Asp Ala Leu Val
   580 585 590
   Ala Arg Thr Pro Phe Ser Glu Leu Val Asn Ser Arg Ala Thr Thr Glu
           595                 600                 605
   Ser Ser Thr Gly Ile Leu Val Glu Gly Ser Pro Asp Val Met Ala Lys
       610                 615                 620
   Leu Ala Lys Cys Cys Met Pro Val Pro Gly Asp Glu Ile Phe Gly Phe
   625                 630                 635                 640
   Val Thr Arg Gly Gly Gly Val Ser Val His Arg Thr Asp Cys Thr Asn
                   645                 650                 655
   Val Glu Lys Leu Lys Glu Glu Pro Glu Arg Ile Val Ser Val Ser Trp
               660                 665                 670
   Ala Ser Glu Gly Gln Gly Ser Val Phe Ser Ala Thr Leu Gln Leu Glu
           675                 680                 685
   Ala Leu Asp Arg Ala Gly Leu Leu Phe Glu Leu Thr Arg Val Ile Asn
       690                 695                 700
   Glu Gln Lys Val Ser Val Thr Ala Met Asn Ser His Cys Ser Glu Asp
   705                 710                 715                 720
   Arg Val Ala Thr Val Arg Phe Thr Phe Ala Val Ser Asp Thr Lys Gln
                   725                 730                 735
   Leu Gly Ser Leu Met Thr Gln Leu Arg Asn Ala Glu Gly Val Phe Asp
               740                 745                 750
   Val Tyr Arg Val Thr Ser Gly Gly
           755                 760
<210> 3
   <211> 3800
   <212> DNA
   <213> Corynebacterium glutamicum
<220>
   <221> CDS
   <222> (751)..(3033)
   <223> rel-Wildtyp-Gen
<220>
   <221> misc_feature
   <222> (1535)..(1535)
   <223> Guanin
<400> 3
   aggcgcaact gaagaacaat tgtgggctga agtcccatca attccactag cagcgcaacc 60
   ccgagtgttt gtgatagatc gcacagtcgg taacgttgtt gttaatacag acctagccgg     120
   tatcggatgg aacatgaccg ttggtccaga agtgaggaat aagtagtgag cgaacaagct     180
   ctaagcacct tcgacagggc acgtgaggcc ctggacaaga aaacccgata tgtgcaggat     240
   ttcccagaaa agggtgtgct ttttgaagac ctcaccccgg tgttgggcga tgcagaatca     300
   tttgtggccg tggtggacgc catggctgaa gctgcagaaa aactgaatgc agaaatcatc     360
   ggtggcttgg atgcgcgagg attcctcctc ggatctgctg tcgcttacaa actcggccta     420
   ggtgtgctgg ctatccgcaa gaagggaaag ctccccccac ctgtggtgac ccaggagtat     480
   gaacttgaat acggcactgc agcactcgag ctgcccagtg aaggaatcga cattgctggt     540
   aaaaacatcg ttttgatcga cgatgtgctg gcaaccggcg gcaccttggg cgctgcacgt     600
   aaactaattg aatcgtgtga cggacatgtt tccggatatg ttcttgccat tgaggtccca     660
   ggcctcggcg gtagggataa tcttggtgat aggcccgtca ttgtggtcag agatcctcag     720
   tagaaggatc gaaagaaagg cggcaggaaa atg agt ctg gag cgc aac aca caa      774
                                    Met Ser Leu Glu Arg Asn Thr Gln
                                    1               5
   aaa tct tcc atg ggt gtg cga agc atg tca gcc agg ctt gcc cgc agc      822
   Lys Ser Ser Met Gly Val Arg Ser Met Ser Ala Arg Leu Ala Arg Ser
       10                  15                  20
   ctc aca gga aac cgc gtt cgc acc aac cct gtg ctg gat ccg ctg ctg      870
   Leu Thr Gly Asn Arg Val Arg Thr Asn Pro Val Leu Asp Pro Leu Leu
   25                  30                  35                  40
   agc atc cac cgg caa ttt cac cca cgc gcc gac gta caa gtg ttg gaa      918
   Ser Ile His Arg Gln Phe His Pro Arg Ala Asp Val Gln Val Leu Glu
                   45                  50                  55
   cgt gca tat gac acc gcg gaa cgt ctt cat gat ggt gtg att cga aaa      966
   Arg Ala Tyr Asp Thr Ala Glu Arg Leu His Asp Gly Val Ile Arg Lys
               60                  65                  70
   tcg ggc gat ccg tat att acc cac ccg ttg gct gtc gcc acc atc gcc     1014
   Ser Gly Asp Pro Tyr Ile Thr His Pro Leu Ala Val Ala Thr Ile Ala
           75                  80                  85
   gcg gaa atc ggc atg gac acc acc acg ctc gtc gca gcc ttg ttg cat     1062
   Ala Glu Ile Gly Met Asp Thr Thr Thr Leu Val Ala Ala Leu Leu His
       90                  95                  100
   gac acg gtg gaa gac acc gac tac tct ttg gac gat ctc acc cga gat     1110
   Asp Thr Val Glu Asp Thr Asp Tyr Ser Leu Asp Asp Leu Thr Arg Asp
   105                 110                 115                 120
   ttc gga gaa gaa gtt gcc agg ctt gtc gac ggt gtc acc aag ctc gac     1158
   Phe Gly Glu Glu Val Ala Arg Leu Val Asp Gly Val Thr Lys Leu Asp
                   125                 130                 135
   aaa gtc gca cta ggt gct gcc gcg gag gcc gaa acg att cgc aaa atg     1206
   Lys Val Ala Leu Gly Ala Ala Ala Glu Ala Glu Thr Ile Arg Lys Met
               140                 145                 150
   atc gtc gcc atg agc cag gac ccc cgc gtg ctg gtg att aaa gtg gcc     1254
   Ile Val Ala Met Ser Gln Asp Pro Arg Val Leu Val Ile Lys Val Ala
           155                 160                 165
   gac cgt ttg cac aat atg cgc acc atg cgg ttc ctg ccg ccg gaa aag      1302
   Asp Arg Leu His Asn Met Arg Thr Met Arg Phe Leu Pro Pro Glu Lys
       170                 175                 180
   caa gct aaa aaa gca cgc caa acc ctt gaa gtg att gct cct ttg gca      1350
   Gln Ala Lys Lys Ala Arg Gln Thr Leu Glu Val Ile Ala Pro Leu Ala
   185                 190                 195                 200
   cac cgc ctg ggc atg gcc agc gtg aaa tgg gaa ttg gaa gat cta tcc      1398
   His Arg Leu Gly Met Ala Ser Val Lys Trp Glu Leu Glu Asp Leu Ser
                   205                 210                 215
   ttt gcc att ttg tac ccc aag aag tac gaa gag atc gtg cgt ctt gtt      1446
   Phe Ala Ile Leu Tyr Pro Lys Lys Tyr Glu Glu Ile Val Arg Leu Val
               220                 225                 230
   gcc gac cgc gcg ccc tct aga gac cgg tac ctc aaa gaa att att gat      1494
   Ala Asp Arg Ala Pro Ser Arg Asp Arg Tyr Leu Lys Glu Ile Ile Asp
           235                 240                 245
   caa gtc acc ggt ggc ttg cgc gaa aac aac atc gcg gca gga gtg ctt      1542
   Gln Val Thr Gly Gly Leu Arg Glu Asn Asn Ile Ala Ala Gly Val Leu
       250                 255                 260
   ggt cga cca aag cac tac tgg tct atc tat caa aag atg atc gtt cgc      1590
   Gly Arg Pro Lys His Tyr Trp Ser Ile Tyr Gln Lys Met Ile Val Arg
   265                 270                 275                 280
   ggt cgt gat ttt gac gat att ttt gat ctt gtt ggc atc cgc atc ctg      1638
   Gly Arg Asp Phe Asp Asp Ile Phe Asp Leu Val Gly Ile Arg Ile Leu
                   285                 290                 295
   gta gac aac gtg aac aac tgt tac gcc gcc atc ggt gtc gtg cac tcc      1686
   Val Asp Asn Val Asn Asn Cys Tyr Ala Ala Ile Gly Val Val His Ser
               300                 305                 310
   ctg ttc aat gct ctg cct ggc cga ttc aaa gac tat att tca gcc ccg      1734
   Leu Phe Asn Ala Leu Pro Gly Arg Phe Lys Asp Tyr Ile Ser Ala Pro
           315                 320                 325
   cgc ttc ggt gtc tac caa tcc ctg cac acc acc gtg atg gga cct ggc      1782
   Arg Phe Gly Val Tyr Gln Ser Leu His Thr Thr Val Met Gly Pro Gly
       330                 335                 340
   ggt aag cct ctg gaa gtt cag gca cgt acc cac gac atg cac tac aac      1830
   Gly Lys Pro Leu Glu Val Gln Ala Arg Thr His Asp Met His Tyr Asn
   345                 350                 355                 360
   gcc gaa ttc ggc att gca gcg cac tgg cga tac aaa gaa acc aaa ggc      1878
   Ala Glu Phe Gly Ile Ala Ala His Trp Arg Tyr Lys Glu Thr Lys Gly
                   365                 370                 375
   agc cac agt ggc gag caa gcc gaa gtg gat caa atg gcg tgg atg cgc      1926
   Ser His Ser Gly Glu Gln Ala Glu Val Asp Gln Met Ala Trp Met Arg
               380                 385                 390
   caa ctt ctg gac tgg caa aaa gaa gca gcc gac ccc aac gag ttc ctg      1974
   Gln Leu Leu Asp Trp Gln Lys Glu Ala Ala Asp Pro Asn Glu Phe Leu
           395                 400                 405
   gac agc ctg cgc tac gat ctg act tcc aag cag atc ttc gtg ttc aca     2022
   Asp Ser Leu Arg Tyr Asp Leu Thr Ser Lys Gln Ile Phe Val Phe Thr
       410                 415                 420
   ccc aaa ggt gat gtg gtc aac ctg ccg gtg aac tcc acc ccg gtg gac     2070
   Pro Lys Gly Asp Val Val Asn Leu Pro Val Asn Ser Thr Pro Val Asp
   425                 430                 435                 440
   ttc gcc tac gcg gtg cac acc gaa gtg ggg cac cgc tgc atc ggc gcc     2118
   Phe Ala Tyr Ala Val His Thr Glu Val Gly His Arg Cys Ile Gly Ala
                   445                 450                 455
   aaa atc aac ggc aaa ctg gtc gct ttg gaa acg aaa ctc aaa tcc ggc     2166
   Lys Ile Asn Gly Lys Leu Val Ala Leu Glu Thr Lys Leu Lys Ser Gly
               460                 465                 470
   gat cgt gtt gaa gtc ttt acc tcc aag gac caa aac gct ggc cca agt     2214
   Asp Arg Val Glu Val Phe Thr Ser Lys Asp Gln Asn Ala Gly Pro Ser
           475                 480                 485
   agg gga tgg caa gaa ttt gtt gtc tca cct cgt gca aag gcc aag att     2262
   Arg Gly Trp Gln Glu Phe Val Val Ser Pro Arg Ala Lys Ala Lys Ile
       490                 495                 500
   cgc cag tgg ttt gcc aag gaa cga cgc gaa gaa tac cta gaa gcc gga     2310
   Arg Gln Trp Phe Ala Lys Glu Arg Arg Glu Glu Tyr Leu Glu Ala Gly
   505                 510                 515                 520
   cgc gat gcg ctg gca gca gtt att cag cgt ggc ggc ctg cca atg cac     2358
   Arg Asp Ala Leu Ala Ala Val Ile Gln Arg Gly Gly Leu Pro Met His
                   525                 530                 535
   cgc ttg ttc acc gcg tcc tcc atg aag acg gtg gca aca gag ctg cac     2406
   Arg Leu Phe Thr Ala Ser Ser Met Lys Thr Val Ala Thr Glu Leu His
               540                 545                 550
   tac cca gat gta gat gcg ctc tac aca gcc atc ggc tcc ggt tct gta     2454
   Tyr Pro Asp Val Asp Ala Leu Tyr Thr Ala Ile Gly Ser Gly Ser Val
           555                 560                 565
   tct gcg caa cac gta gtc aac cgt ctc atg gct atc ttt ggt gac gaa     2502
   Ser Ala Gln His Val Val Asn Arg Leu Met Ala Ile Phe Gly Asp Glu
       570                 575                 580
   gaa gat gcc gaa gac gca ttg gtt gca cgc acc cca ttc agc gag ctg     2550
   Glu Asp Ala Glu Asp Ala Leu Val Ala Arg Thr Pro Phe Ser Glu Leu
   585                 590                 595                 600
   gtc aac tcc cgt gcc acc acg gaa agc agc acc ggc atc ctg gtc gaa     2598
   Val Asn Ser Arg Ala Thr Thr Glu Ser Ser Thr Gly Ile Leu Val Glu
                   605                 610                 615
   ggc agc cca gat gtc atg gct aag ctc gct aaa tgc tgt atg cca gtg     2646
   Gly Ser Pro Asp Val Met Ala Lys Leu Ala Lys Cys Cys Met Pro Val
               620                 625                 630
   cca gga gat gaa atc ttt gga ttc gtc acc cgt ggt ggc ggt gtc tcc     2694
   Pro Gly Asp Glu Ile Phe Gly Phe Val Thr Arg Gly Gly Gly Val Ser
           635                 640                 645
   gta cac cga aca gac tgc acg aat gtg gaa aag ctc aaa gaa gag cca     2742
   Val His Arg Thr Asp Cys Thr Asn Val Glu Lys Leu Lys Glu Glu Pro
       650                 655                 660
   gaa cgc att gtc tcc gtc tcc tgg gct tcg gaa ggt caa ggt tca gta      2790
   Glu Arg Ile Val Ser Val Ser Trp Ala Ser Glu Gly Gln Gly Ser Val
   665                 670                 675                 680
   ttc tct gcc aca ctg cag ctt gaa gca ctt gat cgc gca ggc ctg ctc      2838
   Phe Ser Ala Thr Leu Gln Leu Glu Ala Leu Asp Arg Ala Gly Leu Leu
                   685                 690                 695
   ttt gag ctc acc cgc gta atc aac gaa caa aag gtc tcc gtt acc gca      2886
   Phe Glu Leu Thr Arg Val Ile Asn Glu Gln Lys Val Ser Val Thr Ala
               700                 705                 710
   atg aac tcc cat tgc tca gaa gac cgc gta gcc acc gtg cgc ttc acc      2934
   Met Asn Ser His Cys Ser Glu Asp Arg Val Ala Thr Val Arg Phe Thr
           715                 720                 725
   ttt gcg gtc tct gac acc aag cag ttg gga tcc ctg atg aca cag ctg      2982
   Phe Ala Val Ser Asp Thr Lys Gln Leu Gly Ser Leu Met Thr Gln Leu
       730                 735                 740
   cgc aat gcc gaa gga gtg ttt gat gtc tac cga gtg acc tcg ggt ggc      3030
   Arg Asn Ala Glu Gly Val Phe Asp Val Tyr Arg Val Thr Ser Gly Gly
   745                 750                 755                 760
   tag aggcctttag attgtgaaaa agccttgggc ttcaaccacc gtgtgttgac           3083
   acggttttgg ggttggattt aacgctggaa attttactgc aatgaacgct ggaaagcagt   3143
   agcgaaggtc aaaccagcgc tgaggacgcc aacgatgcca gtaataacga cgacgtaatc   3203
   caaaatggtg tcagagtcaa tgtcagaaga tccgttggtg ctaccgtttc ttcttctacg   3263
   gtgtcagcca cgtacacaac ctgtgctggt gcgacttctt cagcctgtgc aggaacggag   3323
   atggaggttg ctgccattgc agcagctgtg ccgagtgcga cgaggggagt acgagaaaat   3383
   agcttcattg ctaaacccct taggtttaaa tattcgaata cgaaagttac tttatgtgca   3443
   ctttcaccat tgtgcaacta tatgtgcagg tcaaagctga tattttgcta agaaaagtgc   3503
   aaagcgattc gagctgacgc gcagcccata tcgtcaaaaa atcctttgtg aatggtgatt   3563
   cacaaaggat tagtcgcgta aggcggaaat tacttcagga aggaatccag cttggtactg   3623
   aaggttaaaa tggtggttag tgcaccgatg actgctgtga tgatcttgat ccagttttcg   3683
   atgttgtcag tgtcggaaga accgctggaa ggctcttcgc tggacgggtc ttcgttgaaa   3743
   tcaaagttaa cgtaaacttc tgcttcaacg cctgcttcgt tcttggcaat gatcttt      3800
<210> 4
   <211> 760
   <212> PRT
   <213> Corynebacterium glutamicum
<400> 4
   Met Ser Leu Glu Arg Asn Thr Gln Lys Ser Ser Met Gly Val Arg Ser
   1               5                   10                  15
   Met Ser Ala Arg Leu Ala Arg Ser Leu Thr Gly Asn Arg Val Arg Thr
               20                  25                  30
   Asn Pro Val Leu Asp Pro Leu Leu Ser Ile His Arg Gln Phe His Pro
           35                  40                  45
   Arg Ala Asp Val Gln Val Leu Glu Arg Ala Tyr Asp Thr Ala Glu Arg
       50                  55                  60
   Leu His Asp Gly Val Ile Arg Lys Ser Gly Asp Pro Tyr Ile Thr His
   65                  70                  75                  80
   Pro Leu Ala Val Ala Thr Ile Ala Ala Glu Ile Gly Met Asp Thr Thr
                   85                  90                  95
   Thr Leu Val Ala Ala Leu Leu His Asp Thr Val Glu Asp Thr Asp Tyr
               100                 105                 110
   Ser Leu Asp Asp Leu Thr Arg Asp Phe Gly Glu Glu Val Ala Arg Leu
           115                 120                 125
   Val Asp Gly Val Thr Lys Leu Asp Lys Val Ala Leu Gly Ala Ala Ala
       130                 135                 140
   Glu Ala Glu Thr Ile Arg Lys Met Ile Val Ala Met Ser Gln Asp Pro
   145                 150                 155                 160
   Arg Val Leu Val Ile Lys Val Ala Asp Arg Leu His Asn Met Arg Thr
                   165                 170                 175
   Met Arg Phe Leu Pro Pro Glu Lys Gln Ala Lys Lys Ala Arg Gln Thr
               180                 185                 190
   Leu Glu Val Ile Ala Pro Leu Ala His Arg Leu Gly Met Ala Ser Val
           195                 200                 205
   Lys Trp Glu Leu Glu Asp Leu Ser Phe Ala Ile Leu Tyr Pro Lys Lys
       210                 215                 220
   Tyr Glu Glu Ile Val Arg Leu Val Ala Asp Arg Ala Pro Ser Arg Asp
   225                 230                 235                 240
   Arg Tyr Leu Lys Glu Ile Ile Asp Gln Val Thr Gly Gly Leu Arg Glu
                   245                 250                 255
   Asn Asn Ile Ala Ala Gly Val Leu Gly Arg Pro Lys His Tyr Trp Ser
               260                 265                 270
   Ile Tyr Gln Lys Met Ile Val Arg Gly Arg Asp Phe Asp Asp Ile Phe
           275                 280                 285
   Asp Leu Val Gly Ile Arg Ile Leu Val Asp Asn Val Asn Asn Cys Tyr
       290                 295                 300
   Ala Ala Ile Gly Val Val His Ser Leu Phe Asn Ala Leu Pro Gly Arg
   305                 310                 315                 320
   Phe Lys Asp Tyr Ile Ser Ala Pro Arg Phe Gly Val Tyr Gln Ser Leu
                   325                 330                 335
   His Thr Thr Val Met Gly Pro Gly Gly Lys Pro Leu Glu Val Gln Ala
               340                 345                 350
   Arg Thr His Asp Met His Tyr Asn Ala Glu Phe Gly Ile Ala Ala His
           355                 360                 365
   Trp Arg Tyr Lys Glu Thr Lys Gly Ser His Ser Gly Glu Gln Ala Glu
       370                 375                 380
   Val Asp Gln Met Ala Trp Met Arg Gln Leu Leu Asp Trp Gln Lys Glu
   385                 390                 395                 400
   Ala Ala Asp Pro Asn Glu Phe Leu Asp Ser Leu Arg Tyr Asp Leu Thr
                   405                 410                 415
   Ser Lys Gln Ile Phe Val Phe Thr Pro Lys Gly Asp Val Val Asn Leu
               420                 425                 430
   Pro Val Asn Ser Thr Pro Val Asp Phe Ala Tyr Ala Val His Thr Glu
           435                 440                 445
   Val Gly His Arg Cys Ile Gly Ala Lys Ile Asn Gly Lys Leu Val Ala
       450                 455                 460
   Leu Glu Thr Lys Leu Lys Ser Gly Asp Arg Val Glu Val Phe Thr Ser
   465                 470                 475                 480
   Lys Asp Gln Asn Ala Gly Pro Ser Arg Gly Trp Gln Glu Phe Val Val
                   485                 490                 495
   Ser Pro Arg Ala Lys Ala Lys Ile Arg Gln Trp Phe Ala Lys Glu Arg
               500                 505                 510
   Arg Glu Glu Tyr Leu Glu Ala Gly Arg Asp Ala Leu Ala Ala Val Ile
           515                 520                 525
   Gln Arg Gly Gly Leu Pro Met His Arg Leu Phe Thr Ala Ser Ser Met
       530                 535                 540
   Lys Thr Val Ala Thr Glu Leu His Tyr Pro Asp Val Asp Ala Leu Tyr
   545                 550                 555                 560
   Thr Ala Ile Gly Ser Gly Ser Val Ser Ala Gln His Val Val Asn Arg
                   565                 570                 575
   Leu Met Ala Ile Phe Gly Asp Glu Glu Asp Ala Glu Asp Ala Leu Val
               580                 585                 590
   Ala Arg Thr Pro Phe Ser Glu Leu Val Asn Ser Arg Ala Thr Thr Glu
           595                 600                 605
   Ser Ser Thr Gly Ile Leu Val Glu Gly Ser Pro Asp Val Met Ala Lys
       610                 615                 620
   Leu Ala Lys Cys Cys Met Pro Val Pro Gly Asp Glu Ile Phe Gly Phe
   625                 630                 635                 640
   Val Thr Arg Gly Gly Gly Val Ser Val His Arg Thr Asp Cys Thr Asn
                   645                 650                 655
   Val Glu Lys Leu Lys Glu Glu Pro Glu Arg Ile Val Ser Val Ser Trp
               660                 665                 670
   Ala Ser Glu Gly Gln Gly Ser Val Phe Ser Ala Thr Leu Gln Leu Glu
           675                 680                 685
   Ala Leu Asp Arg Ala Gly Leu Leu Phe Glu Leu Thr Arg Val Ile Asn
       690                 695                 700
   Glu Gln Lys Val Ser Val Thr Ala Met Asn Ser His Cys Ser Glu Asp
   705                 710                 715                 720
   Arg Val Ala Thr Val Arg Phe Thr Phe Ala Val Ser Asp Thr Lys Gln
                   725                 730                 735
   Leu Gly Ser Leu Met Thr Gln Leu Arg Asn Ala Glu Gly Val Phe Asp
               740                 745                 750
   Val Tyr Arg Val Thr Ser Gly Gly
           755                 760
<210> 5
   <211> 2283
   <212> DNA
   <213> Corynebacterium glutamicum
<220>
   <221> CDS
   <222> (1)..(2280)
   <223> rel-Allel
<220>
   <221> mutation
   <222> (113)..(113)
   <223> C → T Transition
<220>
   <221> misc_feature
   <222> (785)..(785)
   <223> Guanin
<400> 5
   atg agt ctg gag cgc aac aca caa aaa tct tcc atg ggt gtg cga agc      48
   Met Ser Leu Glu Arg Asn Thr Gln Lys Ser Ser Met Gly Val Arg Ser
   1               5                   10                  15
   atg tca gcc agg ctt gcc cgc agc ctc aca gga aac cgc gtt cgc acc      96
   Met Ser Ala Arg Leu Ala Arg Ser Leu Thr Gly Asn Arg Val Arg Thr
               20                  25                  30
   aac cct gtg ctg gat ctg ctg ctg agc atc cac cgg caa ttt cac cca     144
   Asn Pro Val Leu Asp Leu Leu Leu Ser Ile His Arg Gln Phe His Pro
           35                  40                  45
   cgc gcc gac gta caa gtg ttg gaa cgt gca tat gac acc gcg gaa cgt     192
   Arg Ala Asp Val Gln Val Leu Glu Arg Ala Tyr Asp Thr Ala Glu Arg
       50                  55                  60
   ctt cat gat ggt gtg att cga aaa tcg ggc gat ccg tat att acc cac     240
   Leu His Asp Gly Val Ile Arg Lys Ser Gly Asp Pro Tyr Ile Thr His
   65                  70                  75                  80
   ccg ttg gct gtc gcc acc atc gcc gcg gaa atc ggc atg gac acc acc     288
   Pro Leu Ala Val Ala Thr Ile Ala Ala Glu Ile Gly Met Asp Thr Thr
                   85                  90                  95
   acg ctc gtc gca gcc ttg ttg cat gac acg gtg gaa gac acc gac tac     336
   Thr Leu Val Ala Ala Leu Leu His Asp Thr Val Glu Asp Thr Asp Tyr
               100                 105                 110
   tct ttg gac gat ctc acc cga gat ttc gga gaa gaa gtt gcc agg ctt     384
   Ser Leu Asp Asp Leu Thr Arg Asp Phe Gly Glu Glu Val Ala Arg Leu
           115                 120                 125
   gtc gac ggt gtc acc aag ctc gac aaa gtc gca cta ggt gct gcc gcg     432
   Val Asp Gly Val Thr Lys Leu Asp Lys Val Ala Leu Gly Ala Ala Ala
       130                 135                 140
   gag gcc gaa acg att cgc aaa atg atc gtc gcc atg agc cag gac ccc     480
   Glu Ala Glu Thr Ile Arg Lys Met Ile Val Ala Met Ser Gln Asp Pro
   145                 150                 155                 160
   cgc gtg ctg gtg att aaa gtg gcc gac cgt ttg cac aat atg cgc acc     528
   Arg Val Leu Val Ile Lys Val Ala Asp Arg Leu His Asn Met Arg Thr
               165                 170                 175
   atg cgg ttc ctg ccg ccg gaa aag caa gct aaa aaa gca cgc caa acc      576
   Met Arg Phe Leu Pro Pro Glu Lys Gln Ala Lys Lys Ala Arg Gln Thr
           180                 185                 190
   ctt gaa gtg att gct cct ttg gca cac cgc ctg ggc atg gcc agc gtg      624
   Leu Glu Val Ile Ala Pro Leu Ala His Arg Leu Gly Met Ala Ser Val
       195                 200                 205
   aaa tgg gaa ttg gaa gat cta tcc ttt gcc att ttg tac ccc aag aag      672
   Lys Trp Glu Leu Glu Asp Leu Ser Phe Ala Ile Leu Tyr Pro Lys Lys
   210                 215                 220
   tac gaa gag atc gtg cgt ctt gtt gcc gac cgc gcg ccc tct aga gac      720
   Tyr Glu Glu Ile Val Arg Leu Val Ala Asp Arg Ala Pro Ser Arg Asp
   225                 230                 235                 240
   cgg tac ctc aaa gaa att att gat caa gtc acc ggt ggc ttg cgc gaa      768
   Arg Tyr Leu Lys Glu Ile Ile Asp Gln Val Thr Gly Gly Leu Arg Glu
               245                 250                 255
   aac aac atc gcg gca gga gtg ctt ggt cga cca aag cac tac tgg tct      816
   Asn Asn Ile Ala Ala Gly Val Leu Gly Arg Pro Lys His Tyr Trp Ser
           260                 265                 270
   atc tat caa aag atg atc gtt cgc ggt cgt gat ttt gac gat att ttt      864
   Ile Tyr Gln Lys Met Ile Val Arg Gly Arg Asp Phe Asp Asp Ile Phe
       275                 280                 285
   gat ctt gtt ggc atc cgc atc ctg gta gac aac gtg aac aac tgt tac      912
   Asp Leu Val Gly Ile Arg Ile Leu Val Asp Asn Val Asn Asn Cys Tyr
   290                 295                 300
   gcc gcc atc ggt gtc gtg cac tcc ctg ttc aat gct ctg cct ggc cga      960
   Ala Ala Ile Gly Val Val His Ser Leu Phe Asn Ala Leu Pro Gly Arg
   305                 310                 315                 320
   ttc aaa gac tat att tca gcc ccg cgc ttc ggt gtc tac caa tcc ctg     1008
   Phe Lys Asp Tyr Ile Ser Ala Pro Arg Phe Gly Val Tyr Gln Ser Leu
               325                 330                 335
   cac acc acc gtg atg gga cct ggc ggt aag cct ctg gaa gtt cag gca     1056
   His Thr Thr Val Met Gly Pro Gly Gly Lys Pro Leu Glu Val Gln Ala
           340                 345                 350
   cgt acc cac gac atg cac tac aac gcc gaa ttc ggc att gca gcg cac     1104
   Arg Thr His Asp Met His Tyr Asn Ala Glu Phe Gly Ile Ala Ala His
       355                 360                 365
   tgg cga tac aaa gaa acc aaa ggc agc cac agt ggc gag caa gcc gaa     1152
   Trp Arg Tyr Lys Glu Thr Lys Gly Ser His Ser Gly Glu Gln Ala Glu
   370                 375                 380
   gtg gat caa atg gcg tgg atg cgc caa ctt ctg gac tgg caa aaa gaa     1200
   Val Asp Gln Met Ala Trp Met Arg Gln Leu Leu Asp Trp Gln Lys Glu
   385                 390                 395                 400
   gca gcc gac ccc aac gag ttc ctg gac agc ctg cgc tac gat ctg act     1248
   Ala Ala Asp Pro Asn Glu Phe Leu Asp Ser Leu Arg Tyr Asp Leu Thr
               405                 410                 415
   tcc aag cag atc ttc gtg ttc aca ccc aaa ggt gat gtg gtc aac ctg     1296
   Ser Lys Gln Ile Phe Val Phe Thr Pro Lys Gly Asp Val Val Asn Leu
               420                 425                 430
   ccg gtg aac tcc acc ccg gtg gac ttc gcc tac gcg gtg cac acc gaa      1344
   Pro Val Asn Ser Thr Pro Val Asp Phe Ala Tyr Ala Val His Thr Glu
           435                 440                 445
   gtg ggg cac cgc tgc atc ggc gcc aaa atc aac ggc aaa ctg gtc gct      1392
   Val Gly His Arg Cys Ile Gly Ala Lys Ile Asn Gly Lys Leu Val Ala
       450                 455                 460
   ttg gaa acg aaa ctc aaa tcc ggc gat cgt gtt gaa gtc ttt acc tcc      1440
   Leu Glu Thr Lys Leu Lys Ser Gly Asp Arg Val Glu Val Phe Thr Ser
   465                 470                 475                 480
   aag gac caa aac gct ggc cca agt agg gga tgg caa gaa ttt gtt gtc      1488
   Lys Asp Gln Asn Ala Gly Pro Ser Arg Gly Trp Gln Glu Phe Val Val
                   485                 490                 495
   tca cct cgt gca aag gcc aag att cgc cag tgg ttt gcc aag gaa cga      1536
   Ser Pro Arg Ala Lys Ala Lys Ile Arg Gln Trp Phe Ala Lys Glu Arg
               500                 505                 510
   cgc gaa gaa tac cta gaa gcc gga cgc gat gcg ctg gca gca gtt att      1584
   Arg Glu Glu Tyr Leu Glu Ala Gly Arg Asp Ala Leu Ala Ala Val Ile
           515                 520                 525
   cag cgt ggc ggc ctg cca atg cac cgc ttg ttc acc gcg tcc tcc atg      1632
   Gln Arg Gly Gly Leu Pro Met His Arg Leu Phe Thr Ala Ser Ser Met
       530                 535                 540
   aag acg gtg gca aca gag ctg cac tac cca gat gta gat gcg ctc tac      1680
   Lys Thr Val Ala Thr Glu Leu His Tyr Pro Asp Val Asp Ala Leu Tyr
   545                 550                 555                 560
   aca gcc atc ggc tcc ggt tct gta tct gcg caa cac gta gtc aac cgt      1728
   Thr Ala Ile Gly Ser Gly Ser Val Ser Ala Gln His Val Val Asn Arg
                   565                 570                 575
   ctc atg gct atc ttt ggt gac gaa gaa gat gcc gaa gac gca ttg gtt      1776
   Leu Met Ala Ile Phe Gly Asp Glu Glu Asp Ala Glu Asp Ala Leu Val
               580                 585                 590
   gca cgc acc cca ttc agc gag ctg gtc aac tcc cgt gcc acc acg gaa      1824
   Ala Arg Thr Pro Phe Ser Glu Leu Val Asn Ser Arg Ala Thr Thr Glu
           595                 600                 605
   agc agc acc ggc atc ctg gtc gaa ggc agc cca gat gtc atg gct aag      1872
   Ser Ser Thr Gly Ile Leu Val Glu Gly Ser Pro Asp Val Met Ala Lys
       610                 615                 620
   ctc gct aaa tgc tgt atg cca gtg cca gga gat gaa atc ttt gga ttc      1920
   Leu Ala Lys Cys Cys Met Pro Val Pro Gly Asp Glu Ile Phe Gly Phe
   625                 630                 635                 640
   gtc acc cgt ggt ggc ggt gtc tcc gta cac cga aca gac tgc acg aat      1968
   Val Thr Arg Gly Gly Gly Val Ser Val His Arg Thr Asp Cys Thr Asn
                   645                 650                 655
   gtg gaa aag ctc aaa gaa gag cca gaa cgc att gtc tcc gtc tcc tgg      2016
   Val Glu Lys Leu Lys Glu Glu Pro Glu Arg Ile Val Ser Val Ser Trp
               660                 665                 670
   gct tcg gaa ggt caa ggt tca gta ttc tct gcc aca ctg cag ctt gaa      2064
   Ala Ser Glu Gly Gln Gly Ser Val Phe Ser Ala Thr Leu Gln Leu Glu
           675                 680                 685
   gca ctt gat cgc gca ggc ctg ctc ttt gag ctc acc cgc gta atc aac      2112
   Ala Leu Asp Arg Ala Gly Leu Leu Phe Glu Leu Thr Arg Val Ile Asn
       690                 695                 700
   gaa caa aag gtc tcc gtt acc gca atg aac tcc cat tgc tca gaa gac      2160
   Glu Gln Lys Val Ser Val Thr Ala Met Asn Ser His Cys Ser Glu Asp
   705                 710                 715                 720
   cgc gta gcc acc gtg cgc ttc acc ttt gcg gtc tct gac acc aag cag      2208
   Arg Val Ala Thr Val Arg Phe Thr Phe Ala Val Ser Asp Thr Lys Gln
                   725                 730                 735
   ttg gga tcc ctg atg aca cag ctg cgc aat gcc gaa gga gtg ttt gat      2256
   Leu Gly Ser Leu Met Thr Gln Leu Arg Asn Ala Glu Gly Val Phe Asp
               740                 745                 750
   gtc tac cga gtg acc tcg ggt ggc tag                                  2283
   Val Tyr Arg Val Thr Ser Gly Gly
           755                 760
<210> 6
   <211> 760
   <212> PRT
   <213> Corynebacterium glutamicum
<400> 6
   Met Ser Leu Glu Arg Asn Thr Gln Lys Ser Ser Met Gly Val Arg Ser
   1               5                   10                  15
   Met Ser Ala Arg Leu Ala Arg Ser Leu Thr Gly Asn Arg Val Arg Thr
               20                  25                  30
   Asn Pro Val Leu Asp Leu Leu Leu Ser Ile His Arg Gln Phe His Pro
           35                  40                  45
   Arg Ala Asp Val Gln Val Leu Glu Arg Ala Tyr Asp Thr Ala Glu Arg
       50                  55                  60
   Leu His Asp Gly Val Ile Arg Lys Ser Gly Asp Pro Tyr Ile Thr His
   65                  70                  75                  80
   Pro Leu Ala Val Ala Thr Ile Ala Ala Glu Ile Gly Met Asp Thr Thr
                   85                  90                  95
   Thr Leu Val Ala Ala Leu Leu His Asp Thr Val Glu Asp Thr Asp Tyr
               100                 105                 110
   Ser Leu Asp Asp Leu Thr Arg Asp Phe Gly Glu Glu Val Ala Arg Leu
           115                 120                 125
   Val Asp Gly Val Thr Lys Leu Asp Lys Val Ala Leu Gly Ala Ala Ala
       130                 135                 140
   Glu Ala Glu Thr Ile Arg Lys Met Ile Val Ala Met Ser Gln Asp Pro
   145                 150                 155                 160
   Arg Val Leu Val Ile Lys Val Ala Asp Arg Leu His Asn Met Arg Thr
                   165                 170                 175
   Met Arg Phe Leu Pro Pro Glu Lys Gln Ala Lys Lys Ala Arg Gln Thr
               180                 185                 190
   Leu Glu Val Ile Ala Pro Leu Ala His Arg Leu Gly Met Ala Ser Val
           195                 200                 205
   Lys Trp Glu Leu Glu Asp Leu Ser Phe Ala Ile Leu Tyr Pro Lys Lys
       210                 215                 220
   Tyr Glu Glu Ile Val Arg Leu Val Ala Asp Arg Ala Pro Ser Arg Asp
   225                 230                 235                 240
   Arg Tyr Leu Lys Glu Ile Ile Asp Gln Val Thr Gly Gly Leu Arg Glu
                   245                 250                 255
   Asn Asn Ile Ala Ala Gly Val Leu Gly Arg Pro Lys His Tyr Trp Ser
               260                 265                 270
   Ile Tyr Gln Lys Met Ile Val Arg Gly Arg Asp Phe Asp Asp Ile Phe
           275                 280                 285
   Asp Leu Val Gly Ile Arg Ile Leu Val Asp Asn Val Asn Asn Cys Tyr
       290                 295                 300
   Ala Ala Ile Gly Val Val His Ser Leu Phe Asn Ala Leu Pro Gly Arg
   305                 310                 315                 320
   Phe Lys Asp Tyr Ile Ser Ala Pro Arg Phe Gly Val Tyr Gln Ser Leu
                   325                 330                 335
   His Thr Thr Val Met Gly Pro Gly Gly Lys Pro Leu Glu Val Gln Ala
               340                 345                 350
   Arg Thr His Asp Met His Tyr Asn Ala Glu Phe Gly Ile Ala Ala His
           355                 360                 365
   Trp Arg Tyr Lys Glu Thr Lys Gly Ser His Ser Gly Glu Gln Ala Glu
       370                 375                 380
   Val Asp Gln Met Ala Trp Met Arg Gln Leu Leu Asp Trp Gln Lys Glu
   385 390 395 400
   Ala Ala Asp Pro Asn Glu Phe Leu Asp Ser Leu Arg Tyr Asp Leu Thr
   405 410 415
   Ser Lys Gln Ile Phe Val Phe Thr Pro Lys Gly Asp Val Val Asn Leu
   420 425 430
   Pro Val Asn Ser Thr Pro Val Asp Phe Ala Tyr Ala Val His Thr Glu
   435 440 445
   Val Gly His Arg Cys Ile Gly Ala Lys Ile Asn Gly Lys Leu Val Ala
   450 455 460
   Leu Glu Thr Lys Leu Lys Ser Gly Asp Arg Val Glu Val Phe Thr Ser
   465 470 475 480
   Lys Asp Gln Asn Ala Gly Pro Ser Arg Gly Trp Gln Glu Phe Val Val
   485 490 495
   Ser Pro Arg Ala Lys Ala Lys Ile Arg Gln Trp Phe Ala Lys Glu Arg
   500 505 510
   Arg Glu Glu Tyr Leu Glu Ala Gly Arg Asp Ala Leu Ala Ala Val Ile
   515 520 525
   Gln Arg Gly Gly Leu Pro Met His Arg Leu Phe Thr Ala Ser Ser Met
   530 535 540
   Lys Thr Val Ala Thr Glu Leu His Tyr Pro Asp Val Asp Ala Leu Tyr
   545 550 555 560
   Thr Ala Ile Gly Ser Gly Ser Val Ser Ala Gln His Val Val Asn Arg
   565 570 575
   Leu Met Ala Ile Phe Gly Asp Glu Glu Asp Ala Glu Asp Ala Leu Val
   580 585 590
   Ala Arg Thr Pro Phe Ser Glu Leu Val Asn Ser Arg Ala Thr Thr Glu
   595 600 605
   Ser Ser Thr Gly Ile Leu Val Glu Gly Ser Pro Asp Val Met Ala Lys
   610 615 620
   Leu Ala Lys Cys Cys Met Pro Val Pro Gly Asp Glu Ile Phe Gly Phe
   625                 630                 635                 640
   Val Thr Arg Gly Gly Gly Val Ser Val His Arg Thr Asp Cys Thr Asn
                   645                 650                 655
   Val Glu Lys Leu Lys Glu Glu Pro Glu Arg Ile Val Ser Val Ser Trp
               660                 665                 670
   Ala Ser Glu Gly Gln Gly Ser Val Phe Ser Ala Thr Leu Gln Leu Glu
           675                 680                 685
   Ala Leu Asp Arg Ala Gly Leu Leu Phe Glu Leu Thr Arg Val Ile Asn
       690                 695                 700
   Glu Gln Lys Val Ser Val Thr Ala Met Asn Ser His Cys Ser Glu Asp
   705                 710                 715                 720
   Arg Val Ala Thr Val Arg Phe Thr Phe Ala Val Ser Asp Thr Lys Gln
                   725                 730                 735
   Leu Gly Ser Leu Met Thr Gln Leu Arg Asn Ala Glu Gly Val Phe Asp
               740                 745                 750
   Val Tyr Arg Val Thr Ser Gly Gly
           755                 760
<210> 7
   <211> 3800
   <212> DNA
   <213> Corynebacterium glutamicum
<220>
   <221> CDS
   <222> (751)..(3033)
   <223> rel-Allel
<220>
   <221> mutation
   <222> (863)..(863)
   <223> C → T Transition
<220>
   <221> misc_feature
   <222> (1535)..(1535)
   <223> Guanin
<400> 7
   aggcgcaact gaagaacaat tgtgggctga agtcccatca attccactag cagcgcaacc     60
   ccgagtgttt gtgatagatc gcacagtcgg taacgttgtt gttaatacag acctagccgg    120
   tatcggatgg aacatgaccg ttggtccaga agtgaggaat aagtagtgag cgaacaagct    180
   ctaagcacct tcgacagggc acgtgaggcc ctggacaaga aaacccgata tgtgcaggat    240
   ttcccagaaa agggtgtgct ttttgaagac ctcaccccgg tgttgggcga tgcagaatca    300
   tttgtggccg tggtggacgc catggctgaa gctgcagaaa aactgaatgc agaaatcatc    360
   ggtggcttgg atgcgcgagg attcctcctc ggatctgctg tcgcttacaa actcggccta    420
   ggtgtgctgg ctatccgcaa gaagggaaag ctccccccac ctgtggtgac ccaggagtat    480
   gaacttgaat acggcactgc agcactcgag ctgcccagtg aaggaatcga cattgctggt    540
   aaaaacatcg ttttgatcga cgatgtgctg gcaaccggcg gcaccttggg cgctgcacgt    600
   aaactaattg aatcgtgtga cggacatgtt tccggatatg ttcttgccat tgaggtccca    660
   ggcctcggcg gtagggataa tcttggtgat aggcccgtca ttgtggtcag agatcctcag    720
   tagaaggatc gaaagaaagg cggcaggaaa atg agt ctg gag cgc aac aca caa     774
                                    Met Ser Leu Glu Arg Asn Thr Gln
                                    1               5
   aaa tct tcc atg ggt gtg cga agc atg tca gcc agg ctt gcc cgc agc      822
   Lys Ser Ser Met Gly Val Arg Ser Met Ser Ala Arg Leu Ala Arg Ser
       10                  15                  20
   ctc aca gga aac cgc gtt cgc acc aac cct gtg ctg gat ctg ctg ctg      870
   Leu Thr Gly Asn Arg Val Arg Thr Asn Pro Val Leu Asp Leu Leu Leu
   25                  30                  35                  40
   agc atc cac cgg caa ttt cac cca cgc gcc gac gta caa gtg ttg gaa      918
   Ser Ile His Arg Gln Phe His Pro Arg Ala Asp Val Gln Val Leu Glu
                   45                  50                  55
   cgt gca tat gac acc gcg gaa cgt ctt cat gat ggt gtg att cga aaa      966
   Arg Ala Tyr Asp Thr Ala Glu Arg Leu His Asp Gly Val Ile Arg Lys
               60                  65                  70
   tcg ggc gat ccg tat att acc cac ccg ttg gct gtc gcc acc atc gcc     1014
   Ser Gly Asp Pro Tyr Ile Thr His Pro Leu Ala Val Ala Thr Ile Ala
           75                  80                  85
   gcg gaa atc ggc atg gac acc acc acg ctc gtc gca gcc ttg ttg cat     1062
   Ala Glu Ile Gly Met Asp Thr Thr Thr Leu Val Ala Ala Leu Leu His
       90                  95                  100
   gac acg gtg gaa gac acc gac tac tct ttg gac gat ctc acc cga gat     1110
   Asp Thr Val Glu Asp Thr Asp Tyr Ser Leu Asp Asp Leu Thr Arg Asp
   105                 110                 115                 120
   ttc gga gaa gaa gtt gcc agg ctt gtc gac ggt gtc acc aag ctc gac     1158
   Phe Gly Glu Glu Val Ala Arg Leu Val Asp Gly Val Thr Lys Leu Asp
                   125                 130                 135
   aaa gtc gca cta ggt gct gcc gcg gag gcc gaa acg att cgc aaa atg     1206
   Lys Val Ala Leu Gly Ala Ala Ala Glu Ala Glu Thr Ile Arg Lys Met
               140                 145                 150
   atc gtc gcc atg agc cag gac ccc cgc gtg ctg gtg att aaa gtg gcc     1254
   Ile Val Ala Met Ser Gln Asp Pro Arg Val Leu Val Ile Lys Val Ala
           155                 160                 165
   gac cgt ttg cac aat atg cgc acc atg cgg ttc ctg ccg ccg gaa aag     1302
   Asp Arg Leu His Asn Met Arg Thr Met Arg Phe Leu Pro Pro Glu Lys
       170                 175                 180
   caa gct aaa aaa gca cgc caa acc ctt gaa gtg att gct cct ttg gca      1350
   Gln Ala Lys Lys Ala Arg Gln Thr Leu Glu Val Ile Ala Pro Leu Ala
   185                 190                 195                 200
   cac cgc ctg ggc atg gcc agc gtg aaa tgg gaa ttg gaa gat cta tcc      1398
   His Arg Leu Gly Met Ala Ser Val Lys Trp Glu Leu Glu Asp Leu Ser
                   205                 210                 215
   ttt gcc att ttg tac ccc aag aag tac gaa gag atc gtg cgt ctt gtt      1446
   Phe Ala Ile Leu Tyr Pro Lys Lys Tyr Glu Glu Ile Val Arg Leu Val
               220                 225                 230
   gcc gac cgc gcg ccc tct aga gac cgg tac ctc aaa gaa att att gat      1494
   Ala Asp Arg Ala Pro Ser Arg Asp Arg Tyr Leu Lys Glu Ile Ile Asp
           235                 240                 245
   caa gtc acc ggt ggc ttg cgc gaa aac aac atc gcg gca gga gtg ctt      1542
   Gln Val Thr Gly Gly Leu Arg Glu Asn Asn Ile Ala Ala Gly Val Leu
       250                 255                 260
   ggt cga cca aag cac tac tgg tct atc tat caa aag atg atc gtt cgc      1590
   Gly Arg Pro Lys His Tyr Trp Ser Ile Tyr Gln Lys Met Ile Val Arg
   265                 270                 275                 280
   ggt cgt gat ttt gac gat att ttt gat ctt gtt ggc atc cgc atc ctg      1638
   Gly Arg Asp Phe Asp Asp Ile Phe Asp Leu Val Gly Ile Arg Ile Leu
                   285                 290                 295
   gta gac aac gtg aac aac tgt tac gcc gcc atc ggt gtc gtg cac tcc      1686
   Val Asp Asn Val Asn Asn Cys Tyr Ala Ala Ile Gly Val Val His Ser
               300                 305                 310
   ctg ttc aat gct ctg cct ggc cga ttc aaa gac tat att tca gcc ccg      1734
   Leu Phe Asn Ala Leu Pro Gly Arg Phe Lys Asp Tyr Ile Ser Ala Pro
           315                 320                 325
   cgc ttc ggt gtc tac caa tcc ctg cac acc acc gtg atg gga cct ggc      1782
   Arg Phe Gly Val Tyr Gln Ser Leu His Thr Thr Val Met Gly Pro Gly
       330                 335                 340
   ggt aag cct ctg gaa gtt cag gca cgt acc cac gac atg cac tac aac      1830
   Gly Lys Pro Leu Glu Val Gln Ala Arg Thr His Asp Met His Tyr Asn
   345                 350                 355                 360
   gcc gaa ttc ggc att gca gcg cac tgg cga tac aaa gaa acc aaa ggc      1878
   Ala Glu Phe Gly Ile Ala Ala His Trp Arg Tyr Lys Glu Thr Lys Gly
                   365                 370                 375
   agc cac agt ggc gag caa gcc gaa gtg gat caa atg gcg tgg atg cgc      1926
   Ser His Ser Gly Glu Gln Ala Glu Val Asp Gln Met Ala Trp Met Arg
               380                 385                 390
   caa ctt ctg gac tgg caa aaa gaa gca gcc gac ccc aac gag ttc ctg      1974
   Gln Leu Leu Asp Trp Gln Lys Glu Ala Ala Asp Pro Asn Glu Phe Leu
           395                 400                 405
   gac agc ctg cgc tac gat ctg act tcc aag cag atc ttc gtg ttc aca      2022
   Asp Ser Leu Arg Tyr Asp Leu Thr Ser Lys Gln Ile Phe Val Phe Thr
       410                 415                 420
   ccc aaa ggt gat gtg gtc aac ctg ccg gtg aac tcc acc ccg gtg gac      2070
   Pro Lys Gly Asp Val Val Asn Leu Pro Val Asn Ser Thr Pro Val Asp
   425                 430                 435                 440
   ttc gcc tac gcg gtg cac acc gaa gtg ggg cac cgc tgc atc ggc gcc      2118
   Phe Ala Tyr Ala Val His Thr Glu Val Gly His Arg Cys Ile Gly Ala
                   445                 450                 455
   aaa atc aac ggc aaa ctg gtc gct ttg gaa acg aaa ctc aaa tcc ggc      2166
   Lys Ile Asn Gly Lys Leu Val Ala Leu Glu Thr Lys Leu Lys Ser Gly
               460                 465                 470
   gat cgt gtt gaa gtc ttt acc tcc aag gac caa aac gct ggc cca agt      2214
   Asp Arg Val Glu Val Phe Thr Ser Lys Asp Gln Asn Ala Gly Pro Ser
           475                 480                 485
   agg gga tgg caa gaa ttt gtt gtc tca cct cgt gca aag gcc aag att      2262
   Arg Gly Trp Gln Glu Phe Val Val Ser Pro Arg Ala Lys Ala Lys Ile
       490                 495                 500
   cgc cag tgg ttt gcc aag gaa cga cgc gaa gaa tac cta gaa gcc gga      2310
   Arg Gln Trp Phe Ala Lys Glu Arg Arg Glu Glu Tyr Leu Glu Ala Gly
   505                 510                 515                 520
   cgc gat gcg ctg gca gca gtt att cag cgt ggc ggc ctg cca atg cac      2358
   Arg Asp Ala Leu Ala Ala Val Ile Gln Arg Gly Gly Leu Pro Met His
                   525                 530                 535
   cgc ttg ttc acc gcg tcc tcc atg aag acg gtg gca aca gag ctg cac      2406
   Arg Leu Phe Thr Ala Ser Ser Met Lys Thr Val Ala Thr Glu Leu His
               540                 545                 550
   tac cca gat gta gat gcg ctc tac aca gcc atc ggc tcc ggt tct gta      2454
   Tyr Pro Asp Val Asp Ala Leu Tyr Thr Ala Ile Gly Ser Gly Ser Val
           555                 560                 565
   tct gcg caa cac gta gtc aac cgt ctc atg gct atc ttt ggt gac gaa      2502
   Ser Ala Gln His Val Val Asn Arg Leu Met Ala Ile Phe Gly Asp Glu
       570                 575                 580
   gaa gat gcc gaa gac gca ttg gtt gca cgc acc cca ttc agc gag ctg      2550
   Glu Asp Ala Glu Asp Ala Leu Val Ala Arg Thr Pro Phe Ser Glu Leu
   585                 590                 595                 600
   gtc aac tcc cgt gcc acc acg gaa agc agc acc ggc atc ctg gtc gaa      2598
   Val Asn Ser Arg Ala Thr Thr Glu Ser Ser Thr Gly Ile Leu Val Glu
                   605                 610                 615
   ggc agc cca gat gtc atg gct aag ctc gct aaa tgc tgt atg cca gtg      2646
   Gly Ser Pro Asp Val Met Ala Lys Leu Ala Lys Cys Cys Met Pro Val
               620                 625                 630
   cca gga gat gaa atc ttt gga ttc gtc acc cgt ggt ggc ggt gtc tcc      2694
   Pro Gly Asp Glu Ile Phe Gly Phe Val Thr Arg Gly Gly Gly Val Ser
           635                 640                 645
   gta cac cga aca gac tgc acg aat gtg gaa aag ctc aaa gaa gag cca      2742
   Val His Arg Thr Asp Cys Thr Asn Val Glu Lys Leu Lys Glu Glu Pro
       650                 655                 660
   gaa cgc att gtc tcc gtc tcc tgg gct tcg gaa ggt caa ggt tca gta      2790
   Glu Arg Ile Val Ser Val Ser Trp Ala Ser Glu Gly Gln Gly Ser Val
   665                 670                 675                 680
   ttc tct gcc aca ctg cag ctt gaa gca ctt gat cgc gca ggc ctg ctc      2838
   Phe Ser Ala Thr Leu Gln Leu Glu Ala Leu Asp Arg Ala Gly Leu Leu
                   685                 690                 695
   ttt gag ctc acc cgc gta atc aac gaa caa aag gtc tcc gtt acc gca      2886
   Phe Glu Leu Thr Arg Val Ile Asn Glu Gln Lys Val Ser Val Thr Ala
               700                 705                 710
   atg aac tcc cat tgc tca gaa gac cgc gta gcc acc gtg cgc ttc acc      2934
   Met Asn Ser His Cys Ser Glu Asp Arg Val Ala Thr Val Arg Phe Thr
           715                 720                 725
   ttt gcg gtc tct gac acc aag cag ttg gga tcc ctg atg aca cag ctg      2982
   Phe Ala Val Ser Asp Thr Lys Gln Leu Gly Ser Leu Met Thr Gln Leu
       730                 735                 740
   cgc aat gcc gaa gga gtg ttt gat gtc tac cga gtg acc tcg ggt ggc      3030
   Arg Asn Ala Glu Gly Val Phe Asp Val Tyr Arg Val Thr Ser Gly Gly
   745                 750                 755                 760
   tag aggcctttag attgtgaaaa agccttgggc ttcaaccacc gtgtgttgac           3083
   acggttttgg ggttggattt aacgctggaa attttactgc aatgaacgct ggaaagcagt   3143
   agcgaaggtc aaaccagcgc tgaggacgcc aacgatgcca gtaataacga cgacgtaatc   3203
   caaaatggtg tcagagtcaa tgtcagaaga tccgttggtg ctaccgtttc ttcttctacg   3263
   gtgtcagcca cgtacacaac ctgtgctggt gcgacttctt cagcctgtgc aggaacggag   3323
   atggaggttg ctgccattgc agcagctgtg ccgagtgcga cgaggggagt acgagaaaat   3383
   agcttcattg ctaaacccct taggtttaaa tattcgaata cgaaagttac tttatgtgca   3443
   ctttcaccat tgtgcaacta tatgtgcagg tcaaagctga tattttgcta agaaaagtgc   3503
   aaagcgattc gagctgacgc gcagcccata tcgtcaaaaa atcctttgtg aatggtgatt   3563
   cacaaaggat tagtcgcgta aggcggaaat tacttcagga aggaatccag cttggtactg   3623
   aaggttaaaa tggtggttag tgcaccgatg actgctgtga tgatcttgat ccagttttcg   3683
   atgttgtcag tgtcggaaga accgctggaa ggctcttcgc tggacgggtc ttcgttgaaa   3743
   tcaaagttaa cgtaaacttc tgcttcaacg cctgcttcgt tcttggcaat gatcttt      3800
<210> 8
   <211> 760
   <212> PRT
   <213> Corynebacterium glutamicum
<400> 8
   Met Ser Leu Glu Arg Asn Thr Gln Lys Ser Ser Met Gly Val Arg Ser
   1               5                   10                  15
   Met Ser Ala Arg Leu Ala Arg Ser Leu Thr Gly Asn Arg Val Arg Thr
               20                  25                  30
   Asn Pro Val Leu Asp Leu Leu Leu Ser Ile His Arg Gln Phe His Pro
   35 40 45
   Arg Ala Asp Val Gln Val Leu Glu Arg Ala Tyr Asp Thr Ala Glu Arg
   50 55 60
   Leu His Asp Gly Val Ile Arg Lys Ser Gly Asp Pro Tyr Ile Thr His
   65 70 75 80
   Pro Leu Ala Val Ala Thr Ile Ala Ala Glu Ile Gly Met Asp Thr Thr
   85 90 95
   Thr Leu Val Ala Ala Leu Leu His Asp Thr Val Glu Asp Thr Asp Tyr
   100 105 110
   Ser Leu Asp Asp Leu Thr Arg Asp Phe Gly Glu Glu Val Ala Arg Leu
   115 120 125
   Val Asp Gly Val Thr Lys Leu Asp Lys Val Ala Leu Gly Ala Ala Ala
   130 135 140
   Glu Ala Glu Thr Ile Arg Lys Met Ile Val Ala Met Ser Gln Asp Pro
   145 150 155 160
   Arg Val Leu Val Ile Lys Val Ala Asp Arg Leu His Asn Met Arg Thr
   165 170 175
   Met Arg Phe Leu Pro Pro Glu Lys Gln Ala Lys Lys Ala Arg Gln Thr
   180 185 190
   Leu Glu Val Ile Ala Pro Leu Ala His Arg Leu Gly Met Ala Ser Val
   195 200 205
   Lys Trp Glu Leu Glu Asp Leu Ser Phe Ala Ile Leu Tyr Pro Lys Lys
   210 215 220
   Tyr Glu Glu Ile Val Arg Leu Val Ala Asp Arg Ala Pro Ser Arg Asp
   225 230 235 240
   Arg Tyr Leu Lys Glu Ile Ile Asp Gln Val Thr Gly Gly Leu Arg Glu
   245 250 255
   Asn Asn Ile Ala Ala Gly Val Leu Gly Arg Pro Lys His Tyr Trp Ser
   260 265 270
   Ile Tyr Gln Lys Met Ile Val Arg Gly Arg Asp Phe Asp Asp Ile Phe
   275 280 285
   Asp Leu Val Gly Ile Arg Ile Leu Val Asp Asn Val Asn Asn Cys Tyr
       290                 295                 300
   Ala Ala Ile Gly Val Val His Ser Leu Phe Asn Ala Leu Pro Gly Arg
   305                 310                 315                 320
   Phe Lys Asp Tyr Ile Ser Ala Pro Arg Phe Gly Val Tyr Gln Ser Leu
                   325                 330                 335
   His Thr Thr Val Met Gly Pro Gly Gly Lys Pro Leu Glu Val Gln Ala
               340                 345                 350
   Arg Thr His Asp Met His Tyr Asn Ala Glu Phe Gly Ile Ala Ala His
           355                 360                 365
   Trp Arg Tyr Lys Glu Thr Lys Gly Ser His Ser Gly Glu Gln Ala Glu
       370                 375                 380
   Val Asp Gln Met Ala Trp Met Arg Gln Leu Leu Asp Trp Gln Lys Glu
   385                 390                 395                 400
   Ala Ala Asp Pro Asn Glu Phe Leu Asp Ser Leu Arg Tyr Asp Leu Thr
                   405                 410                 415
   Ser Lys Gln Ile Phe Val Phe Thr Pro Lys Gly Asp Val Val Asn Leu
               420                 425                 430
   Pro Val Asn Ser Thr Pro Val Asp Phe Ala Tyr Ala Val His Thr Glu
           435                 440                 445
   Val Gly His Arg Cys Ile Gly Ala Lys Ile Asn Gly Lys Leu Val Ala
       450                 455                 460
   Leu Glu Thr Lys Leu Lys Ser Gly Asp Arg Val Glu Val Phe Thr Ser
   465                 470                 475                 480
   Lys Asp Gln Asn Ala Gly Pro Ser Arg Gly Trp Gln Glu Phe Val Val
                   485                 490                 495
   Ser Pro Arg Ala Lys Ala Lys Ile Arg Gln Trp Phe Ala Lys Glu Arg
               500                 505                 510
   Arg Glu Glu Tyr Leu Glu Ala Gly Arg Asp Ala Leu Ala Ala Val Ile
           515                 520                 525
   Gln Arg Gly Gly Leu Pro Met His Arg Leu Phe Thr Ala Ser Ser Met
       530                 535                 540
   Lys Thr Val Ala Thr Glu Leu His Tyr Pro Asp Val Asp Ala Leu Tyr
   545                 550                 555                 560
   Thr Ala Ile Gly Ser Gly Ser Val Ser Ala Gln His Val Val Asn Arg
                   565                 570                 575
   Leu Met Ala Ile Phe Gly Asp Glu Glu Asp Ala Glu Asp Ala Leu Val
               580                 585                 590
   Ala Arg Thr Pro Phe Ser Glu Leu Val Asn Ser Arg Ala Thr Thr Glu
           595                 600                 605
   Ser Ser Thr Gly Ile Leu Val Glu Gly Ser Pro Asp Val Met Ala Lys
       610                 615                 620
   Leu Ala Lys Cys Cys Met Pro Val Pro Gly Asp Glu Ile Phe Gly Phe
   625                 630                 635                 640
   Val Thr Arg Gly Gly Gly Val Ser Val His Arg Thr Asp Cys Thr Asn
                   645                 650                 655
   Val Glu Lys Leu Lys Glu Glu Pro Glu Arg Ile Val Ser Val Ser Trp
               660                 665                 670
   Ala Ser Glu Gly Gln Gly Ser Val Phe Ser Ala Thr Leu Gln Leu Glu
           675                 680                 685
   Ala Leu Asp Arg Ala Gly Leu Leu Phe Glu Leu Thr Arg Val Ile Asn
       690                 695                 700
   Glu Gln Lys Val Ser Val Thr Ala Met Asn Ser His Cys Ser Glu Asp
   705                 710                 715                 720
   Arg Val Ala Thr Val Arg Phe Thr Phe Ala Val Ser Asp Thr Lys Gln
                   725                 730                 735
   Leu Gly Ser Leu Met Thr Gln Leu Arg Asn Ala Glu Gly Val Phe Asp
               740                 745                 750
   Val Tyr Arg Val Thr Ser Gly Gly
           755                 760
<210> 9
   <211> 1263
   <212> DNA
   <213> Corynebacterium glutamicum
<220>
   <221> CDS
   <222> (1)..(1263)
   <223> lysC-Wildtyp-Gen
<400> 9
   gtg gcc ctg gtc gta cag aaa tat ggc ggt tcc tcg ctt gag agt gcg        48
   Met Ala Leu Val Val Gln Lys Tyr Gly Gly Ser Ser Leu Glu Ser Ala
   1               5                   10                  15
   gaa cgc att aga aac gtc gct gaa cgg atc gtt gcc acc aag aag gct        96
   Glu Arg Ile Arg Asn Val Ala Glu Arg Ile Val Ala Thr Lys Lys Ala
               20                  25                  30
   gga aat gat gtc gtg gtt gtc tgc tcc gca atg gga gac acc acg gat       144
   Gly Asn Asp Val Val Val Val Cys Ser Ala Met Gly Asp Thr Thr Asp
           35                  40                  45
   gaa ctt cta gaa ctt gca gcg gca gtg aat ccc gtt ccg cca gct cgt       192
   Glu Leu Leu Glu Leu Ala Ala Ala Val Asn Pro Val Pro Pro Ala Arg
       50                  55                  60
   gaa atg gat atg ctc ctg act gct ggt gag cgt att tct aac gct ctc       240
   Glu Met Asp Met Leu Leu Thr Ala Gly Glu Arg Ile Ser Asn Ala Leu
   65                  70                  75                  80
   gtc gcc atg gct att gag tcc ctt ggc gca gaa gcc caa tct ttc acg       288
   Val Ala Met Ala Ile Glu Ser Leu Gly Ala Glu Ala Gln Ser Phe Thr
                   85                  90                  95
   ggc tct cag gct ggt gtg ctc acc acc gag cgc cac gga aac gca cgc       336
   Gly Ser Gln Ala Gly Val Leu Thr Thr Glu Arg His Gly Asn Ala Arg
               100                 105                 110
   att gtt gat gtc act cca ggt cgt gtg cgt gaa gca ctc gat gag ggc       384
   Ile Val Asp Val Thr Pro Gly Arg Val Arg Glu Ala Leu Asp Glu Gly
           115                 120                 125
   aag atc tgc att gtt gct ggt ttc cag ggt gtt aat aaa gaa acc cgc       432
   Lys Ile Cys Ile Val Ala Gly Phe Gln Gly Val Asn Lys Glu Thr Arg
       130                 135                 140
   gat gtc acc acg ttg ggt cgt ggt ggt tct gac acc act gca gtt gcg       480
   Asp Val Thr Thr Leu Gly Arg Gly Gly Ser Asp Thr Thr Ala Val Ala
   145                 150                 155                 160
   ttg gca gct gct ttg aac gct gat gtg tgt gag att tac tcg gac gtt       528
   Leu Ala Ala Ala Leu Asn Ala Asp Val Cys Glu Ile Tyr Ser Asp Val
                   165                 170                 175
   gac ggt gtg tat acc gct gac ccg cgc atc gtt cct aat gca cag aag       576
   Asp Gly Val Tyr Thr Ala Asp Pro Arg Ile Val Pro Asn Ala Gln Lys
               180                 185                 190
   ctg gaa aag ctc agc ttc gaa gaa atg ctg gaa ctt gct gct gtt ggc       624
   Leu Glu Lys Leu Ser Phe Glu Glu Met Leu Glu Leu Ala Ala Val Gly
           195                 200                 205
   tcc aag att ttg gtg ctg cgc agt gtt gaa tac gct cgt gca ttc aat       672
   Ser Lys Ile Leu Val Leu Arg Ser Val Glu Tyr Ala Arg Ala Phe Asn
       210                 215                 220
   gtg cca ctt cgc gta cgc tcg tct tat agt aat gat ccc ggc act ttg      720
   Val Pro Leu Arg Val Arg Ser Ser Tyr Ser Asn Asp Pro Gly Thr Leu
   225                 230                 235                 240
   att gcc ggc tct atg gag gat att cct gtg gaa gaa gca gtc ctt acc      768
   Ile Ala Gly Ser Met Glu Asp Ile Pro Val Glu Glu Ala Val Leu Thr
                   245                 250                 255
   ggt gtc gca acc gac aag tcc gaa gcc aaa gta acc gtt ctg ggt att      816
   Gly Val Ala Thr Asp Lys Ser Glu Ala Lys Val Thr Val Leu Gly Ile
               260                 265                 270
   tcc gat aag cca ggc gag gct gcg aag gtt ttc cgt gcg ttg gct gat      864
   Ser Asp Lys Pro Gly Glu Ala Ala Lys Val Phe Arg Ala Leu Ala Asp
           275                 280                 285
   gca gaa atc aac att gac atg gtt ctg cag aac gtc tct tct gta gaa      912
   Ala Glu Ile Asn Ile Asp Met Val Leu Gln Asn Val Ser Ser Val Glu
       290                 295                 300
   gac ggc acc acc gac atc acc ttc acc tgc cct cgt tcc gac ggc cgc      960
   Asp Gly Thr Thr Asp Ile Thr Phe Thr Cys Pro Arg Ser Asp Gly Arg
   305                 310                 315                 320
   cgc gcg atg gag atc ttg aag aag ctt cag gtt cag ggc aac tgg acc     1008
   Arg Ala Met Glu Ile Leu Lys Lys Leu Gln Val Gln Gly Asn Trp Thr
                   325                 330                 335
   aat gtg ctt tac gac gac cag gtc ggc aaa gtc tcc ctc gtg ggt gct     1056
   Asn Val Leu Tyr Asp Asp Gln Val Gly Lys Val Ser Leu Val Gly Ala
               340                 345                 350
   ggc atg aag tct cac cca ggt gtt acc gca gag ttc atg gaa gct ctg     1104
   Gly Met Lys Ser His Pro Gly Val Thr Ala Glu Phe Met Glu Ala Leu
           355                 360                 365
   cgc gat gtc aac gtg aac atc gaa ttg att tcc acc tct gag att cgt     1152
   Arg Asp Val Asn Val Asn Ile Glu Leu Ile Ser Thr Ser Glu Ile Arg
       370                 375                 380
   att tcc gtg ctg atc cgt gaa gat gat ctg gat gct gct gca cgt gca     1200
   Ile Ser Val Leu Ile Arg Glu Asp Asp Leu Asp Ala Ala Ala Arg Ala
   385                 390                 395                 400
   ttg cat gag cag ttc cag ctg ggc ggc gaa gac gaa gcc gtc gtt tat     1248
   Leu His Glu Gln Phe Gln Leu Gly Gly Glu Asp Glu Ala Val Val Tyr
                   405                 410                 415
   gca ggc acc gga cgc                                                 1263
   Ala Gly Thr Gly Arg
               420
<210> 10
   <211> 421
   <212> PRT
   <213> Corynebacterium glutamicum
<400> 10
   Met Ala Leu Val Val Gln Lys Tyr Gly Gly Ser Ser Leu Glu Ser Ala
   1               5                   10                  15
   Glu Arg Ile Arg Asn Val Ala Glu Arg Ile Val Ala Thr Lys Lys Ala
               20                  25                  30
   Gly Asn Asp Val Val Val Val Cys Ser Ala Met Gly Asp Thr Thr Asp
           35                  40                  45
   Glu Leu Leu Glu Leu Ala Ala Ala Val Asn Pro Val Pro Pro Ala Arg
       50                  55                  60
   Glu Met Asp Met Leu Leu Thr Ala Gly Glu Arg Ile Ser Asn Ala Leu
   65                  70                  75                  80
   Val Ala Met Ala Ile Glu Ser Leu Gly Ala Glu Ala Gln Ser Phe Thr
                   85                  90                  95
   Gly Ser Gln Ala Gly Val Leu Thr Thr Glu Arg His Gly Asn Ala Arg
               100                 105                 110
   Ile Val Asp Val Thr Pro Gly Arg Val Arg Glu Ala Leu Asp Glu Gly
           115                 120                 125
   Lys Ile Cys Ile Val Ala Gly Phe Gln Gly Val Asn Lys Glu Thr Arg
       130                 135                 140
   Asp Val Thr Thr Leu Gly Arg Gly Gly Ser Asp Thr Thr Ala Val Ala
   145                 150                 155                 160
   Leu Ala Ala Ala Leu Asn Ala Asp Val Cys Glu Ile Tyr Ser Asp Val
                   165                 170                 175
   Asp Gly Val Tyr Thr Ala Asp Pro Arg Ile Val Pro Asn Ala Gln Lys
               180                 185                 190
   Leu Glu Lys Leu Ser Phe Glu Glu Met Leu Glu Leu Ala Ala Val Gly
           195                 200                 205
   Ser Lys Ile Leu Val Leu Arg Ser Val Glu Tyr Ala Arg Ala Phe Asn
       210                 215                 220
   Val Pro Leu Arg Val Arg Ser Ser Tyr Ser Asn Asp Pro Gly Thr Leu
   225                 230                 235                 240
   Ile Ala Gly Ser Met Glu Asp Ile Pro Val Glu Glu Ala Val Leu Thr
                   245                 250                 255
   Gly Val Ala Thr Asp Lys Ser Glu Ala Lys Val Thr Val Leu Gly Ile
               260                 265                 270
   Ser Asp Lys Pro Gly Glu Ala Ala Lys Val Phe Arg Ala Leu Ala Asp
           275                 280                 285
   Ala Glu Ile Asn Ile Asp Met Val Leu Gln Asn Val Ser Ser Val Glu
       290                 295                 300
   Asp Gly Thr Thr Asp Ile Thr Phe Thr Cys Pro Arg Ser Asp Gly Arg
   305                 310                 315                 320
   Arg Ala Met Glu Ile Leu Lys Lys Leu Gln Val Gln Gly Asn Trp Thr
                   325                 330                 335
   Asn Val Leu Tyr Asp Asp Gln Val Gly Lys Val Ser Leu Val Gly Ala
               340                 345                 350
   Gly Met Lys Ser His Pro Gly Val Thr Ala Glu Phe Met Glu Ala Leu
           355                 360                 365
   Arg Asp Val Asn Val Asn Ile Glu Leu Ile Ser Thr Ser Glu Ile Arg
       370                 375                 380
   Ile Ser Val Leu Ile Arg Glu Asp Asp Leu Asp Ala Ala Ala Arg Ala
   385                 390                 395                 400
   Leu His Glu Gln Phe Gln Leu Gly Gly Glu Asp Glu Ala Val Val Tyr
                   405                 410                 415
   Ala Gly Thr Gly Arg
               420
<210> 11
   <211> 117
   <212> DNA
   <213> Corynebacterium glutamicum
<220>
   <221> CDS
   <222> (1)..(117)
<220>
   <221> misc_feature
   <222> (58)..(60)
   <223> n is a, c, g, or t
<400> 11
   gcc agg ctt gcc cgc agc ctc aca gga aac cgc gtt cgc acc aac cct        48
   Ala Arg Leu Ala Arg Ser Leu Thr Gly Asn Arg Val Arg Thr Asn Pro
   1               5                   10                  15
   gtg ctg gat nnn ctg ctg agc atc cac cgg caa ttt cac cca cgc gcc        96
   Val Leu Asp Xaa Leu Leu Ser Ile His Arg Gln Phe His Pro Arg Ala
               20                  25                  30
   gac gta caa gtg ttg gaa cgt                                        117
   Asp Val Gln Val Leu Glu Arg
           35
<210> 12
   <211> 39
   <212> PRT
   <213> Corynebacterium glutamicum
<220>
   <221> misc_feature
   <222> (20)..(20)
   <223> The 'Xaa' at location 20 stands for Lys, Asn, Arg, Ser, Thr, Ile, Met, Glu, Asp, Gly, Ala, Val, Gln, His, Leu, Tyr, Trp, Cys, or Phe.
<400> 12
   Ala Arg Leu Ala Arg Ser Leu Thr Gly Asn Arg Val Arg Thr Asn Pro
   1               5                   10                  15
   Val Leu Asp Xaa Leu Leu Ser Ile His Arg Gln Phe His Pro Arg Ala
               20                  25                  30
   Asp Val Gln Val Leu Glu Arg
           35
<210> 13
   <211> 117
   <212> DNA
   <213> Corynebacterium glutamicum
<220>
   <221> CDS
   <222> (1)..(117)
<400> 13
   gcc agg ctt gcc cgc agc ctc aca gga aac cgc gtt cgc acc aac cct     48
   Ala Arg Leu Ala Arg Ser Leu Thr Gly Asn Arg Val Arg Thr Asn Pro
   1               5                   10                  15
   gtg ctg gat ctg ctg ctg agc atc cac cgg caa ttt cac cca cgc gcc     96
   Val Leu Asp Leu Leu Leu Ser Ile His Arg Gln Phe His Pro Arg Ala
               20                  25                  30
   gac gta caa gtg ttg gaa cgt                                        117
   Asp Val Gln Val Leu Glu Arg
           35
<210> 14
   <211> 39
   <212> PRT
   <213> Corynebacterium glutamicum
<400> 14
   Ala Arg Leu Ala Arg Ser Leu Thr Gly Asn Arg Val Arg Thr Asn Pro
   1               5                   10                  15
   Val Leu Asp Leu Leu Leu Ser Ile His Arg Gln Phe His Pro Arg Ala
               20                  25                  30
   Asp Val Gln Val Leu Glu Arg
           35
<210> 15
   <211> 1530
   <212> DNA
   <213> Corynebacterium glutamicum
<220>
   <221> misc_feature
   <222> (1)..(2)
<220>
   <221> misc_structure
   <222> (3)..(8)
   <223> EcoRI-Restriktionsschnittstelle
<220>
   <221> CDS
   <222> (639)..(1520)
<220>
   <221> mutation
   <222> (751)..(751)
<220>
   <221> misc_feature
   <222> (1423)..(1423)
   <223> Guanin
<220>
   <221> misc_structure
   <222> (1523)..(1528)
   <223> EcoRI-Restriktionsschnittstelle
<220>
   <221> misc_feature
   <222> (1529)..(1530)
<400> 15
   gcgaattcta tcggatggaa catgaccgtt ggtccagaag tgaggaataa gtagtgagcg      60
   aacaagctct aagcaccttc gacagggcac gtgaggccct ggacaagaaa acccgatatg     120
   tgcaggattt cccagaaaag ggtgtgcttt ttgaagacct caccccggtg ttgggcgatg     180
   cagaatcatt tgtggccgtg gtggacgcca tggctgaagc tgcagaaaaa ctgaatgcag     240
   aaatcatcgg tggcttggat gcgcgaggat tcctcctcgg atctgctgtc gcttacaaac     300
   tcggcctagg tgtgctggct atccgcaaga agggaaagct ccccccacct gtggtgaccc     360
   aggagtatga acttgaatac ggcactgcag cactcgagct gcccagtgaa ggaatcgaca    420
   ttgctggtaa aaacatcgtt ttgatcgacg atgtgctggc aaccggcggc accttgggcg    480
   ctgcacgtaa actaattgaa tcgtgtgacg gacatgtttc cggatatgtt cttgccattg    540
   aggtcccagg cctcggcggt agggataatc ttggtgatag gcccgtcatt gtggtcagag    600
   atcctcagta gaaggatcga aagaaaggcg gcaggaaa atg agt ctg gag cgc aac    656
                                            Met Ser Leu Glu Arg Asn
                                            1               5
   aca caa aaa tct tcc atg ggt gtg cga agc atg tca gcc agg ctt gcc      704
   Thr Gln Lys Ser Ser Met Gly Val Arg Ser Met Ser Ala Arg Leu Ala
               10                  15                  20
   cgc agc ctc aca gga aac cgc gtt cgc acc aac cct gtg ctg gat ctg      752
   Arg Ser Leu Thr Gly Asn Arg Val Arg Thr Asn Pro Val Leu Asp Leu
           25                  30                  35
   ctg ctg agc atc cac cgg caa ttt cac cca cgc gcc gac gta caa gtg      800
   Leu Leu Ser Ile His Arg Gln Phe His Pro Arg Ala Asp Val Gln Val
       40                  45                  50
   ttg gaa cgt gca tat gac acc gcg gaa cgt ctt cat gat ggt gtg att      848
   Leu Glu Arg Ala Tyr Asp Thr Ala Glu Arg Leu His Asp Gly Val Ile
   55                  60                  65                  70
   cga aaa tcg ggc gat ccg tat att acc cac ccg ttg gct gtc gcc acc      896
   Arg Lys Ser Gly Asp Pro Tyr Ile Thr His Pro Leu Ala Val Ala Thr
                   75                  80                  85
   atc gcc gcg gaa atc ggc atg gac acc acc acg ctc gtc gca gcc ttg      944
   Ile Ala Ala Glu Ile Gly Met Asp Thr Thr Thr Leu Val Ala Ala Leu
               90                  95                  100
   ttg cat gac acg gtg gaa gac acc gac tac tct ttg gac gat ctc acc      992
   Leu His Asp Thr Val Glu Asp Thr Asp Tyr Ser Leu Asp Asp Leu Thr
           105                 110                 115
   cga gat ttc gga gaa gaa gtt gcc agg ctt gtc gac ggt gtc acc aag      1040
   Arg Asp Phe Gly Glu Glu Val Ala Arg Leu Val Asp Gly Val Thr Lys
       120                 125                 130
   ctc gac aaa gtc gca cta ggt gct gcc gcg gag gcc gaa acg att cgc      1088
   Leu Asp Lys Val Ala Leu Gly Ala Ala Ala Glu Ala Glu Thr Ile Arg
   135                 140                 145                 150
   aaa atg atc gtc gcc atg agc cag gac ccc cgc gtg ctg gtg att aaa      1136
   Lys Met Ile Val Ala Met Ser Gln Asp Pro Arg Val Leu Val Ile Lys
                   155                 160                 165
   gtg gcc gac cgt ttg cac aat atg cgc acc atg cgg ttc ctg ccg ccg      1184
   Val Ala Asp Arg Leu His Asn Met Arg Thr Met Arg Phe Leu Pro Pro
               170                 175                 180
   gaa aag caa gct aaa aaa gca cgc caa acc ctt gaa gtg att gct cct      1232
   Glu Lys Gln Ala Lys Lys Ala Arg Gln Thr Leu Glu Val Ile Ala Pro
           185                 190                 195
   ttg gca cac cgc ctg ggc atg gcc agc gtg aaa tgg gaa ttg gaa gat      1280
   Leu Ala His Arg Leu Gly Met Ala Ser Val Lys Trp Glu Leu Glu Asp
       200                 205                 210
   cta tcc ttt gcc att ttg tac ccc aag aag tac gaa gag atc gtg cgt      1328
   Leu Ser Phe Ala Ile Leu Tyr Pro Lys Lys Tyr Glu Glu Ile Val Arg
   215                 220                 225                 230
   ctt gtt gcc gac cgc gcg ccc tct aga gac cgg tac ctc aaa gaa att      1376
   Leu Val Ala Asp Arg Ala Pro Ser Arg Asp Arg Tyr Leu Lys Glu Ile
                   235                 240                 245
   att gat caa gtc acc ggt ggc ttg cgc gaa aac aac atc gcg gca gga      1424
   Ile Asp Gln Val Thr Gly Gly Leu Arg Glu Asn Asn Ile Ala Ala Gly
               250                 255                 260
   gtg ctt ggt cga cca aag cac tac tgg tct atc tat caa aag atg atc      1472
   Val Leu Gly Arg Pro Lys His Tyr Trp Ser Ile Tyr Gln Lys Met Ile
           265                 270                 275
   gtt cgc ggt cgt gat ttt gac gat att ttt gat ctt gtt ggc atc cgc      1520
   Val Arg Gly Arg Asp Phe Asp Asp Ile Phe Asp Leu Val Gly Ile Arg
       280                 285                 290
   atgaattcgc                                                           1530
<210> 16
   <211> 294
   <212> PRT
   <213> Corynebacterium glutamicum
<400> 16
   Met Ser Leu Glu Arg Asn Thr Gln Lys Ser Ser Met Gly Val Arg Ser
   1               5                   10                  15
   Met Ser Ala Arg Leu Ala Arg Ser Leu Thr Gly Asn Arg Val Arg Thr
               20                  25                  30
   Asn Pro Val Leu Asp Leu Leu Leu Ser Ile His Arg Gln Phe His Pro
           35                  40                  45
   Arg Ala Asp Val Gln Val Leu Glu Arg Ala Tyr Asp Thr Ala Glu Arg
       50                  55                  60
   Leu His Asp Gly Val Ile Arg Lys Ser Gly Asp Pro Tyr Ile Thr His
   65                  70                  75                  80
   Pro Leu Ala Val Ala Thr Ile Ala Ala Glu Ile Gly Met Asp Thr Thr
                   85                  90                  95
   Thr Leu Val Ala Ala Leu Leu His Asp Thr Val Glu Asp Thr Asp Tyr
               100                 105                 110
   Ser Leu Asp Asp Leu Thr Arg Asp Phe Gly Glu Glu Val Ala Arg Leu
           115                 120                 125
   Val Asp Gly Val Thr Lys Leu Asp Lys Val Ala Leu Gly Ala Ala Ala
       130                 135                 140
   Glu Ala Glu Thr Ile Arg Lys Met Ile Val Ala Met Ser Gln Asp Pro
   145                 150                 155                 160
   Arg Val Leu Val Ile Lys Val Ala Asp Arg Leu His Asn Met Arg Thr
                   165                 170                 175
   Met Arg Phe Leu Pro Pro Glu Lys Gln Ala Lys Lys Ala Arg Gln Thr
               180                 185                 190
   Leu Glu Val Ile Ala Pro Leu Ala His Arg Leu Gly Met Ala Ser Val
           195                 200                 205
   Lys Trp Glu Leu Glu Asp Leu Ser Phe Ala Ile Leu Tyr Pro Lys Lys
       210                 215                 220
   Tyr Glu Glu Ile Val Arg Leu Val Ala Asp Arg Ala Pro Ser Arg Asp
   225                 230                 235                 240
   Arg Tyr Leu Lys Glu Ile Ile Asp Gln Val Thr Gly Gly Leu Arg Glu
                   245                 250                 255
   Asn Asn Ile Ala Ala Gly Val Leu Gly Arg Pro Lys His Tyr Trp Ser
               260                 265                 270
   Ile Tyr Gln Lys Met Ile Val Arg Gly Arg Asp Phe Asp Asp Ile Phe
           275                 280                 285
   Asp Leu Val Gly Ile Arg
       290
<210> 17
   <211> 1311
   <212> DNA
   <213> Corynebacterium glutamicum
<220>
   <221> CDS
   <222> (1)..(1308)
   <223> ilvA-Wildtyp-Gen
<400> 17
   atg agt gaa aca tac gtg tct gag aaa agt cca gga gtg atg gct agc        48
   Met Ser Glu Thr Tyr Val Ser Glu Lys Ser Pro Gly Val Met Ala Ser
   1               5                   10                  15
   gga gcg gag ctg att cgt gcc gcc gac att caa acg gcg cag gca cga        96
   Gly Ala Glu Leu Ile Arg Ala Ala Asp Ile Gln Thr Ala Gln Ala Arg
                   20                  25                  30
   att tcc tcc gtc att gca cca act cca ttg cag tat tgc cct cgt ctt      144
   Ile Ser Ser Val Ile Ala Pro Thr Pro Leu Gln Tyr Cys Pro Arg Leu
           35                  40                  45
   tct gag gaa acc gga gcg gaa atc tac ctt aag cgt gag gat ctg cag      192
   Ser Glu Glu Thr Gly Ala Glu Ile Tyr Leu Lys Arg Glu Asp Leu Gln
       50                  55                  60
   gat gtt cgt tcc tac aag atc cgc ggt gcg ctg aac tct gga gcg cag      240
   Asp Val Arg Ser Tyr Lys Ile Arg Gly Ala Leu Asn Ser Gly Ala Gln
   65                  70                  75                  80
   ctc acc caa gag cag cgc gat gca ggt atc gtt gcc gca tct gca ggt      288
   Leu Thr Gln Glu Gln Arg Asp Ala Gly Ile Val Ala Ala Ser Ala Gly
                   85                  90                  95
   aac cat gcc cag ggc gtg gcc tat gtg tgc aag tcc ttg ggc gtt cag      336
   Asn His Ala Gln Gly Val Ala Tyr Val Cys Lys Ser Leu Gly Val Gln
               100                 105                 110
   gga cgc atc tat gtt cct gtg cag act cca aag caa aag cgt gac cgc      384
   Gly Arg Ile Tyr Val Pro Val Gln Thr Pro Lys Gln Lys Arg Asp Arg
           115                 120                 125
   atc atg gtt cac ggc gga gag ttt gtc tcc ttg gtg gtc act ggc aat      432
   Ile Met Val His Gly Gly Glu Phe Val Ser Leu Val Val Thr Gly Asn
       130                 135                 140
   aac ttc gac gaa gca tcg gct gca gcg cat gaa gat gca gag cgc acc      480
   Asn Phe Asp Glu Ala Ser Ala Ala Ala His Glu Asp Ala Glu Arg Thr
   145                 150                 155                 160
   ggc gca acg ctg atc gag cct ttc gat gct cgc aac acc gtc atc ggt      528
   Gly Ala Thr Leu Ile Glu Pro Phe Asp Ala Arg Asn Thr Val Ile Gly
                   165                 170                 175
   cag ggc acc gtg gct gct gag atc ttg tcg cag ctg act tcc atg ggc      576
   Gln Gly Thr Val Ala Ala Glu Ile Leu Ser Gln Leu Thr Ser Met Gly
               180                 185                 190
   aag agt gca gat cac gtg atg gtt cca gtc ggc ggt ggc gga ctt ctt      624
   Lys Ser Ala Asp His Val Met Val Pro Val Gly Gly Gly Gly Leu Leu
           195                 200                 205
   gca ggt gtg gtc agc tac atg gct gat atg gca cct cgc act gcg atc      672
   Ala Gly Val Val Ser Tyr Met Ala Asp Met Ala Pro Arg Thr Ala Ile
       210                 215                 220
   gtt ggt atc gaa cca gcg gga gca gca tcc atg cag gct gca ttg cac      720
   Val Gly Ile Glu Pro Ala Gly Ala Ala Ser Met Gln Ala Ala Leu His
   225                 230                 235                 240
   aat ggt gga cca atc act ttg gag act gtt gat ccc ttt gtg gac ggc      768
   Asn Gly Gly Pro Ile Thr Leu Glu Thr Val Asp Pro Phe Val Asp Gly
                   245                 250                 255
   gca gca gtc aaa cgt gtc gga gat ctc aac tac acc atc gtg gag aag      816
   Ala Ala Val Lys Arg Val Gly Asp Leu Asn Tyr Thr Ile Val Glu Lys
               260                 265                 270
   aac cag ggt cgc gtg cac atg atg agc gcg acc gag ggc gct gtg tgt      864
   Asn Gln Gly Arg Val His Met Met Ser Ala Thr Glu Gly Ala Val Cys
           275                 280                 285
   act gag atg ctc gat ctt tac caa aac gaa ggc atc atc gcg gag cct      912
   Thr Glu Met Leu Asp Leu Tyr Gln Asn Glu Gly Ile Ile Ala Glu Pro
       290                 295                 300
   gct ggc gcg ctg tct atc gct ggg ttg aag gaa atg tcc ttt gca cct      960
   Ala Gly Ala Leu Ser Ile Ala Gly Leu Lys Glu Met Ser Phe Ala Pro
   305                 310                 315                 320
   ggt tct gtc gtg gtg tgc atc atc tct ggt ggc aac aac gat gtg ctg     1008
   Gly Ser Val Val Val Cys Ile Ile Ser Gly Gly Asn Asn Asp Val Leu
                   325                 330                 335
   cgt tat gcg gaa atc gct gag cgc tcc ttg gtg cac cgc ggt ttg aag     1056
   Arg Tyr Ala Glu Ile Ala Glu Arg Ser Leu Val His Arg Gly Leu Lys
               340                 345                 350
   cac tac ttc ttg gtg aac ttc ccg caa aag cct ggt cag ttg cgt cac     1104
   His Tyr Phe Leu Val Asn Phe Pro Gln Lys Pro Gly Gln Leu Arg His
           355                 360                 365
   ttc ctg gaa gat atc ctg gga ccg gat gat gac atc acg ctg ttt gag     1152
   Phe Leu Glu Asp Ile Leu Gly Pro Asp Asp Asp Ile Thr Leu Phe Glu
       370                 375                 380
   tac ctc aag cgc aac aac cgt gag acc ggt act gcg ttg gtg ggt att     1200
   Tyr Leu Lys Arg Asn Asn Arg Glu Thr Gly Thr Ala Leu Val Gly Ile
   385                 390                 395                 400
   cac ttg agt gaa gca tca gga ttg gat tct ttg ctg gaa cgt atg gag     1248
   His Leu Ser Glu Ala Ser Gly Leu Asp Ser Leu Leu Glu Arg Met Glu
                   405                 410                 415
   gaa tcg gca att gat tcc cgt cgc ctc gag ccg ggc acc cct gag tac     1296
   Glu Ser Ala Ile Asp Ser Arg Arg Leu Glu Pro Gly Thr Pro Glu Tyr
               420                 425                 430
   gaa tac ttg acc taa                                                 1311
   Glu Tyr Leu Thr
           435
<210> 18
   <211> 436
   <212> PRT
   <213> Corynebacterium glutamicum
<400> 18
   Met Ser Glu Thr Tyr Val Ser Glu Lys Ser Pro Gly Val Met Ala Ser
   1               5                   10                  15
   Gly Ala Glu Leu Ile Arg Ala Ala Asp Ile Gln Thr Ala Gln Ala Arg
               20                  25                  30
   Ile Ser Ser Val Ile Ala Pro Thr Pro Leu Gln Tyr Cys Pro Arg Leu
           35                  40                  45
   Ser Glu Glu Thr Gly Ala Glu Ile Tyr Leu Lys Arg Glu Asp Leu Gln
       50                  55                  60
   Asp Val Arg Ser Tyr Lys Ile Arg Gly Ala Leu Asn Ser Gly Ala Gln
   65                  70                  75                  80
   Leu Thr Gln Glu Gln Arg Asp Ala Gly Ile Val Ala Ala Ser Ala Gly
                   85                  90                  95
   Asn His Ala Gln Gly Val Ala Tyr Val Cys Lys Ser Leu Gly Val Gln
               100                 105                 110
   Gly Arg Ile Tyr Val Pro Val Gln Thr Pro Lys Gln Lys Arg Asp Arg
           115                 120                 125
   Ile Met Val His Gly Gly Glu Phe Val Ser Leu Val Val Thr Gly Asn
       130                 135                 140
   Asn Phe Asp Glu Ala Ser Ala Ala Ala His Glu Asp Ala Glu Arg Thr
   145                 150                 155                 160
   Gly Ala Thr Leu Ile Glu Pro Phe Asp Ala Arg Asn Thr Val Ile Gly
                   165                 170                 175
   Gln Gly Thr Val Ala Ala Glu Ile Leu Ser Gln Leu Thr Ser Met Gly
               180                 185                 190
   Lys Ser Ala Asp His Val Met Val Pro Val Gly Gly Gly Gly Leu Leu
           195                 200                 205
   Ala Gly Val Val Ser Tyr Met Ala Asp Met Ala Pro Arg Thr Ala Ile
       210                 215                 220
   Val Gly Ile Glu Pro Ala Gly Ala Ala Ser Met Gln Ala Ala Leu His
   225                 230                 235                 240
   Asn Gly Gly Pro Ile Thr Leu Glu Thr Val Asp Pro Phe Val Asp Gly
                   245                 250                 255
   Ala Ala Val Lys Arg Val Gly Asp Leu Asn Tyr Thr Ile Val Glu Lys
               260                 265                 270
   Asn Gln Gly Arg Val His Met Met Ser Ala Thr Glu Gly Ala Val Cys
           275                 280                 285
   Thr Glu Met Leu Asp Leu Tyr Gln Asn Glu Gly Ile Ile Ala Glu Pro
       290                 295                 300
   Ala Gly Ala Leu Ser Ile Ala Gly Leu Lys Glu Met Ser Phe Ala Pro
   305                 310                 315                 320
   Gly Ser Val Val Val Cys Ile Ile Ser Gly Gly Asn Asn Asp Val Leu
                   325                 330                 335
   Arg Tyr Ala Glu Ile Ala Glu Arg Ser Leu Val His Arg Gly Leu Lys
               340                 345                 350
   His Tyr Phe Leu Val Asn Phe Pro Gln Lys Pro Gly Gln Leu Arg His
           355                 360                 365
   Phe Leu Glu Asp Ile Leu Gly Pro Asp Asp Asp Ile Thr Leu Phe Glu
       370                 375                 380
   Tyr Leu Lys Arg Asn Asn Arg Glu Thr Gly Thr Ala Leu Val Gly Ile
   385                 390                 395                 400
   His Leu Ser Glu Ala Ser Gly Leu Asp Ser Leu Leu Glu Arg Met Glu
                   405                 410                 415
   Glu Ser Ala Ile Asp Ser Arg Arg Leu Glu Pro Gly Thr Pro Glu Tyr
               420                 425                 430
   Glu Tyr Leu Thr
           435
<210> 19
   <211> 28
   <212> DNA
   <213> Artificial Sequence
<220>
   <223> Primer rel_XL_A1
<220>
   <221> misc_feature
   <222> (1)..(2)
<220>
   <221> misc_structure
   <222> (3)..(8)
   <223> EcoRI-Restriktionsschnittstelle
<400> 19
   gcgaattcta tcggatggaa catgaccg 28
<210> 20
   <211> 28
   <212> DNA
   <213> Artificial Sequence
<220>
   <223> Primer rel_XL_E1
<220>
   <221> misc_feature
   <222> (1)..(2)
<220>
   <221> misc_structure
   <222> (3)..(8)
   <223> EcoRI-Restriktionsschnittstelle
<400> 20
   gcgaattcat gcggatgcca acaagatc 28
<210> 21
   <211> 2283
   <212> DNA
   <213> Corynebacterium glutamicum
<220>
   <221> CDS
   <222> (1)..(2280)
   <223> rel-Wildtyp-Gen gemäß EP 1 108 790
<220>
   <221> misc_feature
   <222> (785)..(785)
   <223> Adenin
<400> 21
   atg agt ctg gag cgc aac aca caa aaa tct tcc atg ggt gtg cga agc      48
   Met Ser Leu Glu Arg Asn Thr Gln Lys Ser Ser Met Gly Val Arg Ser
   1               5                   10                  15
   atg tca gcc agg ctt gcc cgc agc ctc aca gga aac cgc gtt cgc acc      96
   Met Ser Ala Arg Leu Ala Arg Ser Leu Thr Gly Asn Arg Val Arg Thr
               20                  25                  30
   aac cct gtg ctg gat ccg ctg ctg agc atc cac cgg caa ttt cac cca     144
   Asn Pro Val Leu Asp Pro Leu Leu Ser Ile His Arg Gln Phe His Pro
           35                  40                  45
   cgc gcc gac gta caa gtg ttg gaa cgt gca tat gac acc gcg gaa cgt     192
   Arg Ala Asp Val Gln Val Leu Glu Arg Ala Tyr Asp Thr Ala Glu Arg
       50                  55                  60
   ctt cat gat ggt gtg att cga aaa tcg ggc gat ccg tat att acc cac     240
   Leu His Asp Gly Val Ile Arg Lys Ser Gly Asp Pro Tyr Ile Thr His
   65                  70                  75                  80
   ccg ttg gct gtc gcc acc atc gcc gcg gaa atc ggc atg gac acc acc     288
   Pro Leu Ala Val Ala Thr Ile Ala Ala Glu Ile Gly Met Asp Thr Thr
                   85                  90                  95
   acg ctc gtc gca gcc ttg ttg cat gac acg gtg gaa gac acc gac tac     336
   Thr Leu Val Ala Ala Leu Leu His Asp Thr Val Glu Asp Thr Asp Tyr
               100                 105                 110
   tct ttg gac gat ctc acc cga gat ttc gga gaa gaa gtt gcc agg ctt      384
   Ser Leu Asp Asp Leu Thr Arg Asp Phe Gly Glu Glu Val Ala Arg Leu
           115                 120                 125
   gtc gac ggt gtc acc aag ctc gac aaa gtc gca cta ggt gct gcc gcg      432
   Val Asp Gly Val Thr Lys Leu Asp Lys Val Ala Leu Gly Ala Ala Ala
       130                 135                 140
   gag gcc gaa acg att cgc aaa atg atc gtc gcc atg agc cag gac ccc      480
   Glu Ala Glu Thr Ile Arg Lys Met Ile Val Ala Met Ser Gln Asp Pro
   145                 150                 155                 160
   cgc gtg ctg gtg att aaa gtg gcc gac cgt ttg cac aat atg cgc acc      528
   Arg Val Leu Val Ile Lys Val Ala Asp Arg Leu His Asn Met Arg Thr
                   165                 170                 175
   atg cgg ttc ctg ccg ccg gaa aag caa gct aaa aaa gca cgc caa acc      576
   Met Arg Phe Leu Pro Pro Glu Lys Gln Ala Lys Lys Ala Arg Gln Thr
               180                 185                 190
   ctt gaa gtg att gct cct ttg gca cac cgc ctg ggc atg gcc agc gtg      624
   Leu Glu Val Ile Ala Pro Leu Ala His Arg Leu Gly Met Ala Ser Val
           195                 200                 205
   aaa tgg gaa ttg gaa gat cta tcc ttt gcc att ttg tac ccc aag aag      672
   Lys Trp Glu Leu Glu Asp Leu Ser Phe Ala Ile Leu Tyr Pro Lys Lys
       210                 215                 220
   tac gaa gag atc gtg cgt ctt gtt gcc gac cgc gcg ccc tct aga gac      720
   Tyr Glu Glu Ile Val Arg Leu Val Ala Asp Arg Ala Pro Ser Arg Asp
   225                 230                 235                 240
   cgg tac ctc aaa gaa att att gat caa gtc acc ggt ggc ttg cgc gaa      768
   Arg Tyr Leu Lys Glu Ile Ile Asp Gln Val Thr Gly Gly Leu Arg Glu
                   245                 250                 255
   aac aac atc gcg gca gaa gtg ctt ggt cga cca aag cac tac tgg tct      816
   Asn Asn Ile Ala Ala Glu Val Leu Gly Arg Pro Lys His Tyr Trp Ser
               260                 265                 270
   atc tat caa aag atg atc gtt cgc ggt cgt gat ttt gac gat att ttt      864
   Ile Tyr Gln Lys Met Ile Val Arg Gly Arg Asp Phe Asp Asp Ile Phe
           275                 280                 285
   gat ctt gtt ggc atc cgc atc ctg gta gac aac gtg aac aac tgt tac      912
   Asp Leu Val Gly Ile Arg Ile Leu Val Asp Asn Val Asn Asn Cys Tyr
       290                 295                 300
   gcc gcc atc ggt gtc gtg cac tcc ctg ttc aat gct ctg cct ggc cga      960
   Ala Ala Ile Gly Val Val His Ser Leu Phe Asn Ala Leu Pro Gly Arg
   305                 310                 315                 320
   ttc aaa gac tat att tca gcc ccg cgc ttc ggt gtc tac caa tcc ctg     1008
   Phe Lys Asp Tyr Ile Ser Ala Pro Arg Phe Gly Val Tyr Gln Ser Leu
                   325                 330                 335
   cac acc acc gtg atg gga cct ggc ggt aag cct ctg gaa gtt cag gca     1056
   His Thr Thr Val Met Gly Pro Gly Gly Lys Pro Leu Glu Val Gln Ala
               340                 345                 350
   cgt acc cac gac atg cac tac aac gcc gaa ttc ggc att gca gcg cac     1104
   Arg Thr His Asp Met His Tyr Asn Ala Glu Phe Gly Ile Ala Ala His
           355                 360                 365
   tgg cga tac aaa gaa acc aaa ggc agc cac agt ggc gag caa gcc gaa      1152
   Trp Arg Tyr Lys Glu Thr Lys Gly Ser His Ser Gly Glu Gln Ala Glu
       370                 375                 380
   gtg gat caa atg gcg tgg atg cgc caa ctt ctg gac tgg caa aaa gaa      1200
   Val Asp Gln Met Ala Trp Met Arg Gln Leu Leu Asp Trp Gln Lys Glu
   385                 390                 395                 400
   gca gcc gac ccc aac gag ttc ctg gac agc ctg cgc tac gat ctg act      1248
   Ala Ala Asp Pro Asn Glu Phe Leu Asp Ser Leu Arg Tyr Asp Leu Thr
                   405                 410                 415
   tcc aag cag atc ttc gtg ttc aca ccc aaa ggt gat gtg gtc aac ctg      1296
   Ser Lys Gln Ile Phe Val Phe Thr Pro Lys Gly Asp Val Val Asn Leu
               420                 425                 430
   ccg gtg aac tcc acc ccg gtg gac ttc gcc tac gcg gtg cac acc gaa      1344
   Pro Val Asn Ser Thr Pro Val Asp Phe Ala Tyr Ala Val His Thr Glu
           435                 440                 445
   gtg ggg cac cgc tgc atc ggc gcc aaa atc aac ggc aaa ctg gtc gct      1392
   Val Gly His Arg Cys Ile Gly Ala Lys Ile Asn Gly Lys Leu Val Ala
       450                 455                 460
   ttg gaa acg aaa ctc aaa tcc ggc gat cgt gtt gaa gtc ttt acc tcc      1440
   Leu Glu Thr Lys Leu Lys Ser Gly Asp Arg Val Glu Val Phe Thr Ser
   465                 470                 475                 480
   aag gac caa aac gct ggc cca agt agg gga tgg caa gaa ttt gtt gtc      1488
   Lys Asp Gln Asn Ala Gly Pro Ser Arg Gly Trp Gln Glu Phe Val Val
                   485                 490                 495
   tca cct cgt gca aag gcc aag att cgc cag tgg ttt gcc aag gaa cga      1536
   Ser Pro Arg Ala Lys Ala Lys Ile Arg Gln Trp Phe Ala Lys Glu Arg
               500                 505                 510
   cgc gaa gaa tac cta gaa gcc gga cgc gat gcg ctg gca gca gtt att      1584
   Arg Glu Glu Tyr Leu Glu Ala Gly Arg Asp Ala Leu Ala Ala Val Ile
           515                 520                 525
   cag cgt ggc ggc ctg cca atg cac cgc ttg ttc acc gcg tcc tcc atg      1632
   Gln Arg Gly Gly Leu Pro Met His Arg Leu Phe Thr Ala Ser Ser Met
       530                 535                 540
   aag acg gtg gca aca gag ctg cac tac cca gat gta gat gcg ctc tac      1680
   Lys Thr Val Ala Thr Glu Leu His Tyr Pro Asp Val Asp Ala Leu Tyr
   545                 550                 555                 560
   aca gcc atc ggc tcc ggt tct gta tct gcg caa cac gta gtc aac cgt      1728
   Thr Ala Ile Gly Ser Gly Ser Val Ser Ala Gln His Val Val Asn Arg
                   565                 570                 575
   ctc atg gct atc ttt ggt gac gaa gaa gat gcc gaa gac gca ttg gtt      1776
   Leu Met Ala Ile Phe Gly Asp Glu Glu Asp Ala Glu Asp Ala Leu Val
               580                 585                 590
   gca cgc acc cca ttc agc gag ctg gtc aac tcc cgt gcc acc acg gaa      1824
   Ala Arg Thr Pro Phe Ser Glu Leu Val Asn Ser Arg Ala Thr Thr Glu
           595                 600                 605
   agc agc acc ggc atc ctg gtc gaa ggc agc cca gat gtc atg gct aag      1872
   Ser Ser Thr Gly Ile Leu Val Glu Gly Ser Pro Asp Val Met Ala Lys
       610                 615                 620
   ctc gct aaa tgc tgt atg cca gtg cca gga gat gaa atc ttt gga ttc      1920
   Leu Ala Lys Cys Cys Met Pro Val Pro Gly Asp Glu Ile Phe Gly Phe
   625                 630                 635                 640
   gtc acc cgt ggt ggc ggt gtc tcc gta cac cga aca gac tgc acg aat      1968
   Val Thr Arg Gly Gly Gly Val Ser Val His Arg Thr Asp Cys Thr Asn
                   645                 650                 655
   gtg gaa aag ctc aaa gaa gag cca gaa cgc att gtc tcc gtc tcc tgg      2016
   Val Glu Lys Leu Lys Glu Glu Pro Glu Arg Ile Val Ser Val Ser Trp
               660                 665                 670
   gct tcg gaa ggt caa ggt tca gta ttc tct gcc aca ctg cag ctt gaa      2064
   Ala Ser Glu Gly Gln Gly Ser Val Phe Ser Ala Thr Leu Gln Leu Glu
           675                 680                 685
   gca ctt gat cgc gca ggc ctg ctc ttt gag ctc acc cgc gta atc aac      2112
   Ala Leu Asp Arg Ala Gly Leu Leu Phe Glu Leu Thr Arg Val Ile Asn
       690                 695                 700
   gaa caa aag gtc tcc gtt acc gca atg aac tcc cat tgc tca gaa gac      2160
   Glu Gln Lys Val Ser Val Thr Ala Met Asn Ser His Cys Ser Glu Asp
   705                 710                 715                 720
   cgc gta gcc acc gtg cgc ttc acc ttt gcg gtc tct gac acc aag cag      2208
   Arg Val Ala Thr Val Arg Phe Thr Phe Ala Val Ser Asp Thr Lys Gln
                   725                 730                 735
   ttg gga tcc ctg atg aca cag ctg cgc aat gcc gaa gga gtg ttt gat      2256
   Leu Gly Ser Leu Met Thr Gln Leu Arg Asn Ala Glu Gly Val Phe Asp
               740                 745                 750
   gtc tac cga gtg acc tcg ggt ggc tag                                  2283
   Val Tyr Arg Val Thr Ser Gly Gly
           755                 760
<210> 22
   <211> 760
   <212> PRT
   <213> Corynebacterium glutamicum
<400> 22
   Met Ser Leu Glu Arg Asn Thr Gln Lys Ser Ser Met Gly Val Arg Ser
   1               5                   10                  15
   Met Ser Ala Arg Leu Ala Arg Ser Leu Thr Gly Asn Arg Val Arg Thr
               20                  25                  30
   Asn Pro Val Leu Asp Pro Leu Leu Ser Ile His Arg Gln Phe His Pro
           35                  40                  45
   Arg Ala Asp Val Gln Val Leu Glu Arg Ala Tyr Asp Thr Ala Glu Arg
       50                  55                  60
   Leu His Asp Gly Val Ile Arg Lys Ser Gly Asp Pro Tyr Ile Thr His
   65                  70                  75                  80
   Pro Leu Ala Val Ala Thr Ile Ala Ala Glu Ile Gly Met Asp Thr Thr
                   85                  90                  95
   Thr Leu Val Ala Ala Leu Leu His Asp Thr Val Glu Asp Thr Asp Tyr
               100                 105                 110
   Ser Leu Asp Asp Leu Thr Arg Asp Phe Gly Glu Glu Val Ala Arg Leu
           115                 120                 125
   Val Asp Gly Val Thr Lys Leu Asp Lys Val Ala Leu Gly Ala Ala Ala
       130                 135                 140
   Glu Ala Glu Thr Ile Arg Lys Met Ile Val Ala Met Ser Gln Asp Pro
   145                 150                 155                 160
   Arg Val Leu Val Ile Lys Val Ala Asp Arg Leu His Asn Met Arg Thr
                   165                 170                 175
   Met Arg Phe Leu Pro Pro Glu Lys Gln Ala Lys Lys Ala Arg Gln Thr
               180                 185                 190
   Leu Glu Val Ile Ala Pro Leu Ala His Arg Leu Gly Met Ala Ser Val
           195                 200                 205
   Lys Trp Glu Leu Glu Asp Leu Ser Phe Ala Ile Leu Tyr Pro Lys Lys
       210                 215                 220
   Tyr Glu Glu Ile Val Arg Leu Val Ala Asp Arg Ala Pro Ser Arg Asp
   225                 230                 235                 240
   Arg Tyr Leu Lys Glu Ile Ile Asp Gln Val Thr Gly Gly Leu Arg Glu
                   245                 250                 255
   Asn Asn Ile Ala Ala Glu Val Leu Gly Arg Pro Lys His Tyr Trp Ser
               260                 265                 270
   Ile Tyr Gln Lys Met Ile Val Arg Gly Arg Asp Phe Asp Asp Ile Phe
           275                 280                 285
   Asp Leu Val Gly Ile Arg Ile Leu Val Asp Asn Val Asn Asn Cys Tyr
       290                 295                 300
   Ala Ala Ile Gly Val Val His Ser Leu Phe Asn Ala Leu Pro Gly Arg
   305                 310                 315                 320
   Phe Lys Asp Tyr Ile Ser Ala Pro Arg Phe Gly Val Tyr Gln Ser Leu
                   325                 330                 335
   His Thr Thr Val Met Gly Pro Gly Gly Lys Pro Leu Glu Val Gln Ala
               340                 345                 350
   Arg Thr His Asp Met His Tyr Asn Ala Glu Phe Gly Ile Ala Ala His
           355                 360                 365
   Trp Arg Tyr Lys Glu Thr Lys Gly Ser His Ser Gly Glu Gln Ala Glu
       370                 375                 380
   Val Asp Gln Met Ala Trp Met Arg Gln Leu Leu Asp Trp Gln Lys Glu
   385                 390                 395                 400
   Ala Ala Asp Pro Asn Glu Phe Leu Asp Ser Leu Arg Tyr Asp Leu Thr
                   405                 410                 415
   Ser Lys Gln Ile Phe Val Phe Thr Pro Lys Gly Asp Val Val Asn Leu
               420                 425                 430
   Pro Val Asn Ser Thr Pro Val Asp Phe Ala Tyr Ala Val His Thr Glu
           435                 440                 445
   Val Gly His Arg Cys Ile Gly Ala Lys Ile Asn Gly Lys Leu Val Ala
       450                 455                 460
   Leu Glu Thr Lys Leu Lys Ser Gly Asp Arg Val Glu Val Phe Thr Ser
   465                 470                 475                 480
   Lys Asp Gln Asn Ala Gly Pro Ser Arg Gly Trp Gln Glu Phe Val Val
                   485                 490                 495
   Ser Pro Arg Ala Lys Ala Lys Ile Arg Gln Trp Phe Ala Lys Glu Arg
               500                 505                 510
   Arg Glu Glu Tyr Leu Glu Ala Gly Arg Asp Ala Leu Ala Ala Val Ile
           515                 520                 525
   Gln Arg Gly Gly Leu Pro Met His Arg Leu Phe Thr Ala Ser Ser Met
       530                 535                 540
   Lys Thr Val Ala Thr Glu Leu His Tyr Pro Asp Val Asp Ala Leu Tyr
   545                 550                 555                 560
   Thr Ala Ile Gly Ser Gly Ser Val Ser Ala Gln His Val Val Asn Arg
                   565                 570                 575
   Leu Met Ala Ile Phe Gly Asp Glu Glu Asp Ala Glu Asp Ala Leu Val
                   580                 585                 590
   Ala Arg Thr Pro Phe Ser Glu Leu Val Asn Ser Arg Ala Thr Thr Glu
           595                 600                 605
   Ser Ser Thr Gly Ile Leu Val Glu Gly Ser Pro Asp Val Met Ala Lys
       610                 615                 620
   Leu Ala Lys Cys Cys Met Pro Val Pro Gly Asp Glu Ile Phe Gly Phe
   625                 630                 635                 640
   Val Thr Arg Gly Gly Gly Val Ser Val His Arg Thr Asp Cys Thr Asn
               645                 650                 655
   Val Glu Lys Leu Lys Glu Glu Pro Glu Arg Ile Val Ser Val Ser Trp
           660                 665                 670
   Ala Ser Glu Gly Gln Gly Ser Val Phe Ser Ala Thr Leu Gln Leu Glu
           675                 680                 685
   Ala Leu Asp Arg Ala Gly Leu Leu Phe Glu Leu Thr Arg Val Ile Asn
       690                 695                 700
   Glu Gln Lys Val Ser Val Thr Ala Met Asn Ser His Cys Ser Glu Asp
   705                 710                 715                 720
   Arg Val Ala Thr Val Arg Phe Thr Phe Ala Val Ser Asp Thr Lys Gln
                   725                 730                 735
   Leu Gly Ser Leu Met Thr Gln Leu Arg Asn Ala Glu Gly Val Phe Asp
               740                 745                 750
   Val Tyr Arg Val Thr Ser Gly Gly
           755                 760

## Beschreibung

Gegenstand der Erfindung sind Mutanten und Allele des rel-Gens coryneformer Bakterien kodierend für Varianten der GTP-Pyrophosphatkinase (EC Nr. 2.7.6.5) und Verfahren zur Herstellung von Aminosäuren, insbesondere L-Lysin, L-Tryptophan, L-Prolin, L-Valin, L-Isoleucin und L-Homoserin unter Verwendung von Bakterien, die diese Allele enthalten.

### Stand der Technik

Aminosäuren finden in der Humanmedizin, in der pharmazeutischen Industrie, in der Lebensmittelindustrie und ganz besonders in der Tierernährung Anwendung.

Es ist bekannt, dass Aminosäuren durch Fermentation von Stämmen coryneformer Bakterien, insbesondere Corynebacterium glutamicum, hergestellt werden. Wegen der großen Bedeutung wird ständig an der Verbesserung der Herstellverfahren gearbeitet. Verfahrensverbesserungen können fermentationstechnische Maßnahmen wie zum Beispiel Rührung und Versorgung mit Sauerstoff, oder die Zusammensetzung der Nährmedien, wie zum Beispiel die Zuckerkonzentration während der Fermentation, oder die Aufarbeitung zur Produktform durch zum Beispiel Ionenaustauschchromatographie oder die intrinsischen Leistungseigenschaften des Mikroorganismus selbst betreffen.

Zur Verbesserung der Leistungseigenschaften dieser Mikroorganismen werden Methoden der Mutagenese, Selektion und Mutantenauswahl angewendet. Auf diese Weise erhält man Stämme, die resistent gegen Antimetabolite oder auxotroph für regulatorisch bedeutsame Metabolite sind und die Aminosäuren produzieren. Ein bekannter Antimetabolit ist das Lysinanalogon S-(2-Aminoethyl)-L-Cystein (AEC).

Seit einigen Jahren werden ebenfalls Methoden der rekombinanten DNA-Technik zur Stammverbesserung von L-Aminosäure produzierenden Stämmen von Corynebacterium eingesetzt, indem man einzelne Aminosäure-Biosynthesegene amplifiziert und die Auswirkung auf die Aminosäure-Produktion untersucht.

Das Chromosom von Corynebacterium glutamicum wurde vor einiger Zeit vollständig sequenziert (Kalinowski et al., Journal of Biotechnology 104, 5-25 (2003)). Das Chromosom von Corynebacterium efficiens wurde ebenfalls bereits sequenziert (Nishio et al., Genome Res. 13 (7), 1572-1579 (2003)).

Entsprechende Sequenzangaben können den öffentlichen Datenbanken entnommen werden. Geeignete Datenbanken sind beispielsweise die Datenbank der European Molecular Biologies Laboratories (EMBL, Heidelberg, Deutschland bzw. Cambridge, UK), die Datenbank des National Center for Biotechnology Information (NCBI, Bethesda, MD, USA), die des Swiss Institute of Bioinformatics (Swissprot, Geneve, Switzerland), die Protein Information Resource Database (PIR, Washington, DC, USA) und die DNA Data Bank of Japan (DDBJ, 1111 Yata, Mishima, 411-8540, Japan).

Zusammenfassende Darstellungen zur Genetik, zum Stoffwechsel und zur technischen Bedeutung von Corynebacterium findet man in den Aufsätzen von Ikeda, von Pfefferle et al. und von Mueller und Huebner im Buch "Microbial Production of L-Amino Acids" (Advances in Biochemical Engineering 79, (2003), Springer Verlag, Berlin, Deutschland, Herausgeber: T. Scheper), in der Spezialausgabe "A New Era in Corynebacterium glutamicum Biotechnology" des Journal of Biotechnology (Band 104 (1-3), 2003, Herausgeber: A. Pühler und T. Tauch) und im "Handbook of Corynebacterium glutamicum" (Herausgeber: L. Eggeling und M. Bott, CRC Press, Taylor & Francis Group, Boca Raton, FL, USA, 2005).

Die Nukleotidsequenz des für die GTP-Pyrophosphatkinase von Corynebacterium glutamicum kodierenden rel-Gens ist unter anderem in der Datenbank des National Center for Biotechnology Information (NCBI) der National Library of Medicin (Bethesda, MD, USA) unter der Zugangsnummer AF038651 allgemein verfügbar. Sie kann weiterhin der Patentanmeldung EP 1 108 790 als Sequenz Nr. 1824 entnommen werden.

Wehmeier et al. (Microbiology 144, 1853-1862(1998)) berichten unter anderem über genetische, mikrobiologische und biochemische Untersuchungen an einer Mutante von Corynebacterium glutamicum ATCC 13032, die eine Deletion im rel-Gen trägt.

Der besseren Übersichtlichkeit halber ist die Nukleotidsequenz des für die GTP-Pyrophosphatkinase des Wildtyps von Corynebacterium glutamicum kodierenden rel-Gens ("Wildtyp-Gen") gemäß den Angaben der NCBI-Datenbank in SEQ ID NO:1 und die sich daraus ergebende Aminosäuresequenz der kodierten GTP-Pyrophosphatkinase in SEQ ID NO:2 bzw. 4 dargestellt. In SEQ ID NO:3 sind stromaufwärts (upstream) und stromabwärts (downstream) gelegene Nukleotidsequenzen zusätzlich angegeben. Die Aminosäuresequenz gemäß SEQ ID NO:2 bzw. 4 enthält an Position 262 Glycin. Die in der EP 1 108 790 offenbarte Aminosäuresequenz der Wildtyp GTP-Pyrophosphatkinase enthält an Position 262 L-Glutaminsäure. Die Nukleotidsequenz des Wildtyp rel-Gens gemäß EP 1 108 790 ist in SEQ ID NO:21 dargestellt. In SEQ ID NO:22 ist die kodierte Aminosäuresequenz wiedergegeben.

UNIPROT database accession no. Q4JVE5 offenbart die Aminosäuresequenz einer GTP-Pyrophosphatkinase aus *Corynebacterium jeikeium.*

EP 1 486 570 A offenbart ein Verfahren zur Herstellung von L-Aminosäuren, das die Modifikation eines Bakteriums umfasst, so dass das relA Gen überexprimiert wird.

WO 02/077183 A2 offenbart die Aminosäuresequenz einer GTP-Pyrophosphatkinase aus *Corynebacterium diphteriae.*

### Aufgabe der Erfindung

Die Erfinder haben sich zur Aufgabe gestellt neue Maßnahmen zur verbesserten Herstellung von Aminosäuren, insbesondere L-Lysin, L-Tryptophan, L-Prolin, L-Valin, L-Isoleucin und L-Homoserin, bereitzustellen.

### Beschreibung der Erfindung

Gegenstand der Erfindung sind erzeugte bzw. isolierte Mutanten coryneformer Bakterien, die bevorzugt Aminosäuren ausscheiden, und welche ein Gen bzw. Allel enthalten, das für ein Polypeptid mit GTP-Pyrophosphatkinase Aktivität kodiert, dadurch gekennzeichnet, dass das Polypeptid eine Aminosäuresequenz umfasst, ausgewählt aus der Gruppe
a) Aminosäuresequenz gemäß SEQ ID NO:6, und
b) Aminosäuresequenz gemäß SEQ ID NO:6 einschließlich einer oder mehrerer bis max. 5 Insertionen oder Deletionen von Aminosäuren, welche an Position 38 bzw. an der zu Position 38 entsprechenden Position der Aminosäuresequenz L-Leucin enthält, wobei eine zu Position 38 entsprechende Position so zu verstehen ist, dass durch Insertion oder Deletion eines für eine Aminosäure kodierenden Kodons im N-terminalen Bereich bezogen auf die Position 38 von SEQ ID NO:6 des kodierten Polypeptids die Positionsangabe und Längenangabe im Falle einer Insertion formal um eine Einheit erhöht oder im Falle einer Deletion um eine Einheit verringert wird
wobei gegebenenfalls an Position 262 L-Glutaminsäure enthalten ist.

Bei den coryneformen Bakterien wird die Gattung Corynebacterium bevorzugt. Bei der Gattung Corynebacterium werden folgenden Arten bevorzugt:
Corynebacterium efficiens (Typ-Stamm DSM44549),
Corynebacterium glutamicum (Typ-Stamm ATCC13032),
Corynebacterium thermoaminogenes (beispielsweise der Stamm FERM BP-1539), und
Corynebacterium ammoniagenes (Typ-Stamm ATCC6871),
wobei die Art Corynebacterium glutamicum ganz besonders bevorzugt wird.

Einige Vertreter der Art Corynebacterium glutamicum sind im Stand der Technik auch unter anderen Artbezeichnungen bekannt. Hierzu gehören beispielsweise:
Corynebacterium acetoacidophilum ATCC13870,
Corynebacterium lilium DSM20137,
Corynebacterium melassecola ATCC17965,
Brevibacterium flavum ATCC14067,
Brevibacterium lactofermentum ATCC13869,
Brevibacterium divaricatum ATCC14020, und
Microbacterium ammoniaphilum ATCC15354.

Der Begriff "Micrococcus glutamicus" für Corynebacterium glutamicum war ebenfalls gebräuchlich.

Die für die Massnahmen der Erfindung eingesetzten Stämme coryneformer Bakterien besitzen bevorzugt bereits die Fähigkeit die gewünschte Aminosäure in der Zelle anzureichern oder in das sie umgebende Nährmedium auszuscheiden und zu akkumulieren. Hierfür wird im Folgenden auch der Ausdruck "produzieren" verwendet. Insbesondere besitzen die eingesetzten Stämme coryneformer Bakterien die Fähigkeit ≥ (mindestens) 0,25 g/l, ≥ 0,5 g/l, ≥ 1,0 g/l, ≥ 1,5 g/l, ≥ 2,0 g/l, ≥ 4 g/l oder ≥ 10 g/l der gewünschten Aminosäure in ≤ (maximal) 120 Stunden, ≤ 96 Stunden, ≤ 48 Stunden, ≤ 36 Stunden, ≤ 24 Stunden oder ≤ 12 Stunden in der Zelle oder im Nährmedium anzureichern bzw. zu akkumulieren. Hierbei kann es sich um Stämme handeln, die durch Mutagenese und Selektion, durch rekombinante DNA-Techniken oder durch eine Kombination beider Methoden hergestellt wurden.

Bekannte Vertreter L-Lysin produzierender bzw. ausscheidender Stämme coryneformer Bakterien sind beispielsweise:
Corynebacterium glutamicum DM58-1/pDM6 (= DSM4697) beschrieben in EP 0 358 940,
Corynebacterium glutamicum MH20-22B (= DSM16835) beschrieben in Menkel et al. (Applied and Environmental Microbiology 55(3), 684-688 (1989)),
Corynebacterium glutamicum AHP-3 (= Ferm BP-7382) beschrieben in EP 1 108 790,
Corynebacterium glutamicum NRRL B-11474 beschrieben in US 4,275,157, und
Corynebacterium thermoaminogenes AJ12521 (= FERM BP-3304) beschrieben in US 5,250,423.

Bekannte Vertreter L- Tryptophan produzierender bzw. ausscheidender Stämme coryneformer Bakterien sind beispielsweise:
Corynebacterium glutamicum K76 (=Ferm BP-1847) beschrieben in US 5,563,052,
Corynebacterium glutamicum BPS13 (=Ferm BP-1777) beschrieben in US 5,605,818, und
Corynebacterium glutamicum Ferm BP-3055 beschrieben in US 5,235,940.

Bekannte Vertreter L-Prolin produzierender bzw. ausscheidender Stämme coryneformer Bakterien sind beispielsweise:
Brevibacterium lactofermentum NRRL B-11421 beschrieben in US 4,224,409,
Brevibacterium flavum NRRL B-11422 beschrieben in US 4,224,409,
Brevibacterium flavum FERM BP-2214 beschrieben in US 5,294,547,
Corynebacterium glutamicum NRRL B-11423 beschrieben in US 4,224,409,
Corynebacterium glutamicum ATCC 21157 beschrieben in US 4,444,885,
Corynebacterium glutamicum ATCC 21158 beschrieben in US 4,444,885,
Corynebacterium glutamicum ATCC 21159 beschrieben in US 4,444,885,
Corynebacterium glutamicum ATCC 21355 beschrieben in US 4,444,885, und
Microbacterium ammoniaphilum NRRL B-11424 beschrieben in US 4,224,409.

Bekannte Vertreter L-Valin produzierender bzw. ausscheidender Stämme coryneformer Bakterien sind beispielsweise:
Brevibacterium lactofermentum FERM BP-1763 beschrieben in US 5,188,948,
Brevibacterium lactofermentum FERM BP-3007 beschrieben in US 5,521,074,
Corynebacterium glutamicum FERM BP-3006 beschrieben in US 5,521,074, und
Corynebacterium glutamicum FERM BP-1764 beschrieben in US 5,188,948.

Bekannte Vertreter L-Isoleucin produzierender bzw. ausscheidender Stämme coryneformer Bakterien sind beispielsweise:
Brevibacterium flavum FERM BP-760 beschrieben in US 4,656,135,
Brevibacterium flavum FERM BP-2215 beschrieben in US 5,294,547, und
Corynebacterium glutamicum FERM BP-758 beschrieben in US 4,656,135.

Bekannte Vertreter L-Homoserin produzierender bzw. ausscheidender Stämme coryneformer Bakterien sind beispielsweise:
Micrococcus glutamicus ATCC 14296 beschrieben in US 3,189,526 und
Micrococcus glutamicus ATCC 14297 beschrieben in US 3,189,526.

Angaben zur taxonomischen Einordnung von Stämmen dieser Gruppe von Bakterien findet man unter anderem bei Seiler (Journal of General Microbiology 129, 1433-1477 (1983)), Kinoshita (1985, Glutamic Acid Bacteria, p 115-142. In: Demain and Solomon (ed), Biology of Industrial Microorganisms. The Benjamin/Cummins Publishing Co., London, UK), Kämpfer und Kroppenstedt (Canadian Journal of Microbiology 42, 989-1005 (1996)), Liebl et al (International Journal of Systematic Bacteriology 41, 255-260 (1991)) und in der US-A-5,250,434.

Stämme mit der Bezeichnung "ATCC" können von der American Type Culture Collection (Manassas, VA, USA) bezogen werden. Stämme mit der Bezeichnung "DSM" können von der Deutschen Sammlung von Mikroorganismen und Zellkulturen (DSMZ, Braunschweig, Deutschland) bezogen werden. Stämme mit der Bezeichnung "NRRL" können von der Agricultural Research Service Patent Culture Collection (ARS, Peoria, Illinois, US) bezogen werden. Stämme mit der Bezeichnung "FERM" können vom National Institute of Advanced Industrial Science and Technology (AIST Tsukuba Central 6, 1-1-1 Higashi, Tsukuba Ibaraki, Japan) bezogen werden.

Ein Gen ist chemisch ein Polynucleotid. Ein anderer Begriff hierfür ist Nukleinsäure. Für das vom rel-Gen kodierte Polypeptid mit GTP-Pyrophosphatkinase Aktivität werden im Stand der Technik auch andere Bezeichnungen wie "GTP diphosphokinase", "guanosine 3',5'-polyphosphate synthase" oder "stringent factor" verwendet (Siehe beispielsweise KEGG-Datenbank (Kyoto Encyclopedia of Genes and Genomes) des Kanehisa Laboratory, Bioinformatics Center, Institute for Chemical Research, Kyoto University, Japan). Entsprechend der Nomenklatur der IUPAC (International Union of Pure and Applied Chemistry) hat es die EC-Nummer 2.7.6.5.

Es katalysiert die Reaktion:
ATP + GTP^{→} AMP + Guanosin-3'-diphosphat-5'-triphosphat, wobei anstelle von GTP auch GDP als Substrat verwendet wird.

Unter proteinogenen Aminosäuren versteht man im Allgemeinen die Aminosäuren, die in natürlichen Proteinen, das heißt in Proteinen von Mikroorganismen, Pflanzen, Tieren und Menschen vorkommen. Unter proteinogenen Aminosäuren wird im Zusammenhang mit der vorliegenden Erfindung jedoch die Gruppe der L-Aminosäuren, bestehend aus L-Asparaginsäure, L-Asparagin, L-Threonin, L-Serin, L-Glutaminsäure, L-Glutamin, Glycin, L-Alanin, L-Cystein, L-Valin, L-Methionin, L-Isoleucin, L-Leucin, L-Tyrosin, L-Phenylalanin, L-Histidin, L-Lysin, L-Tryptophan, L-Prolin und L-Arginin verstanden. Zu den L-Aminosäuren gehört ebenfalls das L-Homoserin.

Die erfindungsgemäßen Mutanten scheiden bevorzugt die genannten proteinogenen Aminosäuren, insbesondere L-Lysin, L-Tryptophan, L-Valin, L-Isoleucin oder L-Homoserin aus. Der Begriff Aminosäuren umfasst auch deren Salze wie beispielsweise das Lysin-Monohydrochlorid oder Lysin-Sulfat im Falle der Aminosäure L-Lysin.

Die Beschreibung offenbart weiterhin Mutanten coryneformer Bakterien, die ein rel-Allel enthalten, das für ein Polypeptid mit GTP-Pyrophosphatkinase-Enzymaktivität kodiert, das die Aminosäuresequenz von SEQ ID NO: 2 umfasst, wobei an Position 38 eine der genannten proteinogenen Aminosäuren ausgenommen L-Prolin enthalten ist. Der Austausch von L-Prolin gegen L-Leucin wird bevorzugt.

Die Beschreibung offenbart weiterhin Mutanten coryneformer Bakterien, die ein rel-Allel enthalten, das für ein Polypeptid mit GTP-Pyrophosphatkinase Enzymaktivität kodiert, das an der der Position 38 der Aminosäuresequenz von SEQ ID NO:2 entsprechenden Position eine der proteinogenen Aminosäuren ausgenommen L-Prolin, bevorzugt jedoch L-Leucin, enthält, wobei das Gen eine Nukleotidsequenz umfasst, die mit der Nukleotidsequenz eines Polynukleotids identisch ist, das durch eine Polymerasekettenreaktion (PCR) unter Verwendung eines Primerpaars erhältlich ist, deren Nukleotidsequenzen jeweils mindestens 15 aufeinanderfolgende Nukleotide umfassen, die aus aus der Nukleotidsequenz zwischen Position 1 und 750 von SEQ ID NO:3 oder SEQ ID NO:7 und aus der komplementären Nukleotidsequenz zwischen Position 3800 und 3031 von SEQ ID NO:3 oder SEQ ID NO:7 ausgewählt werden. Ein Beispiel für ein geeignetes Primerpaar ist in SEQ ID NO:19 und SEQ ID NO:20 dargestellt. Als Ausgangsmaterial ("template"-DNA) wird chromosomale DNA coryneformer Bakterien bevorzugt, die insbesondere mit einem Mutagen behandelt worden sind. Besonders bevorzugt wird die chromosomale DNA der Gattung Corynebacterium und ganz besonders bevorzugt die der Art Corynebacterium glutamicum.

Die Beschreibung offenbart weiterhin Mutanten coryneformer Bakterien, die ein rel-Allel enthalten, das für ein Polypeptid mit GTP-Pyrophosphatkinase Enzymaktivität kodiert, das eine Aminosäuresequenz mit einer Länge entsprechend 760 L-Aminosäuren umfasst, wobei an Position 38 eine der proteinogenen Aminosäuren ausgenommen L-Prolin, bevorzugt L-Leucin, enthalten ist.

Die Beschreibung offenbart weiterhin Mutanten coryneformer Bakterien, die ein rel-Allel enthalten, das für ein Polypeptid mit GTP-Pyrophosphatkinase Enzymaktivität kodiert, das an Position 19 bis 57 der Aminosäuresequenz die Aminosäuresequenz entsprechend Position 19 bis 57 von SEQ ID NO:6 bzw. 8 enthält. Bevorzugt enthält die Aminosäuresequenz des kodierten Polypeptids eine Aminosäuresequenz entsprechend Position 9 bis 107 von SEQ ID NO:6 bzw. 8 oder Position 9 bis 207 von SEQ ID NO:6 bzw. 8 oder Position 9 bis 407 von SEQ ID NO:6 bzw. 8 oder Position 9 bis 607 von SEQ ID NO:6 bzw. 8 oder Position 9 bis 707 von SEQ ID NO:6 bzw. 8 oder Position 2 bis 707 von SEQ ID NO:6 bzw. 8 oder Position 9 bis 757 von SEQ ID NO:6 bzw. 8 oder Position 2 bis 757 von SEQ ID NO:6 bzw. 8 oder Position 2 bis 758 von SEQ ID NO:6 bzw. 8 oder Position 2 bis 759 von SEQ ID NO:6 bzw. 8, wobei gegebenenfalls an Position 262 L-Glutaminsäure enthalten ist. Ganz besonders bevorzugt umfasst die Länge des kodierten Polypeptids 760 Aminosäuren.

Die Beschreibung offenbart weiterhin Mutanten coryneformer Bakterien, die ein rel-Allel enthalten, das für ein Polypeptid mit GTP-Pyrophosphatkinase Enzymaktivität kodiert, welches an Position 38 bzw. an der entsprechenden Position der Aminosäuresequenz eine der proteinogenen Aminosäuren ausgenommen L-Prolin enthält, wobei dem Austausch gegen L-Leucin der Vorzug gegeben wird und dessen Aminosäuresequenz außerdem zu mindestens 90%, bevorzugt mindestens 92% oder mindestens 94% oder mindestens 96%, und ganz besonders bevorzugt mindestens 97% oder mindestens 98% oder mindestens 99% identisch ist mit der Aminosäuresequenz von SEQ ID NO:6 bzw. SEQ ID NO:8. Gegebenenfalls ist an Position 262 von SEQ ID NO:6 bzw. SEQ ID NO:8 L-Glutaminsäure enthalten.

Die Beschreibung offenbart weiterhin Mutanten coryneformer Bakterien, die ein rel-Allel enthalten, das für ein Polypeptid mit GTP-Pyrophosphatkinase-Enzymaktivität kodiert, welches an Position 38 bzw. an der entsprechenden Position der Aminosäuresequenz eine der proteinogenen Aminosäuren ausgenommen L-Prolin enthält,
wobei dem Austausch gegen L-Leucin der Vorzug gegeben wird und dessen Nukleotidsequenz außerdem zu mindestens 90%, bevorzugt mindestens 92% oder mindestens 94% oder mindestens 96%, und ganz besonders bevorzugt mindestens 97% oder mindestens 98% oder mindestens 99% identisch ist mit der Nukleotidsequenz von SEQ ID NO:5. Gegebenenfalls ist an Position 785 Adenin enthalten.

Es ist bekannt, dass konservative Aminosäureaustausche die Enzymaktivität nur unwesentlich verändern. Dementsprechend kann das in den erfindungsgemäßen Mutanten enthaltene rel-Allel, das für ein Polypeptid mit GTP-Pyrophosphatkinase Enzymaktivität kodiert, zusätzlich zu der in SEQ ID NO:6 bzw. SEQ ID NO:8 gezeigten Aminosäuresequenz einen (1) oder mehrere konservative Aminosäureaustausch(e) enthalten. Bevorzugt enthält das Polypeptid höchstens zwei (2), höchstens drei (3), höchstens vier (4) oder höchstens fünf (5) konservative Aminosäureaustausche. Gegebenenfalls ist an Position 262 von SEQ ID NO:6 bzw. SEQ ID NO:8 L-Glutaminsäure enthalten.

Bei den aromatischen Aminosäuren spricht man von konservativen Austauschen, wenn Phenylalanin, Tryptophan und Tyrosin gegeneinander ausgetauscht werden. Bei den hydrophoben Aminosäuren spricht man von konservativen Austauschen, wenn Leucin, Isoleucin und Valin gegeneinander ausgetauscht werden. Bei den polaren Aminosäuren spricht man von konservativen Austauschen, wenn Glutamin und Asparagin gegeneinander ausgetauscht werden. Bei den basischen Aminosäuren spricht man von konservativen Austauschen, wenn Arginin, Lysin und Histidin gegeneinander ausgetauscht werden. Bei den sauren Aminosäuren spricht man von konservativen Austauschen, wenn Asparaginsäure und Glutaminsäure gegeneinander ausgetauscht werden. Bei den Hydroxyl-Gruppen enthaltenden Aminosäuren spricht man von konservativen Austauschen, wenn Serin und Threonin gegeneinander ausgetauscht werden.

Bei der Arbeit an der vorliegenden Erfindung wurde durch Vergleich der Aminosäuresequenz mit dem Clustal-Programm (Thompson et al., Nucleic Acids Research 22, 4637-4680 (1994)) festgestellt, dass die Aminosäuresequenzen der GTP-Pyrophosphatkinase verschiedener Bakterien wie bespielsweise Escherichia coli, Bacillus subtilis, Mycobacterium tuberculosis, Mycobacterium bovis, Streptomyces coelicolor, Streptomyces avermitilis, Corynebacterium efficiens und Corynebacterium glutamicum ein Sequenzmotiv bestehend aus der Abfolge Ser-Gly-Asp/Glu-Pro-Tyr-Ile-Thr/Ile-His-Pro-Leu-Ala-Val, ein Sequenzmotiv bestehend aus der Abfolge Ala-Ala/Gly-Leu-Leu-His-Asp-Thr-Val-Glu-Asp-Thr und auch ein Sequenzmotiv bestehend aus der Abfolge Thr-Pro-Val-Asp-Phe-Ala-Tyr-Ala-Val-His-Thr-Glu-Val-Gly-His-Arg enthalten. Die Bezeichnungen "Asp/Glu", "Thr/Ile" und "Ala/Gly" bedeuten, dass an der entsprechenden Position "Asp oder Glu" bzw. "Thr oder Ile" oder "Ala oder Gly" enthalten sind.

Dementsprechend sind solche Mutanten coryneformer Bakterien bevorzugt, die ein rel-Allel enthalten, das für ein Polypeptid mit GTP-Pyrophosphatkinase Enzymaktivität kodiert, welches mindestens eine Aminosäuresequenz ausgewählt aus der Gruppe Ser-Gly-Asp/Glu-Pro-Tyr-Ile-Thr/Ile-His-Pro-Leu-Ala-Val, Ala-Ala/Gly-Leu-Leu-His-Asp-Thr-Val-Glu-Asp-Thr und Thr-Pro-Val-Asp-Phe-Ala-Tyr-Ala-Val-His-Thr-Glu-Val-Gly-His-Arg umfasst und an Position 38 bzw. der entsprechenden oder vergleichbaren Position der Aminosäuresequenz eine der proteinogenen Aminosäuren ausgenommen L-Prolin, bevorzugt L-Leucin, enthält.

Das Aminosäuresequenzmotiv Ser-Gly-Asp/Glu-Pro-Tyr-Ile-Thr/Ile-His-Pro-Leu-Ala-Val ist beispielsweise in der SEQ ID NO:2 bzw. 4 oder 6 bzw. 8 von Position 73 bis 84 enthalten. Das Aminosäuresequenzmotiv Ala-Ala/Gly-Leu-Leu-His-Asp-Thr-Val-Glu-Asp-Thr ist beispielsweise in der SEQ ID NO:2 bzw. 4 oder 6 bzw. 8 von Position 100 bis 110 enthalten. Das Aminosäuresequenzmotiv Thr-Pro-Val-Asp-Phe-Ala-Tyr-Ala-Val-His-Thr-Glu-Val-Gly-His-Arg ist beispielsweise in der SEQ ID NO: 2 bzw. 4 oder 6 bzw. 8 von Position 437 bis 452 enthalten.

Gegenstand der Erfindung sind schließlich Mutanten coryneformer Bakterien, die ein rel-Allel enthalten, das für ein Polypeptid mit GTP-Pyrophosphatkinase Enzymaktivität kodiert, welches die Aminosäuresequenz von SEQ ID NO:6 umfasst, wobei gegebenenfalls an Position 262 L-Glutaminsäure enthalten ist.

Es ist bekannt, dass durch wirtseigene Enzyme, sogenannte Aminopeptidasen, das endständige Methionin bei der Proteinsynthese entfernt wird.

Unter dem Begriff "einer zu Position 38 der Aminosäuresequenz entsprechenden Position" oder "einer zu Position 38 der Aminosäuresequenz vergleichbaren Position" versteht man die Tatsache, dass durch Insertion oder Deletion eines für eine Aminosäure kodierenden Kodons im N-terminalen Bereich (bezogen auf die Position 38 von SEQ ID NO:6 bzw. 8) des kodierten Polypeptids die Positionsangabe und Längenangabe im Falle einer Insertion formal um eine Einheit erhöht oder im Falle einer Deletion um eine Einheit verringert wird. Beispielsweise rückt durch Deletion des für die Aminosäure L-Glutaminsäure kodierenden GAG-Kodons an Position 4 von SEQ ID NO:6 bzw. 8 das L-Leucin von Position 38 an Position 37. Die Längenangabe wäre dann: 759 Aminosäuren. In gleicher Weise wird durch Insertion oder Deletion eines für eine Aminosäure kodierenden Kodons im C-terminalen Bereich (bezogen auf die Position 38) des kodierten Polypeptids die Längenangabe im Falle einer Insertion formal um eine Einheit erhöht oder im Falle einer Deletion um eine Einheit verringert. Derartige vergleichbare Positionen lassen sich durch Vergleich der Aminosäuresequenzen in Form eines "alignments" beispielsweise mit Hilfe des Clustal-Programmes oder des MAFFT-Prgrammes leicht identifizieren.

Durch derartige Insertionen und Deletionen wird die enzymatische Aktivität im wesentlichen nicht berührt. "Im wesentlichen nicht berührt" bedeutet, dass sich die enzymatische Aktivität der genannten Varianten um maximal 10%, maximal 7,5%, maximal 5%, maximal 2,5% oder maximal 1% von der Aktivität des Polypeptids mit der Aminosäuresequenz von SEQ ID NO:6 bzw. 8 unterscheidet, wobei gegebenenfalls an Position 262 L-Glutaminsäure enthalten ist.

Gegenstand der Erfindung sind dementsprechend auch rel-Allele, die für Polypeptid-Varianten von SEQ ID NO:6 kodieren, die eine oder mehrere bis maximal 5 Insertion(en) oder Deletion(en) enthalten, wobei gegebenenfalls an Position 262 L-Glutaminsäure enthalten ist. Bevorzugt enthält das Polypeptid maximal 4, maximal 3 oder maximal 2 Insertionen oder Deletionen von Aminosäuren.

Die zuvor genannten Sequenzmotive Ser-Gly-Asp/Glu-Pro-Tyr-Ile-Thr/Ile-His-Pro-Leu-Ala-Val, Ala-Ala/Gly-Leu-Leu-His-Asp-Thr-Val-Glu-Asp-Thr und Thr-Pro-Val-Asp-Phe-Ala-Tyr-Ala-Val-His-Thr-Glu-Val-Gly-His-Arg werden durch derartige Insertionen/Deletionen vorzugsweise nicht zerrissen.

Zur Herstellung der erfindungsgemäßen Mutanten können klassische in-vivo Mutageneseverfahren mit Zellpopulationen coryneformer Bakterien unter Verwendung mutagener Stoffe wie beispielsweise N-Methyl-N'-Nitro-N-Nitrosoguanidin (MNNG), Ethylmethansulfonat (EMS), 5-Bromuracil, oder ultraviolettes Licht verwendet werden. Mutagenesemethoden sind beispielsweise im Manual of Methods for General Bacteriology (Gerhard et al. (Eds.), American Society for Microbiology, Washington, DC, USA, 1981) oder bei Tosaka et al. (Agricultural and Biological Chemistry 42(4), 745-752 (1978)) oder bei Konicek et al (Folia Microbiologica 33, 337-343 (1988)) beschrieben. Typische Mutagenesen unter Verwendung von MNNG umfassen Konzentrationen von 50 bis 500 mg/l oder auch höhere Konzentrationen bis zu maximal 1 g/l, eine Inkubationszeit von 1 bis 30 Minuten bei einem pH von 5,5 bis 7,5. Unter diesen Bedingungen wird die Zahl der lebensfähigen Zellen um einen Anteil von ca. 50% bis 90% oder ca. 50% bis 99% oder ca. 50% bis 99,9% oder mehr reduziert.

Aus der mutagenisierten Zellpopulation werden Mutanten bzw. Zellen entnommen und vermehrt. Vorzugsweise wird in einem weiteren Schritt deren Fähigkeit untersucht, in einer Satzkultur (batch) unter Verwendung eines geeigneten Nährmediums Aminosäuren, bevorzugt L-Lysin, L-Tryptophan, L-Prolin, L-Valin, L-Isoleucin oder L-Homoserin auszuscheiden. Geeignete Nährmedien und Testbedingungen sind unter anderem in der US 6,221,636, in der US 5,840,551, in der US 5,770,409, in der US 5,605,818, in der US 5,275,940 und in der US 4,224,409 beschrieben. Bei Verwendungen von geeigneten Roboteranlagen, wie beispielsweise bei Zimmermann et al. (VDI Berichte Nr. 1841, VDI-Verlag, Düsseldorf, Deutschland 2004, 439-443) oder Zimmermann (Chemie Ingenenieur Technik 77 (4), 426-428 (2005))beschrieben, können zahlreiche Mutanten in kurzer Zeit untersucht werden. Im Allgemeinen werden maximal 3.000, maximal 10.000, maximal 30.000 oder auch maximal 60.000 Mutanten gegebenenfalls auch mehr untersucht. Auf diese Weise werden Mutanten identifiziert, die im Vergleich zum Elternstamm bzw. nicht mutagenisierten Ausgangsstamm vermehrt Aminosäuren in das Nährmedium oder in das Zellinnere ausscheiden. Dazu gehören beispielsweise solche Mutanten, deren Aminosäuresekretion um mindestens 0,5% erhöht ist.

Anschließend wird aus den Mutanten DNA bereitgestellt, bzw. isoliert und mit Hilfe der Polymerase-Kettenreaktion unter Verwendung von Primerpaaren, die die Amplifizierung des rel-Gens bzw. des erfindungsgemäßen rel-Allels oder der erfindungsgemäßen Mutation an Position 38 erlauben, das entsprechende Polynukleotid synthetisiert. Vorzugsweise wird die DNA aus solchen Mutanten isoliert, die in erhöhter Weise Aminosäuren ausscheiden.

Hierzu können beliebige Primerpaare aus der stromaufwärts und stromabwärts der erfindungsgemäßen Mutation gelegenen Nukleotidsequenz und der dazu komplementären Nukleotidsequenz ausgewählt werden. Ein Primer eines Primerpaares umfasst hierbei bevorzugt mindestens 15, mindestens 18, mindestens 20, mindestens 21 oder mindestens 24 aufeinanderfolgende Nukleotide ausgewählt aus der Nukleotidsequenz zwischen Position 1 und 861 von SEQ ID NO:3 oder SEQ ID NO:7. Der dazugehörige zweite Primer eines Primerpaares umfasst mindestens 15, mindestens 18, mindestens 20, mindestens 21 oder mindestens 24 aufeinanderfolgende Nukleotide ausgewählt aus der komplementären Nukleotidsequenz von Position 3800 und 865 von SEQ ID NO:3 oder SEQ ID NO:7. Ist die Amplifikation der Kodierregion gewünscht, so wird das Primerpaar vorzugsweise aus der Nukleotidsequenz zwischen Position 1 und 750 von SEQ ID NO:3 oder SEQ ID NO:7 und aus der komplementären Nukleotidsequenz zwischen Position 3800 und 3031 von SEQ ID NO:3 oder SEQ ID NO:7 ausgewählt.

Ist die Amplifikation eines Teils der Kodierregion, wie beispielsweise in SEQ ID NO:11 und 13 dargestellt, erwünscht, so wird das Primerpaar vorzugsweise aus der Nukleotidsequenz zwischen Position 751 und 804 oder zwischen Position 1 und 804 von SEQ ID NO:3 oder SEQ ID NO:7 und aus der komplementären Nukleotidsequenz zwischen Position 3030 und 922 oder 3800 und 922 von SEQ ID NO:3 oder SEQ ID NO:7 ausgewählt. Ein geeignetes Primerpaar ist beispielsweise das unter SEQ ID NO:19 und SEQ ID NO:20 wiedergegebene Primerpaar rel_XL_A1 und rel_XL_E1. Der Primer kann überdies mit Erkennungsstellen für Restriktionsenzyme, mit einer Biotin-Gruppe oder weiteren Accessoirs, wie sie im Stand der Technik beschrieben sind, ausgerüstet sein. Die Gesamtlänge des Primers beträgt im Allgemeinen maximal 30, 40, 50 oder 60 Nukleotide.

Für die Herstellung von Polynukleotiden durch Amplifikation ausgewählter Sequenzen, wie dem erfindungsgemäßen rel-Allel, aus vorgelegter, beispielsweise chromosomaler DNA ("template DNA") durch Amplifikation mittels PCR, werden im Allgemeinen thermostabile DNA-Polymerasen eingesetzt. Beispiele derartiger DNA-Polymerasen sind die Taq-Polymerase aus Thermus aquaticus, die unter anderem von der Firma Qiagen (Hilden, Deutschland) vertrieben wird, die Vent-Polymerase aus Thermococcus litoralis, die unter anderem von der Firma New England Biolabs (Frankfurt, Deutschland) vertrieben wird oder die Pfu-Polymerase aus Pyrococcus furiosus, die unter anderem von der Firma Stratagene (La Jolla, USA) vertrieben wird. Bevorzugt werden Polymerasen mit "proof-reading"-Aktivität. "proof-reading"-Aktivität bedeutet, dass diese Polymerasen in der Lage sind, fehlerhaft eingebaute Nukleotide zu erkennen und den Fehler durch erneute Polymerisation zu beheben (Lottspeich und Zorbas, Bioanalytik, Spektrum Akademischer Verlag, Heidelberg, Deutschland (1998)). Beispiele für Polymerasen mit "proof-reading"-Aktivität sind die Vent-Polymerase und die Pfu-Polymerase.

Die Bedingungen im Reaktionsansatz werden nach Angaben des Herstellers eingestellt. Die Polymerasen werden vom Hersteller im Allgemeinen zusammen mit dem gebräuchlichen Puffer geliefert, welcher üblicherweise Konzentrationen von 10 - 100 mM Tris/HCl und 6 - 55 mM KCl bei pH 7,5 - 9,3 aufweist. Magnesiumchlorid wird in einer Konzentration von 0,5 - 10 mM zugesetzt, falls es nicht in dem vom Hersteller gelieferten Puffer enthalten ist. Dem Reaktionsansatz werden weiterhin Desoxynucleosidtriphosphate in einer Konzentration von 0,1 - 16,6 mM zugesetzt. Die Primer werden im Reaktionsansatz mit einer Endkonzentration von 0,1 - 3 µM vorgelegt und die Template-DNA im optimalen Fall mit 10² bis 10⁵ Kopien. Es können auch 10⁶ bis 10⁷ Kopien eingesetzt werden. Die entsprechende Polymerase wird dem Reaktionsansatz in einer Menge von 2-5 Units zugegeben. Ein typischer Reaktionsansatz hat ein Volumen von 20 - 100 µl.

Als weitere Zusätze können der Reaktion Rinderserumalbumin, Tween-20, Gelatin, Glycerin, Formamid oder DMSO beigesetzt werden (Dieffenbach und Dveksler, PCR Primer - A Laboratory Manual, Cold Spring Harbor Laboratory Press, USA 1995).

Ein typischer PCR-Verlauf besteht aus drei unterschiedlichen, sich aufeinanderfolgend wiederholenden Temperaturstufen. Vorab wird die Reaktion mit einer Temperaturerhöhung auf 92°C - 98°C für 4 bis 10 Minuten gestartet, um die vorgelegte DNA zu denaturieren. Dann folgen sich wiederholend zuerst ein Schritt zur Denaturierung der vorgelegten DNA von 10 - 60 Sekunden bei ungefähr 92-98°C, dann ein Schritt zum Binden der Primer an die vorgelegte DNA von 10 - 60 Sekunden bei einer bestimmten, von den Primern abhängigen Temperatur ("Annealing-Temperatur"), die erfahrungsgemäß bei 50°C bis 60°C liegt und sich für jedes Primerpaar einzeln berechnen läßt. Genaue Informationen dazu findet der Fachmann bei Rychlik et al. (Nucleic Acids Research 18 (21): 6409-6412). Anschließend folgt ein Synthese-Schritt zur Verlängerung der vorgelegten Primer ("Extension") bei dem jeweils für die Polymerase angegebenen Aktivitätsoptimum, üblicherweise je nach Polymerase im Bereich von 73°C bis 67°C bevorzugt 72°C bis 68°C. Die Dauer dieses Extensionschrittes hängt von der Leistungsfähigkeit der Polymerase und Länge des zu amplifizierenden PCR-Produktes ab. In einer typischen PCR dauert dieser Schritt 0,5 - 8 Minuten, bevorzugt 2 - 4 Minuten. Diese drei Schritte werden 30 bis 35 mal, gegebenenfalls bis zu 50 mal wiederholt. Ein abschließender "Extension"-Schritt von 4 - 10 Minuten beendet die Reaktion. Die auf diese Weise hergestellten Polynukleotide werden auch als Amplifikate bezeichnet; der Begriff Nukleinsäurefragment ist ebenfalls gebräuchlich.

Weitere Anleitungen und Informationen zur PCR findet der Fachmann beispielsweise im Handbuch "PCR-Strategies" (Innis, Felfand und Sninsky, Academic Press , Inc., 1995), im Handbuch von Diefenbach und Dveksler "PCR Primer - a laboratory manual" (Cold Spring Harbor Laboratory Press,1995), im Handbuch von Gait "Oligonukleotide synthesis: A Practical Approach" (IRL Press, Oxford, UK, 1984) und bei Newton und Graham "PCR" (Spektrum Akademischer Verlag, Heidelberg, Deutschland, 1994).

Die Nukleotidsequenz wird anschließend beispielsweise nach dem Kettenabbruchverfahren von Sanger et al. (Proceedings of the National Academies of Sciences, U.S.A., 74, 5463-5467 (1977)) mit den von Zimmermann et al. (Nucleic Acids Research 18, 1067pp (1990)) angegebenen Modifikationen bestimmt und das von dieser Nukleotidsequenz kodierte Polypeptid insbesondere bezüglich der Aminosäuresequenz analysiert. Dazu wird die Nukleotidsequenz in ein Programm zur Übersetzung von DNA-Sequenz in eine Aminosäuresequenz eingegeben. Geeignete Programme sind beispielsweise das Programm "Patentin", das bei Patentämtern, beispielsweise dem US-Patentamt (USPTO) erhältlich ist, oder das "Translate Tool", das auf dem ExPASy Proteomics Server im World Wide Web (Gasteiger et al., Nucleic Acids Research 31, 3784-3788 (2003)) verfügbar ist.

Auf diese Weise werden Mutanten identifiziert, deren rel-Allele für Polypeptide mit GTP-Pyrophosphatkinase Enzymaktivität kodieren, welche an Position 38 der Aminosäuresequenz, bzw. der entsprechenden oder vergleichbaren Position L-Leucin enthalten. Gegebenenfalls wird das gesamte Chromosom der Mutante bestimmt. Hierbei kann die von Margulies et al. (Nature, 437(7057): 376-380 (2005)) und Velicer et al. (Proceedings of the National Academy of Sciences, U.S.A., 103(21), 8107-8112 (2006)) beschriebene Methode, die unter dem Stichwort "pyro-sequencing" in der Fachwelt bekannt ist und eine rasche Sequenzierung kompletter Genome ermöglicht, eingesetzt werden.

Die Beschreibung offenbart dementsprechend eine Mutante eines coryneformen Bakteriums, dadurch gekennzeichnet, dass diese durch folgende Schritte erhältlich ist:
a) Behandlung eines coryneformen Bakteriums, das die Fähigkeit besitzt Aminosäuren auszuscheiden, mit einem mutagenen Agenz,
b) Isolierung und Vermehrung der in a) erzeugten Mutante,
c) vorzugsweise Bestimmung der Fähigkeit der Mutante in einem Medium mindestens 0,5% mehr Aminosäure auszuscheiden oder im Zellinneren anzureichern als das in a) eingesetzte coryneforme Bakterium,
d) Bereitstellung von Nukleinsäure aus der in b) erhaltenen Mutante,
e) Herstellung eines Nukleinsäuremoleküls (bzw. Amplifikates oder Nukleinsäurefragments) unter Verwendung der Polymerasekettenreaktion, der Nukleinsäure aus d), und eines Primerpaares bestehend aus einem ersten Primer umfassend mindestens 15 aufeinanderfolgenden Nukleotide ausgewählt aus der Nukleotidsequenz zwischen Position 1 und 861, bevorzugt 1 bis 750 von SEQ ID NO:3 oder SEQ ID NO:7 und einem zweitem Primer umfassend mindestens 15 aufeinanderfolgende Nukleotide ausgewählt aus der komplementären Nukleotidsequenz zwischen Position 3800 und 865, bevorzugt 3800 und 3031 von SEQ ID NO:3 oder 7,
f) Bestimmung der Nukleotidsequenz des in e) erhaltenen Nukleinsäuremoleküls, und Bestimmung der kodierten Aminosäuresequenz,
g) gegebenfalls Vergleich der in f) bestimmten Aminosäuresesequenz mit SEQ ID NO:6, wobei gegebenenfalls an Position 262 L-Glutaminsäure enthalten ist, und
h) Identifizierung einer Mutante, die ein Polynukleotid enthält, das für ein Polypeptid kodiert, welches an Position 38 oder an vergleichbarer Position eine der proteinogenen Aminosäuren ausgenommen L-Prolin, bevorzugt L-Leucin, enthält.

Die auf diese Weise erzeugten Mutanten enthalten typischerweise eine (1) Kopie des beschriebenen rel-Allels.

In der SEQ ID NO:5 ist beispielhaft die Kodierregion des rel-Allels einer erfindungsgemäßer Mutante wiedergegeben. Die Kodierregion des Wildtypgens ist als SEQ ID NO:1 wiedergegeben. SEQ ID NO:1 enthält an Position 112 die Nukleobase Cytosin, an Position 113 die Nukleobase Cytosin und an Position 114 die Nukleobase Guanin. SEQ ID NO:1 enthält somit von Position 112 bis 114 das für die Aminosäure L-Prolin kodierende CCG Kodon. SEQ ID NO:5 enthält an Position 113 die Nukleobase Thymin. Durch diese Cytosin zu Thymin Transition entsteht an Position 112 bis 114 das für die Aminosäure L-Leucin kodierende CTG Kodon.

Darüber hinaus können die in SEQ ID NO: 5 bzw. 7 dargestellten Nukleotidsequenzen weitere Basenaustausche enthalten, die sich durch die Mutagenesebehandlung ergeben haben, sich jedoch nicht in einer veränderten Aminosäuresequenz äußern. Derartige Mutationen werden in der Fachwelt auch als stille oder neutrale Mutationen bezeichnet. Diese stillen Mutationen können ebenfalls in dem für die Mutagenesebehandlung eingesetzten coryneformen Bakterium bereits enthalten sein.

Die für die Mutagenese verwendeten coryneformen Bakterien besitzen bevorzugt bereits die Fähigkeit, die gewünschte Aminosäure in das/die sie umgebende Nährmedium bzw. Fermentationsbrühe auszuscheiden oder im Zellinneren anzureichern.

L-Lysin produzierende coryneforme Bakterien besitzen typischerweise eine "feed back" resistente bzw. desensibilisierte Aspartatkinase. Unter "feed back" resistenten Aspartatkinasen versteht man Aspartatkinasen (LysC), die im Vergleich zur Wildform eine geringere Empfindlichkeit gegenüber der Hemmung durch Mischungen von Lysin und Threonin oder Mischungen von AEC (Aminoethylcystein) und Threonin oder Lysin allein oder AEC allein aufweisen. Die für diese desensibilisierten Aspartatkinasen kodierenden Gene bzw. Allele werden auch als lysC^{FBR}-Allele bezeichnet. Im Stand der Technik sind zahlreiche lysC^{FBR}-Allele beschrieben, die für Aspartatkinase-Varianten kodieren, welche Aminosäureaustausche im Vergleich zum Wildtypprotein besitzen. Die Kodierregion des Wildtyp lysC-Gens von Corynebacterium glutamicum entsprechend der Zugangsnummer AX756575 der NCBI Datenbank ist in SEQ ID NO:9 und das von diesem Gen kodierte Polypeptid in SEQ ID NO:10 dargestellt.

Die für die Massnahmen der Erfindung eingesetzten L-Lysin produzierenden coryneformen Bakterien verfügen bevorzugt über ein lysC-Allel, das für eine Aspartatkinasevariante kodiert, welche die Aminosäuresequenz von SEQ ID NO:10 besitzt, wobei diese eine oder mehrere der Aminosäureaustausche ausgewählt aus der Gruppe:
LysC A279T (Austausch von L-Alanin an Position 279 des kodierten Aspartatkinaseproteins gemäß SEQ ID NO: 10 gegen L-Threonin; siehe US 5,688,671 und Zugangsnummern E06825, E06826, E08178 und 174588 bis I74597),
LysC A279V (Austausch von L-Alanin an Position 279 des kodierten Aspartatkinaseproteins gemäß SEQ ID NO: 10 gegen L-Valin, siehe JP 6-261766 und Zugangsnummer E08179),
LysC L297Q (Austausch von L-Leucin an Position 297 des kodierten Aspartatkinaseproteins gemäß SEQ ID NO: 10 gegen L-Glutamin; siehe DE 102006026328,
LysC S301F (Austausch von L-Serin an Position 301 des kodierten Aspartatkinaseproteins gemäß SEQ ID NO: 10 gegen L-Phenylalanin; siehe US 6,844,176 und Zugangsnummer E08180),
LysC S301Y (Austausch von L-Serin an Position 301 des kodierten Aspartatkinaseproteins gemäß SEQ ID NO: 10 gegen L-Tyrosin, siehe Kalinowski et al. (Molecular and General Genetics 224, 317-324 (1990)) und Zugangsnummer X57226),
LysC T308I (Austausch von L-Threonin an Position 308 des kodierten Aspartatkinaseproteins gemäß SEQ ID NO: 10 gegen L-Isoleucin; siehe JP 6-261766 und Zugangsnummer E08181)
LysC T311I (Austausch von L-Threonin an Position 311 des kodierten Aspartatkinaseproteins gemäß SEQ ID NO: 10 gegen L-Isoleucin; siehe WO 00/63388 und US 6,893,848),
LysC S317A (Austausch von L-Serin an Position 317 des kodierten Aspartatkinaseproteins gemäß SEQ ID NO: 10 gegen L-Alanin; siehe US 5,688,671 und Zugangsnummer 174589),
LysC R320G (Austausch von L-Arginin an Position 320 des kodierten Aspartatkinaseproteins gemäß SEQ ID NO: 10 gegen Glycin; siehe Jetten et al. (Applied Microbiology and Biotechnology 43, 76-82 (995)) und Zugangsnummer L27125),
LysC G345D (Austausch von Glycin an Position 345 des kodierten Aspartatkinaseproteins gemäß SEQ ID NO: 10 gegen L-Asparaginsäure; siehe Jetten et al. (Applied Microbiology and Biotechnology 43, 76-82 (995)) und Zugangsnummer L16848),
LysC T380I (Austausch von L-Threonin an Position 380 des kodierten Aspartatkinaseproteins gemäß SEQ ID NO: 10 gegen L-Isoleucin; siehe WO 01/49854 und Zugangsnummer AX192358), und
LysC S381F (Austausch von L-Serin an Position 381 des kodierten Aspartatkinaseproteins gemäß SEQ ID NO: 10 gegen L-Phenylalanin; siehe EP 0435132)
umfasst.

Besonders bevorzugt werden das lysC^{FBR}-Allel lysC T311I (Austausch von Threonin an Position 311 des kodierten Aspartatkinaseproteins gemäß SEQ ID NO: 10 gegen Isoleucin) und ein lysC^{FBR}-Allel enthaltend mindestens einen Austausch ausgewählt aus der Gruppe A279T (Austausch von Alanin an Position 279 des kodierten Aspartatkinaseproteins gemäß SEQ ID NO: 10 gegen Threonin), S381F (Austausch von Serin an Position 381 des kodierten Aspartatkinaseproteins gemäß SEQ ID NO: 10 gegen Phenylalanin) und S317A (Austausch von Serin an Position 317 des kodierten Aspartatkinaseproteins gemäß SEQ ID NO: 10 gegen Alanin).

Ganz besonders bevorzugt wird das lysC^{FBR}-Allel lysC T311I (Austausch von Threonin an Position 311 des kodierten Aspartatkinaseproteins gemäß SEQ ID NO: 10 gegen Isoleucin).

Der Stamm DSM 16833 (WO 06/063660) besitzt ein lysC^{FBR}-Allel, das für ein Aspartatkinaseprotein kodiert, welches den Aminosäureaustausch T311I enthält.

Der Stamm NRRL B-11474 (US 4,275,157)besitzt ein lysC^{FBR}-Allel, das für ein Aspartatkinaseprotein kodiert, welches die Aminosäureaustausche A279T und S381F enthält.

Nach der oben beschriebenen Art und Weise wurde, ausgehend von Stamm DSM 16833, eine als DM1915 bezeichnete Mutante isoliert, die ein rel-Allel enthält, das für ein Polypeptid kodiert, bei dem an Position 38 der Aminosäuresequenz L-Leucin enthalten ist. Die Nukleotidsequenz der Kodierregion des rel-Allels der Mutante DM1915 ist als SEQ ID NO:5 und die Aminosäuresequenz des kodierten Polypeptids als SEQ ID NO:6 bzw. 8 dargestellt.

Darüberhinaus können L-Lysin ausscheidende coryneforme Bakterien verwendet werden, die Eigenschaften besitzen, wie sie aus dem Stand der Technik bekannt sind.

L-Tryptophan produzierende coryneforme Bakterien besitzen typischerweise eine "feed back" resistente bzw. desensibilisierte Anthranilatsynthase. Unter "feed back" resistenter Anthranilatsynthase (TrpE) versteht man Anthranilatsynthasen, die im Vergleich zur Wildform eine um mindestens 5 bis 10%, oder mindestens 10% bis 15% oder mindestens 10% bis 20% geringere Empfindlichkeit gegenüber der Hemmung durch Tryptophan oder 5-Fluorotryptophan (Matsui et al., Journal of Bacteriology 169 (11): 5330 - 5332(1987)) oder ähnliche Analoga aufweisen. Die für diese desensibilisierten Anthranilatsynthasen kodierenden Gene bzw. Allele werden auch als trpE^{FBR} -Allele bezeichnet. Beispiele für derartige Mutanten bzw. Allele sind beispielsweise in der US 6180373 und EP0338474 beschrieben.

L-Prolin produzierende coryneforme Bakterien besitzen unter anderem eine γ-Glutamylkinase (ProB), welche an Aminosäureposition 149 oder vergleichbarer Position eine andere proteinogene Aminosäure aufweist als Glycin, vorzugsweise L-Asparaginsäure (WO006066758).

L-Valin produzierende coryneforme Bakterien besitzen typischerweise eine "feed back" resistente bzw. desensibilisierte Acetolactat Synthase (Acetohydroxyacid Synthase; EC Nr. 2.2.1.6).

Unter "feed back" resistenter Acetolactat Synthase versteht man eine Acetolactat Synthase die im Vergleich zur Wildform eine geringere Empfindlichkeit gegenüber der Hemmung durch eine oder mehrere der Aminosäuren ausgewählt aus der Gruppe L-Valin, L-Isoleucin und L-Leucin, bevorzugt L-Valin aufweist.

Die Acetolactat Synthase (IlvB, IlvN) von Corynebacterium besteht aus einer sogenannten grossen vom ilvB-Gen kodierten Untereinheit und einer sogenannten kleinen vom ilvN-Gen kodierten Untereinheit (Keilhauer et al., Journal of Bacteriology 175(17), 5595-5603 (1993)). In der WO 05/003357 und bei Elisakova et al. (Applied and Environmental Microbiology 71(1):207-13 (2005)) wird von Varianten der IlvN-Untereinheit berichtet, die der Acetolactat Synthase Resistenz gegenüber L-Valin, L-Isoleucin und L-Leucin verleihen. Eine Variante enthält an Position 21 der Aminosäuresequenz L-Asparaginsäure anstelle von L-Isoleucin (IlvN I21D) und an Position 22 L-Phenylalanin anstelle von L-Isoleucin (IlvN I22F). Die zweite Variante enthält an Position 20 der Aminosäuresequenz L-Asparaginsäure anstelle Glycin (IlvN G20D), an Position 21 der Aminosäuresequenz L-Asparaginsäure anstelle von L-Isoleucin (IlvN I21D) und an Position 22 L-Phenylalanin anstelle von L-Isoleucin (IlvN I22F).

L-Isoleucin produzierende coryneforme Bakterien besitzen typischerweise eine "feed back" resistente bzw. desensibilisierte Threonin-Dehydratase (= Threonin-Deaminase).

Unter "feed back" resistenter Threonin-Dehydratase versteht man eine Threonin-Dehydratase (EC Nr. 4.3.1.19), die im Vergleich zur Wildform eine geringere Empfindlichkeit gegenüber der Hemmung durch L-Isoleucin aufweist. Die für diese desensibilisierten Threonindehydratase kodierenden Gene bzw. Allele werden auch als ilvA^{FBR} -Allele bezeichnet.

Die Kodierregion des Wildtyp ilvA-Gens von Corynebacterium glutamicum entsprechend den Zugangsnummer L01508 und NC_006958 der NCBI Datenbank ist in SEQ ID NO:17 und das von diesem Gen kodierte Polypeptid in SEQ ID NO:18 dargestellt.

Die in der US 6,107,063 und bei Morbach et al. (Applied and Environmental Microbiology 61 (12), 4315-4320 (1995)) beschriebenen Threonindehydratasevarianten enthalten einen oder mehreren der Aminosäureaustausche ausgewählt aus der Gruppe:
IlvA M199V (Austausch von L-Methionin an Position 199 des kodierten Threonindehydrataseproteins gemäß SEQ ID NO: 18 gegen L-Valin; siehe US 6,107,063),
IlvA A257G (Austausch von L-Alanin an Position 257 des kodierten Threonindehydrataseproteins gemäß SEQ ID NO: 18 gegen L-Arginin; siehe US 6,107,063),
IlvA H278R (Austausch von L-Histidin an Position 278 des kodierten Threonindehydrataseproteins gemäß SEQ ID NO: 18 gegen L-Arginin; siehe US 6,107,063),
IlvA V323A(Austausch von L-Valin an Position 323 des kodierten Threonindehydrataseproteins gemäß SEQ ID NO: 18 gegen L-Alanin; siehe Morbach et al.),
IlvA L351S (Austausch von L-Leucin an Position 351 des kodierten Threonindehydrataseproteins gemäß SEQ ID NO: 18 gegen L-Serin; siehe US 6,107,063)
IlvA D378G(Austausch von L-Asparaginsäure an Position 378 des kodierten Threonindehydrataseproteins gemäß SEQ ID NO: 18 gegen Glycin; siehe Morbach et al.)

Die erhaltenen Mutanten zeigen eine im Vergleich zum eingesetzten Ausgangsstamm bzw. Elternstamm erhöhte Ausscheidung bzw. Produktion der gewünschten Aminosäure in einem Fermentationsprozess.

Gegenstand der Erfindung ist ebenfalls ein isoliertes Polynukleotid, das für ein Polypeptid mit GTP-Pyrophosphatkinase Enzymaktivität kodiert, welches an Position 38 bzw. an einer entsprechenden oder vergleichbaren Position der Aminosäuresequenz eine der proteinogenen Aminosäuren ausgenommen L-Prolin enthält, wobei der Austausch gegen L-Leucin bevorzugt ist.

Das erfindungsgemäße Polynukleotid kann aus einer erfindungsgemäßen Mutante isoliert werden.

Weiterhin können für die Mutagenese des rel-Gens in-vitro Methoden eingesetzt werden. Bei der Verwendung von in-vitro Methoden werden isolierte Polynukleotide, welche ein rel-Gen eines coryneformen Bakteriums, vorzugsweise das im Stand der Technik beschriebene Wildtyp-Gen von Corynebacterium glutamicum, enthalten, einer mutagenen Behandlung unterzogen.

Bei den isolierten Polynukleotiden kann es sich beispielsweise um isolierte Gesamt-DNA bzw. chromosomale DNA oder auch um Amplifikate des rel-Gens handeln, die mit Hilfe der Polymerasekettenreaktion (PCR) hergestellt wurden. Derartige Amplifikate werden auch als PCR-Produkte bezeichnet. Anleitungen zur Amplifikation von DNA-Sequenzen mit Hilfe der Polymerase-Kettenreaktion findet der Fachmann unter anderem im Handbuch von Gait: Oligonucleotide Synthesis: A Practical Approach (IRL Press, Oxford, UK, 1984) und bei Newton und Graham: PCR (Spektrum Akademischer Verlag, Heidelberg, Deutschland, 1994). Es ist ebenfalls möglich, das zu mutagenisierende rel-Gen zunächst in einen Vektor, beispielsweise in einen Bakteriophagen oder in ein Plasmid einzubauen.

Geeignete Methoden für die in-vitro Mutagenese sind unter anderem die Behandlung mit Hydroxylamin nach Miller (Miller, J. H.: A Short Course in Bacterial Genetics. A Laboratory Manual and Handbook for Escherichia coli and Related Bacteria, Cold Spring Harbor Laboratory Press, Cold Spring Harbor, 1992), die Verwendung mutagener Oligonukleotide (T. A. Brown: Gentechnologie für Einsteiger, Spektrum Akademischer Verlag, Heidelberg, 1993 und R. M. Horton: PCR-Mediated Recombination and Mutagenesis, Molecular Biotechnology 3, 93-99 (1995)) und der Einsatz einer Polymerasekettenreaktion unter Verwendung einer DNA-Polymerase, die eine hohe Fehlerquote aufweist. Eine derartige DNA-Polymerase ist beispielsweise die Mutazyme DNA Polymerase (GeneMorph PCR Mutagenesis Kit, Nr.600550) der Firma Stratagene (LaJolla, CA, USA).

Weitere Anleitungen und Übersichten zur Erzeugung von Mutationen in-vivo oder in-vitro können dem Stand der Technik und bekannten Lehrbüchern der Genetik und Molekularbiologie wie z.B. dem Lehrbuch von Knippers ("Molekulare Genetik", 6. Auflage, Georg Thieme Verlag, Stuttgart, Deutschland, 1995), dem von Winnacker ("Gene und Klone", VCH Verlagsgesellschaft, Weinheim, Deutschland, 1990) oder dem von Hagemann ("Allgemeine Genetik", Gustav Fischer Verlag, Stuttgart, 1986) entnommen werden.

Die Beschreibung offenbart weiterhin ein isoliertes Polynukleotid, dass für ein Polypeptid mit GTP-Pyrophosphatkinase-Enzymaktivität kodiert, welches die Aminosäuresequenz von SEQ ID NO:2 umfasst, wobei an Position 38 der Aminosäuresequenz eine der proteinogenen Aminosäuren ausgenommen L-Prolin enthalten ist. Bevorzugt wird der Austausch gegen L-Leucin. Gegebenenfalls ist an Position 262 von SEQ ID NO:2 L-Glutaminsäure enthalten.

Die Beschreibung offenbart weiterhin ein isoliertes Polynukleotid, dass für ein Polypeptid mit GTP-Pyrophosphatkinase-Enzymaktivität kodiert, welches eine Aminosäuresequenz mit einer Länge von 760 Aminoäuren umfasst und wobei an Position 38 eine der proteinogenen L-Aminosäuren ausgenommen L-Prolin, vorzugsweise L-Leucin, enthalten ist.

Die Beschreibung offenbart weiterhin ein isoliertes Polynukleotid, das für ein Polypeptid mit GTP-Pyrophosphatkinase Enzymaktivität kodiert, das an Position 19 bis 57 der Aminosäuresequenz die Aminosäuresequenz entsprechend Position 19 bis 57 von SEQ ID NO:6 bzw. 8 enthält. Bevorzugt enthält die Aminosäuresequenz des kodierten Polypeptids eine Aminosäuresequenz entsprechend Position 9 bis 107 von SEQ ID NO:6 bzw. 8 oder Position 9 bis 207 von SEQ ID NO:6 bzw. 8 oder Position 9 bis 407 von SEQ ID NO:6 bzw. 8 oder Position 9 bis 607 von SEQ ID NO:6 bzw. 8 oder Position 9 bis 707 von SEQ ID NO:6 bzw. 8 oder Position 2 bis 707 von SEQ ID NO:6 bzw. 8 oder Position 9 bis 757 von SEQ ID NO:6 bzw. 8 oder Position 2 bis 757 von SEQ ID NO:6 bzw. 8 oder Position 2 bis 758 von SEQ ID NO:6 bzw. 8 oder Position 2 bis 759 von SEQ ID NO:6 bzw. 8, wobei gegebenenfalls an Position 262 L-Glutaminsäure enthalten ist. Ganz besonders bevorzugt umfasst die Länge des kodierten Polypeptids 760 Aminosäuren.

Die Beschreibung offenbart weiterhin ein isoliertes Polynukleotid, dass für ein Polypeptid mit GTP-Pyrophosphatkinase Enzymaktivität kodiert, welches an Position 38 der Aminosäuresequenz bzw. einer entsprechenden oder vergleichbaren Position eine der proteinogenen Aminosäuren ausgenommen L-Prolin, bevorzugt L-Leucin, enthält und das eine Nukleotidsequenz umfasst, welche mit der Nukleotidsequenz eines Polynukleotids identisch ist, das durch eine Polymerasekettenreaktion (PCR) unter Verwendung des Primerpaars erhältlich ist, deren Nukleotidsequenzen jeweils mindestens 15 aufeinanderfolgende Nukleotide umfassen, die aus der Nukleotidsequenz zwischen Position 1 und 861, bevorzugt zwischen Position 1 und 750, von SEQ ID NO:3 oder SEQ ID NO:7 und aus der komplementären Nukleotidsequenz zwischen Position 3800 und 865, bevorzugt zwischen Position 3800 und 3031, von SEQ ID NO:3 oder SEQ ID NO:7 ausgewählt werden. Ein Beispiel für ein derartiges Primerpaar ist in SEQ ID NO:19 und SEQ ID N0:20 dargestellt. Als Ausgangsmaterial ("template"-DNA) wird chromosomale DNA coryneformer Bakterien bevorzugt, die insbesondere mit einem Mutagen behandelt worden sind. Besonders bevorzugt wird die chromosomale DNA der Gattung Corynebacterium und ganz besonders bevorzugt die der Art Corynebacterium glutamicum.

Die Beschreibung offenbart weiterhin ein isoliertes Polynukleotid, das mit der zu SEQ ID NO:5 komplementären Nukleotidsequenz unter stringenten Bedingungen hybridisiert und für ein Polypeptid mit GTP-Pyrophosphatkinase Enzymaktivität kodiert, welches an Position 38 der Aminosäuresequenz bzw. einer entsprechenden oder vergleichbaren Position eine der proteinogenen Aminosäuren ausgenommen L-Prolin, bevorzugt L-Leucin enthält.

Anleitungen zur Hybridisierung von Nukleinsäuren bzw. Polynukleotiden findet der Fachmann unter anderem im Handbuch "The DIG System Users Guide for Filter Hybridization" der Firma Boehringer Mannheim GmbH (Mannheim, Deutschland, 1993) und bei Liebl et al. (International Journal of Systematic Bacteriology 41: 255-260 (1991)). Die Hybridisierung findet unter stringenten Bedingungen statt, das heißt, es werden nur Hybride gebildet, bei denen die Sonde d.h. ein Polynukleotid umfassend die zu SEQ ID NO:5 komplementäre Nukleotidsequenz und die Zielsequenz, d. h. die mit der Sonde behandelten bzw. identifizierten Polynukleotide, mindestens 90% identisch sind. Es ist bekannt, dass die Stringenz der Hybridisierung einschließlich der Waschschritte durch Variieren der Pufferzusammensetzung, der Temperatur und der Salzkonzentration beeinflusst bzw. bestimmt wird. Die Hybridisierungsreaktion wird im Allgemeinen bei relativ niedriger Stringenz im Vergleich zu den Waschschritten durchgeführt (Hybaid Hybridisation Guide, Hybaid Limited, Teddington, UK, 1996).

Für die Hybridisierungsreaktion kann beispielsweise ein Puffer entsprechend 5x SSC-Puffer bei einer Temperatur von ca. 50°C - 68°C eingesetzt werden. Dabei können Sonden auch mit Polynukleotiden hybridisieren, die weniger als 90% Identität zur Nukleotidequenz der eingesetzten Sonde aufweisen. Solche Hybride sind weniger stabil und werden durch Waschen unter stringenten Bedingungen entfernt. Dies kann beispielsweise durch Senken der Salzkonzentration auf 2x SSC und gegebenenfalls nachfolgend 0,5x SSC (The DIG System User's Guide for Filter Hybridisation, Boehringer Mannheim, Mannheim, Deutschland, 1995) erreicht werden, wobei eine Temperatur von ca. 50°C - 68°C, ca. 52°C - 68°C, ca. 54°C - 68°C, ca. 56°C - 68°C, ca. 58°C - 68°C, ca. 60°C - 68°C, ca. 62°C - 68°C, ca. 64°C - 68°C, ca. 66°C - 68°C eingestellt wird. Temperaturbereiche von ca. 64°C - 68°C oder ca. 66°C - 68°C werden bevorzugt. Es ist gegebenenfalls möglich, die Salzkonzentration bis auf eine Konzentration entsprechend 0,2x SSC oder 0,1x SSC zu senken. Gegebenenfalls enthält der SSC-Puffer Natriumdodecylsulfat (SDS) in einer Konzentration von 0,1%. Durch schrittweise Erhöhung der Hybridisierungstemperatur

in Schritten von ca. 1 - 2°C von 50°C auf 68°C können Polynukleotidfragmente isoliert werden, die mindestens 90% oder mindestens 91%, bevorzugt mindestens 92% oder mindestens 93% oder mindestens 94% oder mindestens 95% oder mindestens 96% und ganz besonders bevorzugt mindestens 97% oder mindestens 98% oder mindestens 99% Identität zur Sequenz bzw. komplementären Sequenz der eingesetzten Sonde besitzen und für ein Polypeptid mit GTP-Pyrophosphatkinase Enzymaktivität kodieren, welches den erfindungsgemäßen Aminosäureaustausch enthält. Die Nukleotidsequenz des auf diese Weise erhaltenen Polynukleotids wird mit bekannten Methoden bestimmt. Weitere Anleitungen zur Hybridisierung sind in Form sogenannter Kits am Markt erhältlich (z.B. DIG Easy Hyb von der Firma Roche Diagnostics GmbH, Mannheim, Deutschland, Catalog No. 1603558). Die so erhaltenen Nukleotidsequenzen kodieren für Polypeptide mit GTP-Pyrophosphatkinase Enzymaktivität, die mindestens 90% bevorzugt mindestens 92% oder mindestens 94% oder mindestens 96%, und ganz besonders bevorzugt mindestens 97% oder mindestens 98% oder mindestens 99% identisch sind mit der Aminosäuresequenz von SEQ ID NO:6 bzw. SEQ ID NO:8 und den erfindungsgemäßen Aminosäureaustausch enthalten, wobei gegebenenfalls an Position 262 L-Glutaminsäure enthalten ist.

Die Beschreibung offenbart weiterhin ein isoliertes Polynukleotid, das für ein Polypeptid mit GTP-Pyrophosphatkinase Enzymaktivität kodiert, welches an Position 38 bzw. einer entsprechenden oder vergleichbaren Position der Aminosäuresequenz eine der proteinogenen Aminosäuren ausgenommen L-Prolin enthält, wobei dem Austausch gegen L-Leucin der Vorzug gegeben wird, und das eine Aminosäuresequenz umfasst, die außerdem zu mindestens 90%, bevorzugt mindestens 92% oder mindestens 94% oder mindestens 96%, und ganz besonders bevorzugt mindestens 97% oder mindestens 98% oder mindestens 99% identisch ist mit der Aminosäuresequenz von SEQ ID NO:6 bzw. SEQ ID NO:8,
wobei gegebenenfalls an Position 262 L-Glutaminsäure enthalten ist.

Die Beschreibung offenbart weiterhin ein isoliertes Polynukleotid, das für ein Polypeptid mit GTP-Pyrophosphatkinase Enzymaktivität kodiert, welches an Position 38 bzw. einer entsprechenden oder vergleichbaren Position der Aminosäuresequenz eine der proteinogenen Aminosäuren ausgenommen L-Prolin enthält, wobei dem Austausch gegen L-Leucin der Vorzug gegeben wird, und das eine Nukleotidsequenz umfasst, die außerdem zu mindestens 90%, bevorzugt mindestens 92% oder mindestens 94% oder mindestens 96%, und ganz besonders bevorzugt mindestens 97% oder mindestens 98% oder mindestens 99% identisch ist mit der Nukleotidsequenz von SEQ ID NO:5, wobei gegebenenfalls an Position 785 Adenin enthalten ist.

Darüberhinaus offenbart die Beschreibung solche isolierten Polynukleotide, die für ein Polypeptid mit GTP-Pyrophosphatkinase-Enzymaktivität kodieren, welches an Position 38 der Aminosäuresequenz bzw. einer entsprechenden oder vergleichbaren Position eine der proteinogenen Aminosäuren ausgenommen L-Prolin, bevorzugt L-Leucin, enthält und die mindestens ein Sequenzmotiv bzw. eine Aminosäuresequenz ausgewählt aus der Gruppe Ser-Gly-Asp/Glu-Pro-Tyr-Ile-Thr/Ile-His-Pro-Leu-Ala-Val, Ala-Ala/Gly-Leu-Leu-His-Asp-Thr-Val-Glu-Asp-Thr und Thr-Pro-Val-Asp-Phe-Ala-Tyr-Ala-Val-His-Thr-Glu-Val-Gly-His-Arg enthalten.

Die Bezeichnungen "Asp/Glu", "Thr/Ile" und "Ala/Gly" bedeuten, dass an der entsprechenden Position "Asp oder Glu" bzw. "Thr oder Ile" oder "Ala oder Gly" enthalten sind.

Die Beschreibung offenbart weiterhin ein isoliertes Polynukleotid, dass für ein Polypeptid mit GTP-Pyrophosphatkinase Enzymaktivität kodiert, welches die Aminosäuresequenz von SEQ ID NO:6 bzw. 8 umfasst, wobei gegebenenfalls an Position 262 L-Glutaminsäure enthalten ist. Gegebenenfalls enthält das kodierte Polypeptid einen (1) oder mehrere konservative Aminosäuraustausch(e). Bevorzugt enthält das Polypeptid höchstens zwei (2), höchstens drei (3), höchstens vier (4) oder höchstens fünf (5) konservative Aminosäureaustausche.

Die Beschreibung offenbart weiterhin ein isoliertes Polynukleotid, dass für ein Polypeptid mit GTP-Pyrophosphatkinase Enzymaktivität kodiert, welches die Aminosäuresequenz von SEQ ID NO:6 bzw. 8 einschließlich einer Verlängerung am N- oder C-Terminus um mindestens eine (1) Aminosäure umfasst. Diese Verlängerung beträgt nicht mehr als 50, 40, 30, 20, 10, 5, 3 oder 2 Aminosäuren bzw. Aminosäurereste. Gegebenenfalls ist an Position 262 von SEQ ID NO:6 bzw. 8 L-Glutaminsäure enthalten.

Gegenstand der Erfindung sind schließlich auch rel-Allele, die für Polypeptid Varianten von SEQ ID NO:6 kodieren, wobei gegebenenfalls an Position 262 L-Glutaminsäure enthalten ist, die eine oder maximal 5 Insertionen oder Deletionen enthalten. Bevorzugt enthalten diese maximal 4, maximal 3 oder maximal 2 Insertionen oder Deletionen von Aminosäuren. Die Sequenzmotive Ser-Gly-Asp/Glu-Pro-Tyr-Ile-Thr/Ile-His-Pro-Leu-Ala-Val und/oder Ala-Ala/Gly-Leu-Leu-His-Asp-Thr-Val-Glu-Asp-Thr und/oder Thr-Pro-Val-Asp-Phe-Ala-Tyr-Ala-Val-His-Thr-Glu-Val-Gly-His-Arg wird/werden durch derartige Insertionen/Deletionen vorzugsweise nicht zerrissen.

Gegenstand der Erfindung ist weiterhin ein isoliertes Polynukleotid, das die Nukleotidsequenz gemäß SEQ ID NO:5 umfasst, wobei an Position 785 von SEQ ID NO:5 gegebenenfalls Adenin enthalten ist.

Die Beschreibung offenbart schließlich ein isoliertes Polynukleotid enthaltend das rel-Allel der Mutante DM1915.

Die Beschreibung offenbart außerdem ein isoliertes Polynukleotid, das einen Teil der Kodierregion eines erfindungsgemäßen rel-Allels umfasst, wobei das isolierte Polynukleotid in jedem Fall den Teil der Kodierregion umfasst, der den Aminosäureaustausch an der Position 38 der Aminosäuresequenz des kodierten Polypeptids enthält.

Insbesondere ist ein Nukleinsäure-Molekül bzw. DNA-Fragment offenbart, das für mindestens eine Aminosäuresequenz entsprechend Position 19 bis 57 von SEQ ID NO:2, oder das für mindestens eine Aminosäuresequenz entsprechend Position 9 bis 107 von SEQ ID NO:2, oder das für mindestens eine Aminosäuresequenz entsprechend Position 9 bis 207 von SEQ ID NO:2, oder das für mindestens eine Aminosäuresequenz entsprechend Position 9 bis 407 von SEQ ID NO:2, oder das für mindestens eine Aminosäuresequenz entsprechend Position 9 bis 607 von SEQ ID NO:2, oder das für mindestens eine Aminosäuresequenz entsprechend Position 9 bis 707 von SEQ ID NO:2 kodiert, oder das für mindestens eine Aminosäuresequenz entsprechend Position 2 bis 707 von SEQ ID NO:2 kodiert, oder das für mindestens eine Aminosäuresequenz entsprechend Position 9 bis 757 von SEQ ID NO:2 kodiert, oder das für mindestens eine Aminosäuresequenz entsprechend Position 2 bis 757 von SEQ ID NO:2 kodiert, oder das für mindestens eine Aminosäuresequenz entsprechend Position 2 bis 758 von SEQ ID NO:2 kodiert, oder das für mindestens eine Aminosäuresequenz entsprechend Position 2 bis 759 von SEQ ID NO:2 kodiert, wobei an der Position entsprechend 38 von SEQ ID NO:2 eine der proteinogenen Aminsäuren ausgenommen L-Prolin, bevorzugt L-Leucin, enthalten ist. Gegebenenfalls ist an Position 262 von SEQ ID NO:2 L-Glutaminsäure enthalten.

Ein Beispiel eines Leserasters umfassend ein Polynukleotid, das für mindestens die Aminosäuresequenz von Position 19 bis 57 entsprechend SEQ ID NO:2 kodiert, wobei an der Position entsprechend 38 der Aminosäuresequenz eine der proteinogenen Aminosäuren (Xaa) ausgenommen L-Prolin enthalten ist, ist nachfolgend aufgeführt:

Es ist ebenfalls als SEQ ID NO:11 dargestellt. Die von diesem Leseraster kodierte Aminosäuresequenz ist als SEQ ID NO:12 dargestellt. Die Position 20 in SEQ ID NO:12 entspricht der Position 38 von SEQ ID NO:2, 4, 6 bzw. 8.

Bevorzugt werden Nukleinsäuremoleküle die für mindestens eine Aminosäuresequenz entsprechend Position 19 bis 57 von SEQ ID NO:6 bzw. 8, oder mindestens entsprechend Position 9 bis 107 von SEQ ID NO: 6 bzw. 8, oder mindestens entsprechend Position 9 bis 207 von SEQ ID NO: 6 bzw. 8, oder mindestens entsprechend Position 9 bis 407 von SEQ ID NO: 6 bzw. 8, oder mindestens entsprechend Position 9 bis 607 von SEQ ID NO: 6 bzw. 8, oder mindestens entsprechend Position 9 bis 707 von SEQ ID NO: 6 bzw. 8 kodiert, oder mindestens entsprechend Position 2 bis 707 von SEQ ID NO: 6 bzw. 8 kodiert, oder mindestens entsprechend Position 9 bis 757 von SEQ ID NO: 6 bzw. 8 kodiert, oder mindestens entsprechend Position 2 bis 757 von SEQ ID NO: 6 bzw. 8 kodiert, oder mindestens entsprechend Position 2 bis 758 von SEQ ID NO: 6 bzw. 8, oder mindestens entsprechend Position 2 bis 759 von SEQ ID NO: 6 bzw. 8 kodieren, wobei gegebenenfalls an Position 262 L-Glutaminsäure enthalten ist.

Ein Beispiel eines Leserasters umfassend ein Polynukleotid, das für mindestens die Aminosäuresequenz entsprechend Position 19 bis 57 von SEQ ID NO:6 bzw. 8 kodiert, ist nachfolgend aufgeführt:

Das Leseraster ist ebenfalls als SEQ ID NO:13 dargestellt. SEQ ID NO:14 zeigt die von diesem Leseraster kodierte Aminosäuresequenz. Die Position 20 in SEQ ID NO:14 entspricht der Position 38 von SEQ ID NO:2, 4, 6 bzw. 8.

Ganz besonders bevorzugt werden Nukleinsäuremoleküle, die mindestens eine Nukleotidsequenz entsprechend Position 805 bis 921 von SEQ ID NO:7, oder mindestens eine Nukleotidsequenz entsprechend Position 655 bis 1071 von SEQ ID NO: 7, oder mindestens eine Nukleotidsequenz entsprechend Position 355 bis 1371 von SEQ ID NO:7, oder mindestens eine Nukleotidsequenz entsprechend Position 55 bis 2671 von SEQ ID NO:7, oder mindestens eine Nukleotidsequenz entsprechend Position 1 bis 2725 von SEQ ID NO:7, umfassen, wobei gegebenenfalls an Position 1535 Adenin enthalten ist.

Die Leseraster, wie sie beispielhaft in SEQ ID NO:11 und 13 als Nukleotidsequenz und in SEQ ID NO:12 und SEQ ID NO:14 in Form der kodierten Aminosäuresequenz gezeigt sind, können darüberhinaus eine oder mehrere Mutationen enthalten, die zu einem oder mehreren konservativen Aminosäureaustauschen führen. Bevorzugt führen die Mutationen zu maximal 4%, zu maximal 2% oder zu maximal 1% konservativen Aminosäureaustauschen. Weiterhin können die erfindungsgemäßen Leseraster eine oder mehrere stille Mutationen enthalten. Bevorzugt enthalten die erfindungsgemäßen Leseraster höchstens 4% und besonders bevorzugt höchstens 2% bis höchstens 1% stille Mutationen.

Die erfindungsgemäßen, isolierten Polynukleotide können dazu verwendet werden, um rekombinante Stämme von Mikroorganismen herzustellen, die im Vergleich zum Ausgangs- bzw. Elternstamm in verbesserter Weise Aminosäuren in das sie umgebende Medium abgeben oder im Zellinneren anhäufen.

Eine verbreitete Methode zum Einbau von Mutationen in Gene coryneformer Bakterien ist die des Allelaustausches, die auch unter der Bezeichnung "gene replacement" bekannt ist. Bei diesem Verfahren wird ein DNA-Fragment, welches die interessierende Mutation enthält, in den gewünschten Stamm eines coryneformen Bakteriums transferiert und die Mutation durch wenigstens zwei Rekombinationsereignisse bzw. "cross over"-Ereignisse in das Chromosom des gewünschten Stammes inkorporiert bzw. die im betreffenden Stamm vorhandene Sequenz eines Gens gegen die mutierte Sequenz ausgetauscht.

Von Schwarzer und Pühler (Bio/Technology 9, 84-87 (1991) wurde diese Methode verwendet um ein lysA-Allel, welches eine Deletion trug, und ein lysA-Allel, welches eine Insertion trug, in das Chromosom von C. glutamicum anstelle des Wildtypgens einzubauen. Von Schäfer et al. (Gene 145, 69-73 (1994)) wurde diese Methode eingesetzt, um eine Deletion in das hom-thrB Operon von C. glutamicum einzubauen. Von Nakagawa et al. (EP 1108790) und Ohnishi et al. (Applied Microbiology and Biotechnology 58(2), 217-223 (2002)) wurde diese Methode eingesetzt, um verschiedene Mutationen ausgehend von den isolierten Allelen in das Chromosom von C. glutamicum einzubauen. Auf diese Weise gelang es Nakagawa et al. eine als Val59Ala bezeichnete Mutation in das Homoserin-Dehydrogenase Gen (hom), eine als Thr311Ile bezeichnete Mutation in das Aspartatkinase-Gen (lysC bzw. ask), eine als Pro458Ser bezeichnete Mutation in das Pyruvat-Carboxylase-Gen (pyc) und eine als Ala213Thr bezeichnete Mutation in das Glucose-6-phoshat-Dehydrogenase Gen (zwf) von C. glutamicum Stämmen einzubauen.

Für ein erfindungsgemäßes Verfahren kann ein erfindungsgemäßes Polynukleotid verwendet werden, welches die gesamte Kodierregion umfasst, wie beispielsweise in SEQ ID NO:5 gezeigt.

In SEQ ID NO:15 ist ein Beispiel für ein Polynukleotid dargestellt, das einen Teil der Kodierregion umfasst.

Das DNA-Fragment enthaltend die interessierende Mutation liegt bei dieser Methode typischerweise in einem Vektor, insbesondere einem Plasmid, vor, das vorzugsweise von dem mit der Mutation zu versehenden Stamm nicht oder nur begrenzt repliziert wird. Als Hilfs- oder Zwischenwirt, in dem der Vektor replizierbar ist, wird im Allgemeinen ein Bakterium der Gattung Escherichia bevorzugt der Spezies Escherichia coli verwendet.

Beispiele für derartige Plasmidvektoren sind die von Schäfer et al. (Gene 145, 69-73 (1994)) beschriebenen pK*mob und pK*mobsacB Vektoren, wie beispielsweise pK18mobsacB, und die in der WO 02/070685 und WO 03/014362 beschriebenen Vektoren. Diese sind in Escherichia coli aber nicht in coryneformen Bakterien replikativ. Besonders geeignet sind Vektoren, die ein konditional negativ dominant wirkendes Gen wie beispielsweise das sacB-Gen (Levansucrase-Gen) von beispielsweise Bacillus oder das galK-Gen (Galaktosekinase-Gen) von beispielsweise Escherichia coli enthalten. (Unter einem konditional negativ dominant wirkenden Gen versteht man ein Gen, das unter bestimmten Bedingungen nachteilig beispielsweise toxisch für den Wirt ist, unter anderen Bedingungen aber keine negativen Auswirkungen auf den das Gen tragenden Wirt hat.) Diese ermöglichen die Selektion auf Rekombinationsereignisse, bei denen der Vektor aus dem Chromosom eliminiert wird. Weiterhin wurde von Nakamura et al. (US-A-6,303,383) ein Temperatur-sensitives Plasmid für coryneforme Bakterien beschrieben, das lediglich bei Temperaturen unterhalb von 31°C replizieren kann.

Der Vektor wird anschließend durch Konjugation beispielsweise nach der Methode von Schäfer (Journal of Bacteriology 172, 1663-1666 (1990)) oder Transformation beispielsweise nach der Methode von Dunican und Shivnan (Bio/Technology 7, 1067-1070 (1989)) oder der Methode von Thierbach et al. (Applied Microbiology and Biotechnology 29, 356-362 (1988)) in das coryneforme Bakterium transferiert. Gegebenenfalls kann der Transfer der DNA auch durch Partikelbeschuss erzielt werden.

Nach homologer Rekombination mittels eines ersten, Integration bewirkenden "cross-over"-Ereignisses und eines geeigneten zweiten, eine Exzision bewirkenden "cross-over"-Ereignisses im Zielgen bzw. in der Zielsequenz erreicht man den Einbau der Mutation und erhält ein rekombinantes Bakterium.

Zur Identifizierung und Charakterisierung der erhaltenen Stämme können unter anderem die Methoden der Southern Blotting Hybridisierung, der Polymerase-Kettenreaktion, der Sequenzbestimmung, die Methode des "Fluorescence Resonance Energy Transfer" (FRET) (Lay et al. Clinical Chemistry 43, 2262-2267 (1997)) oder Methoden der Enzymologie eingesetzt werden.

Die Beschreibung offenbart auch ein Verfahren zur Herstellung eines coryneformen Bakteriums, bei dem man
a) ein erfindungsgemäßes Polynukleotid in ein coryneformes Bakterium transferiert,
b) das im Chromosom des coryneformen Bakteriums vorhandene GTP-Pyrophosphatkinase Gen, das für eine Aminosäuresequenz mit L-Prolin an Position 38 oder an einer vergleichbaren Position der Aminosäuresequenz kodiert, gegen das Polynukleotid aus a) austauscht, das für eine Aminosäuresequenz kodiert, die an der Position 38 oder an einer vergleichbaren Position der Aminosäuresequenz eine andere proteinogene L-Aminosäure, vorzugsweise L-Leucin, besitzt, und
c) das gemäß Schritt a) und b) erhaltene coryneforme Bakterium vermehrt.

Auf diese Weise erhält man ein rekombinantes coryneformes Bakterium, welches anstelle des Wildtyp rel-Gens ein (1) erfindungsgemäßes rel-Allel enthält.

Ein weiteres Verfahren zur Herstellung eines Mikroorganismus besteht darin, dass man
a) ein erfindungsgemäßes Polynukleotid, das für ein Polypeptid mit GTP-Pyrophosphatkinase Enzymaktivität kodiert, in einen Mikrorganismus transferiert,
b) das Polynukleotid in dem Mikroorganismus repliziert, und
c) den gemäß Schritt a) und b) erhaltenen Mikroorganismus vermehrt.

Auf diese Weise erhält man einen rekombinanten Mikroorganismus, welcher mindestens eine (1) Kopie oder mehrere Kopien eines erfindungsgemäßen Polynukleotids enthält.

Weitere Gegenstände der Erfindung sind dementsprechend Wirte bzw. Wirtszellen, bevorzugt Mikroorganismen, besonders bevorzugt coryneforme Bakterien und Bakterien der Gattung Escherichia, die die erfindungsgemäßen Polynukleotide enthalten. Gegenstand der Erfindung sind gleichfalls Mikrorganismen, die unter Verwendung der isolierten Polynukleotide hergestellt wurden. Derartige Mikroorganismen oder Bakterien werden auch als rekombinante Mikroorganismen oder rekombinante Bakterien bezeichnet. In gleicher Weise sind Vektoren, die die erfindungsgemäßen Polynukleotide enthalten, Gegenstand der Erfindung. Schließlich sind Wirte, die diese Vektoren enthalten, ebenfalls Gegenstand der Erfindung.

Die erfindungsgemäßen, isolierten Polynukleotide können ebenfalls zur Erzielung einer Überexpression der von ihnen kodierten Polypeptide verwendet werden.

Unter Überexpression versteht man allgemein eine Erhöhung der intrazellulären Konzentration oder Aktivität einer Ribonukleinsäure, eines Proteins oder eines Enzyms.

Es ist bekannt, dass durch wirtseigene Enzyme - sogenannte Aminopeptidasen - N-terminale Aminosäuren, insbesondere das N-terminale Methionin, vom gebildeten Polypeptid abgespalten werden können. Die genannte Erhöhung der Konzentration oder Aktivität eines Genproduktes lässt sich beispielsweise dadurch erzielen, dass man die Kopienzahl der entsprechenden Polynukleotide um mindestens eine Kopie erhöht.

Eine weit verbreitete Methode zur Erhöhung der Kopienzahl besteht darin, dass man das entsprechende Gen oder Allel in einen Vektor, bevorzugt ein Plasmid, einbaut, der von einem coryneformen Bakterium repliziert wird. Geeignete Plasmidvektoren sind beispielsweise pZ1 (Menkel et al., Applied and Environmental Microbiology (1989) 64: 549-554) oder die von Tauch et al. (Journal of Biotechnology 99, 79-91 (2002)) beschriebenen pSELF-Vektoren. Ein Übersichtsartikel zum Thema Plasmide in Corynebacterium glutamicum findet sich bei Tauch et al. (Journal of Biotechnology 104, 27-40 (2003)).

Eine andere verbreitete Methode zur Erzielung einer Überexpression ist das Verfahren der chromosomalen Genamplifikation. Bei dieser Methode wird mindestens eine zusätzliche Kopie des interessierenden Gens oder Allels in das Chromosom eines coryneformen Bakteriums eingefügt.

In einer Ausführungsform, wie sie beispielsweise bei Reinscheid et al. (Applied and Environmental Microbiology 60, 126-132 (1994)) für das hom-thrB-Operon beschrieben ist, wird ein in C. glutamicum nicht-replikatives Plasmid, welches das interessierende Gen enthält, in ein coryneformes Bakterium transferiert. Nach homologer Rekombination mittels eines "cross over"-Ereignisses enthält der resultierende Stamm mindestens zwei Kopien des betreffenden Gens bzw. Allels.

In einer anderen Ausführungsform, die in der WO 03/040373 und US-2003-0219881-A1 beschrieben ist, wird eine oder mehrere Kopie(n) des interessierenden Gens mittels mindestens zweier Rekombinationsereignisse in einen gewünschten Ort des Chromosoms von C. glutamicum eingefügt. Auf diese Weise wurde beispielsweise eine Kopie eines lysC-Allels, das für eine L-Lysin insensitive Aspartatkinase kodiert, in das gluB-Gen von C. glutamicum eingebaut.

In einer weiteren Ausführungsform, die in der WO 03/014330 und US-2004-0043458-A1 beschrieben ist, wird mittels mindestens zweier Rekombinationsereignisse am natürlichen Ort mindestens eine weitere Kopie, vorzugsweise in tandem-Anordnung zu dem bereits vorhandenen Gen oder Allel, des interessierenden Gens eingebaut. Auf diese Weise wurde beispielsweise eine tandem-Duplikation eines lysC^{FBR}-Allels am natürlichen lysC-Genort erzielt.

Eine weitere Methode zur Erzielung einer Überexpression besteht darin, das entsprechende Gen bzw. Allel in funktioneller Weise (operably linked) mit einem Promotor bzw. einer Expressionskassette zu verknüpfen. Geeignete Promotoren für Corynebacterium glutamicum sind beispielsweise in dem Übersichtsartikel von Patek et al. (Journal of Biotechnology 104(1-3), 311-323 (2003) beschrieben. In gleicher Weise können die von Vasicova et al (Journal of Bacteriology 181, 6188-6191 (1999)) beschrieben Varianten des dapA-Promotors, beispielsweise der Promotor A25 eingesetzt werden. Weiterhin kann der gap-Promotor von Corynebacterium glutamicum (EP 06007373) verwendet werden. Schließlich können die hinlänglich bekannten, von Amann et al. (Gene 69(2), 301-315 (1988)) und Amann und Brosius (Gene 40(2-3), 183-190 (1985)) beschriebenen Promotoren T3, T7, SP6, M13, lac, tac und trc verwendet werden. Ein derartiger Promotor kann beispielsweise stromaufwärts des rel-Allels, typischerweise im Abstand von ungefähr 1 - 500 Nukleotiden vom Startkodon, eines rekombinanten coryneformen Bakteriums eingefügt werden, welches für ein Protein kodiert, welches anstelle der an Position 38 natürlicherweise vorhanden Aminosäure L-Prolin eine andere proteinogene Aminsäure enthält. Ein derartiger Promotor kann naturgemäß ebenfalls stromaufwärts der Kodierregion des rel-Allels einer erfindungsgemäßen Mutante eingefügt werden. Es ist weiterhin möglich ein erfindungsgemäßes isoliertes Polynukleotid, das für eine erfindungsgemäße Variante der GTP-Pyrophosphatkinase kodiert, mit einem Promotor zu verknüpfen und die erhaltene Expressionseinheit in ein extrachromosomal replizierendes Plasmid oder in das Chromosom eines coryneformen Bakteriums einzubauen.

Darüberhinaus kann die Promotor- und Regulationsregion oder die Ribosomenbindungsstelle, die sich stromaufwärts des Strukturgens befindet, mutiert werden. Durch Maßnahmen zur Verlängerung der Lebensdauer der mRNA wird ebenfalls die Expression verbessert. Weiterhin wird durch Verhinderung des Abbaus des Enzymproteins ebenfalls die Enzymaktivität verstärkt. Alternativ kann weiterhin eine Überexpression des betreffenden Gens oder Allels durch Veränderung der Medienzusammensetzung und Kulturführung erreicht werden.

Durch die Maßnahmen der Überexpression wird die Aktivität oder Konzentration des entsprechenden Proteins im allgemeinen um mindestens 10%, 25%, 50%, 75%, 100%, 150%, 200%, 300%, 400% oder 500%, maximal bis 1000% oder 2000%, bezogen auf die Aktivität oder Konzentration des Proteins im Ausgangs-Mikroorganismus bzw. Elternstammes erhöht. Unter einem Ausgangs-Mikroorganismus oder Elternstamm versteht man einen Mikroorganismus, an dem die Massnahmen der Erfindung durchgeführt werden.

Die Konzentration des Proteins kann über 1- und 2-dimensionale Proteingelauftrennung und anschließende optische Identifizierung der Proteinkonzentration mit entsprechender Auswertesoftware im Gel bestimmt werden. Eine gebräuchliche Methode zur Präparation der Proteingele bei coryneformen Bakterien und zur Identifizierung der Proteine ist die von Hermann et al. (Electrophoresis, 22:1712-23 (2001)) beschriebene Vorgehensweise. Die Proteinkonzentration kann ebenfalls durch Western-Blot-Hybridisierung mit einem für das nachzuweisende Protein spezifischen Antikörper (Sambrook et al., Molecular cloning: a laboratory manual. 2nd Ed. Cold Spring Harbor Laboratory Press, Cold Spring Harbor, N.Y., 1989) und anschließender optischer Auswertung mit entsprechender Software zur Konzentrationsbestimmung (Lohaus und Meyer (1998) Biospektrum 5:32-39; Lottspeich, Angewandte Chemie 38: 2630-2647 (1999)) bestimmt werden.

Die Beschreibung offenbart Verfahren zur Überexpression der erfindungsgemäßen GTP-Pyrophosphatkinasen. Ein Verfahren zur Überexpression besteht unter anderem darin, dass man die Kopienzahl eines erfindungsgemässen Polynukleotids um mindestens eine (1) oder um mehrere Kopien erhöht. Ein weiteres Verfahren besteht darin, dass man einen Promotor funktionell mit dem Polynukleotid verknüpft.

Die Beschreibung offenbart weiterhin Mikroorganismen, die eine erhöhte Konzentration oder Aktivität der erfindungsgemäßen GTP-Pyrophosphatkinase Varianten in ihrem Zellinneren aufweisen.

Zusätzlich kann es für die verbesserte Produktion von L-Aminosäuren vorteilhaft sein, in den erfindungsgemäßen Mutanten oder rekombinanten Stämme verschiedene Gene zu überexprimieren. Die Verwendung endogener Gene wird im Allgemeinen bevorzugt.

Unter "endogenen Genen" oder "endogenen Nukleotidsequenzen" versteht man die in der Population einer Art vorhandenen Gene bzw. Nukleotidsequenzen oder Allele.

So kann für die Herstellung von L-Lysin eines oder mehrere der Gene, ausgewählt aus der Gruppe
- ein für ein Dihydrodipicolinat-Synthase (DapA, EC Nr. 4.2.1.52) kodierendes Gen dapA, wie beispielsweise das in der EP 0 197 335 beschriebene dapA-Gen des Wildtyps von Corynebacterium glutamicum,
- ein für eine Diaminopimelat-Decarboxylase (LysA, EC Nr. 4.1.1.20) kodierendes lysA-Gen, wie beispielsweise das in in der US 6,090,597 beschriebene lysA-Gen von Corynebacterium glutamicum ATCC13869,
- ein für eine Glucose-6-Phosphat Dehydrogenase (Zwf, EC Nr. 1.1.1.49) kodierendes Gen zwf, wie beispielsweise das in der JP-A-09224661 und EP-A-1108790 beschriebene zwf-Gen des Wildtyps von Corynebacterium glutamicum,
- die in der US-2003-0175911-A1 beschriebenen zwf-Allele von Corynebacterium glutamicum, die für ein Protein mit Glucose-6-Phosphat Dehydrogenase Aktivität kodieren, bei dem beispielsweise das L-Alanin an Position 243 der Aminosäuresequenz durch L-Threonin ersetzt ist oder bei dem die L-Asparaginsäure an Position 245 durch L-Serin ersetzt ist,
- ein für eine Pyruvat-Carboxylase (Pyc, EC Nr. 6.4.1.1) kodierendes Gen pyc, wie beispielsweise das in der DE-A-198 31 609 und EP 1108790 beschriebene pyc-Gen des Wildtyps von Corynebacterium glutamicum,
- das für das in der EP 1 108 790 beschriebene pyc-Allel von Corynebacterium glutamicum, das für ein Protein mit Pyruvat-Carboxylase Aktivität kodiert, bei dem L-Prolin an Position 458 der Aminosäuresequenz durch L-Serin ersetzt ist,
- die in der WO 02/31158 und insbesondere EP1325135B1 beschriebene pyc-Allele von Corynebacterium glutamicum, die für Proteine mit Pyruvat-Carboxylase Aktivität kodieren, welche einen oder mehrere der Aminosäureaustausche ausgewählt aus der Gruppe L-Valin an Position 1 ersetzt durch L-Methionin, L-Glutaminsäure an Position 153 ersetzt durch L-Asparaginsäure, L-Alanin an Position 182 ersetzt durch L-Serin, L-Alanin an Position 206 ersetzt durch L-Serin, L-Histidin an Position 227 ersetzt durch L-Arginin, L-Alanin an Position 455 ersetzt durch Glycin und L-Asparaginsäure an Position 1120 ersetzt durch L-Glutaminsäure tragen
- ein für eine Aspartatkinase (LysC, EC Nr. 2.7.2.4)kodierendes lysC Gen wie beispielsweise das als SEQ ID NO:281 in der EP-A-1108790 (Siehe auch Zugangsnummer AX120085 und 120365) und das als SEQ ID NO:25 in der WO 01/00843 (Siehe Zugangsnummer AX063743) beschriebene von lysC-Gen des Wildtyps von Corynebacterium glutamicum,
- ein für eine feed-back resistente Aspartatkinase Variante kodierendes lysC^{FBR} Allel, insbesondere entsprechend Tabelle 1,
- ein für ein Lysin-Export-Protein (LysE) kodierendes Gen lysE, wie beispielsweise das in der DE-A-195 48 222 beschriebene lysE-Gen von des Wildtyps Corynebacterium glutamicum,
- das für eine Aspartat-Aminotransferase (Aat, EC Nr. 2.6.1.1) kodierende aat-Gen (Das aat-Gen von Corynebacterium glutamicum ATCC13032 ist beispielsweise bei Kalinowski et al (Journal of Biotechnology 104 (1-3), 5-25 (2003); siehe auch Zugangsnummer NC_006958) beschrieben. Es wird dort als aspB-Gen bezeichnet. In der US 6,004,773 wird ein für eine Aspartat Aminotransferase kodierendes Gen als aspC bezeichnet. Marienhagen et al (Journal of Bacteriology 187 (22), 7693-7646 (2005) bezeichnen das aat-Gen als aspT-Gen.)),
- das für das Zwa1-Protein kodierende Gen zwa1 des Wildtyps von Corynebacterium glutamicum (US 6,632,644)
überexprimiert werden.

Weiterhin kann es für die Produktion von L-Lysin vorteilhaft sein, neben der Verwendung der erfindungsgemäßen Allele des rel-Gens gleichzeitig, ggf. bei gleichzeitiger Überexpression von mindestens einem der Gene ausgewählt aus der vorstehend genannten Gruppe von Genen, eines oder mehrere der endogenen Gene, ausgewählt aus der Gruppe
- ein für die Glucose-6-Phosphat-Isomerase (Pgi, EC Nr. 5.3.1.9) kodierendes Gen pgi, wie beispielsweise das in der US 6,586,214 und US 6,465,238 beschriebene pgi-Gen von Corynebacterium glutamicum,
- ein für die Homoserin-Dehydrogenase(Hom, EC Nr. 1.1.1.3) kodierendes Gen hom, wie beispielsweise das in der EP-A-0131171 beschriebene hom-Gen von Corynebacterium glutamicum,
- ein für die Homoserin-Kinase (ThrB, EC Nr. 2.7.1.39) kodierendes Gen thrB, wie beispielsweise das von Peoples et al. (Molecular Microbiology 2 (1988): 63 - 72)) beschriebene thrB-Gen von Corynebacterium glutamicum und
- ein für die Phosphofruktokinase(PfkB, EC Nr. 2.7.1.56) kodierendes Gen pfkB, wie beispielsweise das in der WO 01/00844 (Sequenz Nr. 57) beschriebene pfkB-Gen von Corynebacterium glutamicum,
- ein für die Malat-Dehydrogenase (Mdh, EC Nr. 1.1.1.37) kodierendes Gen mdh, wie beispielsweise in der WO 02/02778 beschrieben,
- ein für die Malat-Chinon Oxidoreduktase (Mqo, EC Nr. 1.1.99.16) kodierendes Gen mqo, wie beispielsweise in der US 7,094,106 und PCT/EP2005/057216 beschrieben
abzuschwächen oder auszuschalten.

Der Begriff "Abschwächung" beschreibt in diesem Zusammenhang die Verringerung oder Ausschaltung der intrazellulären Aktivität eines oder mehrerer Enzyme (Proteine) in einem Mikroorganismus, die durch die entsprechende DNA kodiert werden, indem man beispielsweise einen schwachen Promotor verwendet oder ein Gen bzw. Allel verwendet, das für ein entsprechendes Enzym mit einer niedrigen Aktivität kodiert bzw. das entsprechende Gen oder Enzym (Protein) inaktiviert und gegebenenfalls diese Maßnahmen kombiniert.

Durch die Maßnahmen der Abschwächung wird die Aktivität oder Konzentration des entsprechenden Proteins im allgemeinen auf 0 bis 75%, 0 bis 50%, 0 bis 25%, 0 bis 10% oder 0 bis 5% der Aktivität oder Konzentration des Wildtyp-Proteins, bzw. der Aktivität oder Konzentration des Proteins im Ausgangs-Mikroorganismus, herabgesenkt.

Als Mutationen zur Erzeugung einer Abschwächung kommen Transitionen, Transversionen, Insertionen und Deletionen von mindestens einem (1) Basenpaar bzw. Nukleotid in Betracht. In Abhängigkeit von der Wirkung des durch die Mutation hervorgerufenen Aminosäureaustausches auf die Enzymaktivität wird von Fehlsinnmutationen ("missense mutations") oder Nichtsinnmutationen ("nonsense mutations") gesprochen. Die Fehlsinnmutation führt zu einem Austausch einer gegebenen Aminosäure in einem Protein gegen eine andere, wobei es sich insbesondere um einen nichtkonservativen Aminosäureaustausch handelt. Hierdurch wird die Funktionsfähigkeit bzw. Aktivität des Proteins beeinträchtigt und auf einen Wert von 0 bis 75%, 0 bis 50%, 0 bis 25%, 0 bis 10% oder 0 bis 5% reduziert. Die Nichtsinnmutation führt zu einem Stopp-Kodon im Kodierbereich des Gens und damit zu einem vorzeitigen Abbruch der Translation. Insertionen oder Deletionen von mindestens einem Basenpaar in einem Gen führen zu Rasterverschiebungsmutationen ("frame shift mutations"), die dazu führen, dass falsche Aminosäuren eingebaut werden oder die Translation vorzeitig abbricht. Entsteht als Folge der Mutation ein Stopp-Kodon im Kodierbereich, so führt dies ebenfalls zu einem vorzeitigen Abbruch der Translation. Die genannten Massnahmen werden vorzugsweise im 5'-terminalen Teil der Kodierregion durchgeführt, welcher für den N-Terminus des Polypeptids kodiert. Bezeichnet man die Gesamtlänge eines Polypeptids (gemessen als Anzahl der chemisch verbundenen L-Aminosäuren) mit 100%, so gehört - im Rahmen der vorliegenden Erfindung - zum N-Terminus des Polypeptids der Teil der Aminosäuresequenz, welcher, gerechnet ab der Start-Aminosäure L-Formyl-Methionin 80% der nachfolgenden L-Aminosäuren enthält.

Weitere Anleitungen zur Erzeugung derartiger Mutationen gehören zum Stand der Technik und können bekannten Lehrbüchern der Genetik und Molekularbiologie wie z.B. dem Lehrbuch von Knippers ("Molekulare Genetik", 6. Auflage, Georg Thieme Verlag, Stuttgart, Deutschland, 1995), dem von Winnacker ("Gene und Klone", VCH Verlagsgesellschaft, Weinheim, Deutschland, 1990) oder dem von Hagemann ("Allgemeine Genetik", Gustav Fischer Verlag, Stuttgart, 1986) entnommen werden. Weitere Maßnahmen sind im Stand der Technik beschrieben.

Ein Methode zur gezielten Verringerung der Genexpression besteht darin, das abzuschwächende Gen unter die Kontrolle eines durch Zugabe dosierter Mengen von IPTG (Isopropyl-*β-*D-thiogalactopyranosid) induzierbaren Promotors, wie zum Beispiel des trc-Promotors oder des tac-Promotors, zu stellen. Hierzu eignen sich Vektoren wie beispielsweise der Escherichia coli Expressionsvektor pXK99E (WO0226787; hinterlegt gemäß Budapester Vertrag am 31. Juli 2001 in DH5alpha/pXK99E als DSM14440 bei der Deutschen Sammlung für Mikroorganismen und Zellkulturen (DSMZ, Braunschweig, Deutschland)) oder pVWEx2 (Wendisch, Ph. D. thesis, Berichte des Forschungszentrums Jülich, Jül-3397, ISSN 0994-2952, Jülich, Deutschland (1997)), die eine IPTGabhängige Expression des klonierten Gens in Corynebacterium glutamicum ermöglichen.

Eingesetzt wurde diese Methode beispielsweise in der Patentschrift WO0226787 zur regulierten Expression des deaD-Gens durch Integration des Vektors pXK99EdeaD in das Genom von Corynebacterium glutamicum und von Simic et al. (Applied and Environmental Microbiology 68: 3321-3327 (2002)) zur regulierten Expression des glyA-Gens durch Integration des Vektors pK18mobglyA' in Corynebacterium glutamicum.

Eine weitere Methode zur spezifischen Verringerung der Genexpression ist die Antisense-Technik, wobei kurze Oligodesoxnyukleotide oder Vektoren zur Synthese längerer Antisense-RNA in die Zielzellen gebracht werden. Die Antisense-RNA kann dort an komplementäre Abschnitte spezifischer mRNAs binden und deren Stabilität verringern oder die Translatierbarkeit blocken. Ein Beispiel hierzu findet der Fachmann bei Srivastava et al. (Applied Environmental Microbiology 2000 Oct; 66 (10): 4366 - 4371).

Die durch die Massnahmen der Erfindung erhaltenen isolierten coryneformen Bakterien zeigen eine im Vergleich zum eingesetzten Ausgangsstamm bzw. Elternstamm erhöhte Ausscheidung bzw. Produktion der gewünschten Aminosäure in einem Fermentationsprozess.

Unter isolierten Bakterien sind die erfindungsgemäßen, isolierten bzw. erzeugten Mutanten und rekombinanten Bakterien, insbesonders coryneformer Bakterien, zu verstehen, welche ein rel-Allel enthalten, das für eine GTP-Pyrophosphatkinase kodiert, die den beschriebenen Aminosäureaustausch an Position 38 der Aminosäuresequenz enthält.

Die Leistung der isolierten Bakterien bzw. des Fermentationsprozesses unter Verwendung derselben bezüglich eines oder mehrerer der Parameter ausgewählt aus der Gruppe der Produkt-Konzentration (Produkt pro Volumen), der Produkt-Ausbeute (gebildetes Produkt pro verbrauchter Kohlenstoff-Quelle) und der Produkt-Bildung (gebildetes Produkt pro Volumen und Zeit) oder auch anderer Prozess-Parameter und Kombinationen davon, wird um mindestens 0,5%, mindestens 1%, mindestens 1,5% oder mindestens 2% bezogen auf den Ausgangstamm bzw. Elternstamm bzw. den Fermentationsprozess unter Verwendung derselben verbessert.

Die erfindungsgemäßen, isolierten coryneformen Bakterien können kontinuierlich - wie beispielsweise in der PCT/EP2004/008882 beschrieben- oder diskontinuierlich im batch - Verfahren (Satzkultivierung) oder im fed batch (Zulaufverfahren) oder repeated fed batch Verfahren (repetitives Zulaufverfahren) zum Zwecke der Produktion von L-Aminosäuren kultiviert werden. Eine Zusammenfassung allgemeiner Art über bekannte Kultivierungsmethoden ist im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) verfügbar.

Das zu verwendende Kulturmedium bzw. Fermentationsmedium muß in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D.C., USA, 1981) enthalten. Die Begriffe Kulturmedium, Fermentationsmedium und Nährmedium bzw. Medium sind gegenseitig austauschbar.

Als Kohlenstoffquelle können Zucker und Kohlehydrate wie z.B. Glucose, Saccharose, Laktose, Fructose, Maltose, Melasse, Saccharose-haltige Lösungen aus der Zuckerrübenoder Zuckerrohrherstellung, Stärke, Stärkehydrolysat und Cellulose, Öle und Fette, wie zum Beispiel Sojaöl, Sonnenblumenöl, Erdnußöl und Kokosfett, Fettsäuren, wie zum Beispiel Palmitinsäure, Stearinsäure und Linolsäure, Alkohole wie zum Beispiel Glycerin, Methanol und Ethanol und organische Säuren, wie zum Beispiel Essigsäure verwendet werden. Diese Stoffe können einzeln oder als Mischung verwendet werden.

Als Stickstoffquelle können organische Stickstoff-haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden.

Als Phosphorquelle können Phosphorsäure, Kaliumdihydrogenphosphat oder Dikaliumhydrogenphosphat oder die entsprechenden Natrium haltigen Salze verwendet werden.

Das Kulturmedium muß weiterhin Salze beispielsweise in Form von Chloriden oder Sulfaten von Metallen wie beispielsweise Natrium, Kalium, Magnesium, Calcium und Eisen enthalten, wie zum Beispiel Magnesiumsulfat oder Eisensulfat, die für das Wachstum notwendig sind. Schließlich können essentielle Wuchsstoffe wie Aminosäuren beispielsweise Homoserin und Vitamine beispielsweise Thiamin, Biotin oder Pantothensäure zusätzlich zu den oben genannten Stoffen eingesetzt werden. Dem Kulturmedium können überdies geeignete Vorstufen der jeweiligen Aminosäure zugesetzt werden.

Die genannten Einsatzstoffe können zur Kultur in Form eines einmaligen Ansatzes hinzugegeben oder in geeigneter Weise während der Kultivierung zugefüttert werden.

Zur pH - Kontrolle der Kultur werden basische Verbindungen wie Natriumhydroxid, Kaliumhydroxid, Ammoniak bzw. Ammoniakwasser oder saure Verbindungen wie Phosphorsäure oder Schwefelsäure in geeigneter Weise eingesetzt. Der pH wird im Allgemeinen auf einen Wert von 6,0 bis 9,0 vorzugsweise 6,5 bis 8 eingestellt. Zur Kontrolle der Schaumentwicklung können Antischaummittel, wie zum Beispiel Fettsäurepolyglykolester eingesetzt werden. Zur Aufrechterhaltung der Stabilität von Plasmiden können dem Medium geeignete selektiv wirkende Stoffe, wie zum Beispiel Antibiotika hinzugefügt werden. Um aerobe Bedingungen aufrechtzuerhalten, werden Sauerstoff oder Sauerstoffhaltige Gasmischungen, wie zum Beispiel Luft in die Kultur eingetragen. Die Verwendung von Flüssigkeiten, die mit Wasserstoffperoxid angereichert sind, ist ebenfalls möglich. Gegebenenfalls wird die Fermentation bei Überdruck, beispielsweise bei einem Druck von 0,03 bis 0,2 MPa, gefahren. Die Temperatur der Kultur liegt normalerweise bei 20°C bis 45°C und vorzugsweise bei 25°C bis 40°C. Bei batch-Verfahren wird die Kultivierung solange fortgesetzt, bis sich ein Maximum der gewünschten Aminosäure gebildet hat. Dieses Ziel wird normalerweise innerhalb von 10 Stunden bis 160 Stunden erreicht. Bei kontinuierlichen Verfahren sind längere Kultivierungszeiten möglich.

Geeignete Fermentationsmedien sind unter anderem in der US 6,221,636, in der US 5,840,551, in der US 5,770,409, in der US 5,605,818, in der US 5,275,940, in der US 4,275,157 und in der US 4,224,409 beschrieben.

Methoden zur Bestimmung von L-Aminosäuren sind aus dem Stand der Technik bekannt. Die Analyse kann zum Beispiel so wie bei Spackman et al. (Analytical Chemistry, 30, (1958), 1190) beschrieben durch Anionenaustausch-Chromatographie mit anschließender Ninhydrin-Derivatisierung erfolgen, oder sie kann durch reversed phase HPLC erfolgen, so wie bei Lindroth et al. (Analytical Chemistry (1979) 51: 1167-1174) beschrieben.

Gegenstand der Erfindung ist dementsprechend ein Verfahren zur Herstellung einer L-Aminosäure bei dem man
a) ein isoliertes coryneformes Bakterium in einem geeignetem Medium fermentiert, wobei das Bakterium ein für ein Polypeptid mit GTP-Pyrophosphatkinase Enzymaktivität kodierendes Gen enthält, wobei das Polypeptid die Aminosäuresequenz von SEQ ID NO:6 oder die Aminosäuresequenz von SEQ ID NO:6 einschließlich einer bis max. 5 Insertionen oder Deletionen enthält, wobei an Position 38 bzw. an der zu Position 38 entsprechenden Position der Aminosäuresequenz L-Leucin enthalten ist und wobei eine zu Position 38 entsprechende Position so zu verstehen ist, dass durch Insertion oder Deletion eines für eine Aminosäure kodierenden Kodons im N-terminalen Bereich bezogen auf die Position 38 von SEQ ID NO:6 des kodierten Polypeptids die Positionsangabe und Längenangabe im Falle einer Insertion formal um eine Einheit erhöht oder im Falle einer Deletion um eine Einheit verringert wird, und
b) die L-Aminosäure in der Fermentationsbrühe oder in den Zellen des isolierten coryneformen Bakteriums akkumuliert.

Daran schließt sich im Allgemeinen das Sammeln (collecting) der im Nährmedium bzw. in der Fermentationsbrühe und/oder in den Zellen der Bakterien akkumulierten L-Aminosäure an, um zu einem festen oder flüssigen Produkt zu gelangen.

Unter einer Fermentationsbrühe versteht man ein Fermentationsmedium, in dem ein Mikroorganismus für eine gewisse Zeit und bei einer gewissen Temperatur kultiviert wurde. Das Fermentationsmedium bzw. die während der Fermentation eingesetzten Medium enthält/enthalten sämtliche Substanzen bzw. Komponenten, die eine Vermehrung des Mikroorganismus und eine Bildung der gewünschten Aminosäure sicherstellt.

Bei Abschluss der Fermentation enthält die entstandene Fermentationsbrühe dementsprechend a) die infolge der Vermehrung der Zellen des Mikroorganismus entstandene Biomasse (Zellmasse) des Mikroorganismus, b) die im Laufe der Fermentation gebildete, gewünschte Aminosäure, c) die im Laufe der Fermentation gebildeten organischen Nebenprodukte und d) die durch die Fermentation nicht verbrauchten Bestandteile des/der eingesetzten Fermentationsmediums/ Fermentationsmedien bzw. der Einsatzstoffe wie beispielsweise Vitamine wie Biotin, Aminosäuren wie Homoserin oder Salze wie Magnesiumsulfat.

Zu den organischen Nebenprodukte gehören Stoffe, die von den bei der Fermentation eingesetzten Mikroorganismen gegebenenfalls neben der jeweiligen erwünschten L-Aminosäure erzeugt und gegebenenfalls ausgeschieden werden. Hierzu zählen L-Aminosäuren, die im Vergleich zur erwünschten Aminosäure weniger als 30%, 20% oder 10% ausmachen. Hierzu gehören weiterhin organische Säuren, die ein bis drei Carboxyl-Gruppen tragen wie zum Beispiel Essigsäure, Milchsäure, Zitronensäure, Apfelsäure oder Fumarsäure. Schließlich gehören dazu auch Zucker wie zum Beispiel Trehalose.

Typische für industrielle Zwecke geeignete Fermentationsbrühen haben typischerweise einen Aminosäuregehalt von 30 g/kg bis 200 g/kg oder 40 g/kg bis 175 g/kg oder 50 g/kg bis 150 g/kg. Der Gehalt an Biomasse (als getrocknete Biomasse) beträgt im Allgemeinen 20 bis 50 g/kg.

Im Falle der Aminosäure L-Lysin sind im Stand der Technik im wesentlichen vier verschiedene Produktformen bekannt.

Eine Gruppe L-Lysin haltiger Produkte umfasst konzentrierte, wässrige, alkalische Lösungen von aufgereinigtem L-Lysin (EP-B-0534865). Eine weitere Gruppe, wie beispielsweise in der US 6,340,486 und US 6,465,025 beschrieben, umfasst wässrige, saure, Biomasse-haltige Konzentrate von L-Lysin-haltigen Fermentationsbrühen. Die bekannteste Gruppe fester Produkte umfasst pulverförmige oder kristalline Formen von aufgereinigtem bzw. reinem L-Lysin, das typischerweise in Form eines Salzes wie zum Beispiel L-Lysin-Monohydrochlorid vorliegt. Eine weitere Gruppe fester Produktformen ist beispielsweise in der EP-B-0533039 beschrieben. Die dort beschriebene Produktform enthält neben L-Lysin den größten Teil der während der fermentativen Herstellung verwendeten und nicht verbrauchten Einsatzstoffe und gegebenfalls die Biomasse des eingesetzten Mikroorganismus mit einem Anteil von >0% - 100%.

Im Falle der Aminosäuren L-Valin, L-Isoleucin, L-Prolin, L-Tryptophan und L-Homoserin sind im Stand der Technik im wesentlichen Produktformen bekannt, die die betreffenden Aminosäuren in aufgereinigter bzw. reiner Form (≥ 95 Gew.-% oder ≥ 98 Gew.-%) enthalten.

Entsprechend der verschiedenen Produktformen kennt man verschiedenste Verfahren, bei denen die L-Aminosäure aus der Fermentationsbrühe gesammelt, isoliert oder gereinigt wird, um das L-Aminosäure haltige Produkt oder die gereinigte L-Aminosäure herzustellen.

Zur Herstellung von festen, reinen L-Aminosäuren werden im wesentlichen Methoden der Ionenaustauschchromatographie gegebenfalls unter Verwendung von Aktivkohle und Methoden der Kristallisierung angewendet. Im Falle des Lysin erhält man auf diese Weise die entsprechende Base oder ein entsprechendes Salz wie beispielsweise das Monohydrochlorid (Lys-HCl) oder das Lysinsulfat (Lys₂-H₂SO₄).

Im Falle des Lysin ist in der EP-B-0534865 ein Verfahren zur Herstellung wässriger, basischer L-Lysin haltiger Lösungen aus Fermentationsbrühen beschrieben. Bei dem dort beschriebenen Verfahren wird die Biomasse aus der Fermentationsbrühe abgetrennt und verworfen. Mittels einer Base wie beispielsweise Natrium-, Kalium- oder Ammoniumhydroxid wird ein pH-Wert zwischen 9 bis 11 eingestellt. Die mineralischen Bestandteile (anorganischen Salze) werden nach Konzentrierung und Abkühlung durch Kristallisation aus der Brühe abgetrennt und entweder als Dünger verwendet oder verworfen.

Bei Verfahren zur Herstellung von Lysin unter Verwendung der erfindungsgemäßen Bakterien werden auch solche Verfahren eingesetzt, bei denen Produkte erhalten werden, die Bestandteile der Fermentationsbrühe enthalten. Diese werden insbesondere als Tierfuttermitteladditive verwendet.

Je nach Anforderung kann die Biomasse ganz oder teilweise durch Separationsmethoden wie z.B. der Zentrifugation, der Filtration, dem Dekantieren oder einer Kombination hieraus aus der Fermentationsbrühe entfernt oder vollständig in ihr belassen werden. Gegebenenfalls wird die Biomasse bzw. die Biomasse enthaltene Fermentationsbrühe während eines geeigneten Verfahrensschrittes inaktiviert beispielsweise durch thermische Behandlung (Erhitzen) oder durch Säurezugabe.

Die chemischen Bestandteile der Biomasse sind unter anderem die Zellhülle, beispielsweise das Peptidoglykan und das Arabinogalaktan, das Protein bzw. Polypeptid, beispielsweise das GTP-Pyrophosphatkinase Polypeptid, Lipide und Phospholipide und Nukleinsäuren (DNA und RNA), beispielsweise Polynukleotide enthaltend die erfindungsgemäße Mutation. Als Folge der Massnahmen der Inaktivierung und/oder der weitereren Verfahrensschritte (beispielsweise Ansäuerung,Sprühtrocknung, Granulation etc.) liegen Nukleinsäuren typischerweise als Fragmente mit einer Länge von unter anderem ≥40 - 60 bp, >60 - 80 bp, >80 - 100 bp, >100 - 200 bp, >200 - 300 bp, >300 - 400 bp, >400 - 500 bp, >500 - 750 bp, >750 - 1000 bp, >1000 -1250 bp, >1250 - 1500 bp, >1500 - 1750 bp, >1750 - 2000 bp, >2000 - 2500 bp, >2500 - 3000 bp, >3000 - 4000 bp, >4000 - 5000 bp vor.

In einer Verfahrensweise wird die Biomasse vollständig oder nahezu vollständig entfernt, sodass keine (0 %) oder höchstens 30%, höchstens 20%, höchstens 10%, höchstens 5%, höchstens 1% oder höchstens 0,1% Biomasse im hergestellten Produkt verbleibt. In einer weiteren Verfahrensweise wird die Biomasse nicht oder nur in geringfügigen Anteilen entfernt, sodass sämtliche (100%) oder mehr als 70%, 80%, 90%, 95%, 99% oder 99,9% Biomasse im hergestellten Produkt verbleibt. In einem erfindungsgemäßen Verfahren wird dementsprechend die Biomasse in Anteilen ≥ 0% bis ≤ 100% entfernt.

Schließlich kann die nach der Fermentation erhaltene Fermentationsbrühe vor oder nach der vollständigen oder teilweisen Entfernung der Biomasse mit einer anorganischen Säure wie beispielsweise Salzsäure, Schwefelsäure oder Phosphorsäure oder organischen Säure wie beispielsweise Propionsäure auf einen sauren pH Wert gestellt werden (GB 1,439,728 oder EP 1 331 220). Es ist gleichfalls möglich die Fermentationsbrühe mit der vollständig enthaltenen Biomasse anzusäuern (US 6,340,486 oder US 6,465,025). Schließlich kann die Brühe auch durch Zusatz von Natriumbisulfit (NaHSO₃, GB 1,439,728) oder einem anderen Salz beispielsweise Ammonium-, Alkali- oder Erdalkalisalz der schwefligen Säure stabilisiert werden.

Bei der Abtrennung der Biomasse werden gegebenenfalls in der Fermentationsbrühe enthaltene organische oder anorganische Feststoffe teilweise oder ganz entfernt. Die in der Fermentationsbrühe gelösten organischen Nebenprodukte und die gelösten nicht verbrauchten Bestandteile des Fermentationsmediums (Einsatzstoffe) bleiben mindestens teilweise (> 0%), bevorzugt zu mindestens 25%, besonders bevorzugt zu mindestens 50% und ganz besonders bevorzugt zu mindestens 75% im Produkt. Gegebenenfalls bleiben diese auch vollständig (100%) oder nahezu vollständig das heißt > 95% oder > 98% im Produkt. In diesem Sinne bedeutet der Begriff "Fermentationsbrühebasis", dass ein Produkt mindestens einen Teil der Bestandteile der Fermentationsbrühe enthält.

Anschließend wird der Brühe mit bekannten Methoden wie z.B. mit Hilfe eines Rotationsverdampfers, Dünnschichtverdampfers, Fallfilmverdampfers, durch Umkehrosmose oder durch Nanofiltration Wasser entzogen bzw. eingedickt oder konzentriert. Diese aufkonzentrierte Fermentationsbrühe kann anschließend durch Methoden der Gefriertrocknung, der Sprühtrocknung, der Sprühgranulation oder durch anderweitige Verfahren wie zum Beispiel in der zirkulierenden Wirbelschicht gemäß PCT/EP2004/006655 beschrieben, zu rieselfähigen Produkten insbesondere zu einem feinteiligen Pulver oder vorzugsweise grobkörnigem Granulat aufgearbeitet werden. Gegebenenfalls wird aus dem erhaltenen Granulat durch Sieben oder Staubabtrennung ein gewünschtes Produkt isoliert.

Es ist ebenfalls möglich, die Fermentationsbrühe direkt d. h. ohne vorherige Aufkonzentrierung durch Sprühtrocknung oder Sprühgranulation zu trocknen.

Unter "rieselfähig" versteht man Pulver, die aus einer Serie von Glasauslaufgefäßen mit verschieden großen Auslauföffnungen mindestens aus dem Gefäß mit der Öffnung 5 mm (Millimeter) ungehindert auslaufen (Klein: Seifen, Öle, Fette, Wachse 94, 12 (1968)).

Mit "feinteilig" ist ein Pulver mit überwiegendem Anteil (> 50 %) einer Korngröße von 20 bis 200 µm Durchmesser gemeint.

Mit "grobkörnig" ist ein Produkt mit einem überwiegendem Anteil (> 50 %) einer Korngröße von 200 bis 2000 µm Durchmesser gemeint.

Die Korngrößenbestimmung kann mit Methoden der Laserbeugungsspektrometrie durchgeführt werden. Die entsprechenden Methoden sind im Lehrbuch zur "Teilchengrößenmessung in der Laborpraxis" von R. H. Müller und R. Schuhmann, Wissenschaftliche Verlagsgesellschaft Stuttgart (1996) oder im Lehrbuch "Introduction to Particle Technology" von M. Rhodes, Verlag Wiley & Sons (1998) beschrieben.

Das rieselfähige, feinteilige Pulver kann wiederum durch geeignete Kompaktier- oder Granulier-Verfahren in ein grobkörniges, gut rieselfähiges, lagerbares und weitgehend staubfreies Produkt überführt werden.

Der Begriff "staubfrei" bedeutet, daß das Produkt lediglich geringe Anteile (< 5 %) an Körngrößen unter 100 µm Durchmesser enthält.

"Lagerbar", im Sinne dieser Erfindung, bedeutet ein Produkt, das mindestens ein (1) Jahr oder länger, bevorzugt mindestens 1,5 Jahre oder länger, besonders bevorzugt zwei (2) Jahre oder länger in trockener und kühler Umgebung gelagert werden kann ohne das ein wesentlicher Verlust (< 5%) der jeweiligen Aminosäure auftritt.

Die Beschreibung offenbart dementsprechend ein Verfahren zur Herstellung eines L-Aminosäure, bevorzugt L-Lysin oder L-Tryptophan, enthaltenden Produktes, bevorzugt Tierfuttermittel-Additivs, aus Fermentationsbrühen, gekennzeichnet durch die Schritte
a) Kultivierung und Fermentation eines L-Aminosäure ausscheidenden coryneformen Bakteriums, welches mindestens ein rel-Allel enthält, das für ein Polypeptid mit GTP-Pyrophosphatkinase Aktivität kodiert, welches eine Aminosäuresequenz umfasst, bei der an Position 38 oder der vergleichbaren Position eine der proteinogenen Aminosäuren ausgenommen L-Prolin, bevorzugt L-Leucin enthalten ist, in einem Fermentationsmedium,
b) Entfernung der während der Fermentation gebildeten Biomasse in einer Menge von 0 bis 100 Gew.-%, und
c) Trocknung der gemäß a) und/oder b) erhaltenen Fermentationsbrühe, um das Produkt in der gewünschten Pulver- oder Granulatform zu erhalten
wobei gegebenenfalls vor Schritt b) oder c) eine Säure ausgewählt aus der Gruppe Schwefelsäure, Phosphorsäure oder Salzsäure hinzugefügt wird. Vorzugsweise wird im Anschluss an Schritt a) oder b) Wasser aus der L-Aminosäure haltigen Fermentationsbrühe entfernt (Aufkonzentration).

Die Beschreibung offenbart ein Verfahren zur Herstellung eines Lysinsulfat haltigen Produktes, das in seinen Grundzügen in der DE 102006016158 beschrieben ist, und bei dem die unter Verwendung der erfindungsgemäßen Mikroorganismen erhaltene Fermentationsbrühe, aus der die Biomasse gegebenenfalls ganz oder teilweise abgetrennt wurde, weiterverarbeitet wird, indem man ein Verfahren durchführt das mindestens folgende Schritte umfasst:
a) den pH-Wert durch Zugabe von Schwefelsäure auf 4,0 bis 5,2, insbesondere 4,9 bis 5,1, absenkt, und ein molares Sulfat/L-Lysin-Verhältnis von 0,85 bis 1,2, bevorzugt 0,9 bis 1,0, besonders bevorzugt >0,9 bis <0,95, in der Brühe einstellt, gegebenenfalls durch Zugabe von einer weiteren oder mehreren Sulfat-haltigen Verbindung(n) und
b) das so erhaltene Gemisch durch Wasserentzug aufkonzentriert, und gegebenenfalls granuliert, wobei gegebenenfalls vor Schritt a) eine oder beide der folgenden Massnahmen durchgeführt wird/werden:
c) Messung des molaren Verhältnisses von Sulfat/L-Lysin zur Ermittlung der benötigten Menge an Sulfat-haltigen Verbindung(n)
d) Zusatz einer Sulfat-haltigen Verbindung ausgewählt aus der Gruppe Ammoniumsulfat, Ammoniumhydrogensulfat und Schwefelsäure in entsprechenden Verhältnissen.

Gegebenenfalls wird weiterhin vor Schritt b) ein Salz der schwefligen Säure, bevorzugt Alkalihydrogensulfat, besonders bevorzugt Natriumhydrogensulft in einer Konzentration von 0,01 bis 0,5 Gew.-%, bevorzugt 0,1 bis 0,3 Gew.-%, besonders bevorzugt 0,1 bis 0,2 Gew.-% bezogen auf die Fermentationsbrühe hinzugesetzt.

Als bevorzugte Sulfat-haltigen Verbindungen im Sinne der oben genannten Verfahrensschritte sind insbesondere Ammoniumsulfat und/oder Ammoniumhydrogensulfat oder entsprechende Mischungen von Ammoniak und Schwefelsäure und Schwefelsäure selbst zu nennen.

Das molare Sulfat/L-Lysin-Verhältnis V wird nach der Formel: V = 2 x [SO₄²⁻] / [L-Lysin] berechnet. Diese Formel berücksichtigt die Tatsache, dass das SO₄²⁻ Anion zweiwertig ist. Ein Verhältnis V = 1 bedeutet, dass ein stöchiometrisch zusammengesetztes Lys₂(SO₄)vorliegt, während bei einem Verhältnis von V = 0,9 ein 10%iger Sulfatunterschuss und bei einem Verhältnis von V = 1,1 ein 10% Sulfatüberschuss gefunden wird.

Vorteilhaft bei der Granulation oder Kompaktierung ist der Einsatz von üblichen organischen oder anorganischen Hilfsstoffen, bzw. Trägern wie Stärke, Gelatine, Cellulosederivaten oder ähnlichen Stoffen, wie sie üblicherweise in der Lebensmittel- oder Futterverarbeitung als Binde-, Gelier-, oder Verdickungsmittel Verwendung finden, oder von weiteren Stoffen wie zum Beispiel Kieselsäuren, Silikaten (EP0743016A) oder Stearaten.

Weiterhin ist es vorteilhaft die Oberfläche der erhaltenen Granulate mit Ölen zu versehen so wie es in der WO 04/054381 beschrieben ist. Als Öle können Mineralöle, pflanzliche Öle oder Mischungen pflanzlicher Öle verwendet werden. Beispiele für derartige Öle sind Sojaöl, Olivenöl, Sojaöl/Lecithingemische. In gleicher Weise sind auch Silikonöle, Polyethylenglykole oder Hydroxyethycellulose geeignet. Durch die Behandlung der Oberflächen mit den genannten Ölen erzielt man eine erhöhte Abriebfestigkeit des Produktes und einen Verringerung des Staubanteils. Der Gehalt an Öl im Produkt beträgt 0,02 bis 2,0 Gew.-%, bevorzugt 0,02 bis 1,0 Gew.-%, und ganz besonders bevorzugt 0,2 bis 1,0 Gew.-% bezogen auf die Gesamtmenge des Futtermitteladditivs.

Bevorzugt sind Produkte mit einem Anteil von ≥ 97 Gew.-% einer Korngröße von 100 bis 1800 µm oder einem Anteil von ≥ 95 Gew.-% einer Korngröße von 300 bis 1800 µm Durchmesser. Der Anteil an Staub d. h. Partikeln mit einer Korngrösse < 100 µm liegt bevorzugt bei > 0 bis 1 Gew.- besonders bevorzugt bei maximal 0,5 Gew.-%.

Alternativ kann das Produkt aber auch auf einen in der Futtermittelverarbeitung bekannten und üblichen organischen oder anorganischen Trägerstoff wie zum Beispiel Kieselsäuren, Silikate, Schrote, Kleien, Mehle, Stärken Zucker oder andere aufgezogen und/oder mit üblichen Verdickungs- oder Bindemitteln vermischt und stabilisiert werden. Anwendungsbeispiele und Verfahren hierzu sind in der Literatur (Die Mühle + Mischfuttertechnik 132 (1995) 49, Seite 817) beschrieben.

Schließlich kann das Produkt auch durch Beschichtungsverfahren ("Coating") mit Filmbildnern wie beispielsweise Metallcarbonate, Kieselsäuren, Silikate, Alginate, Stearate, Stärken, Gummis und Celluloseether, wie in der DE-C-4100920 beschrieben, in einen Zustand gebracht werden, in dem es stabil gegenüber der Verdauung durch Tiermägen insbesondere dem Magen von Wiederkäuern ist.

Zur Einstellung einer gewünschten Aminosäurekonzentration im Produkt kann je nach Anforderung die entsprechende Aminosäure während des Verfahrens in Form eines Konzentrates oder gebenenfalls einer weitgehend reinen Substanz bzw. dessen Salz in flüssiger oder fester Form hinzugefügt werden. Diese können einzeln oder als Mischungen zur erhaltenen oder aufkonzentrierten Fermentationsbrühe, oder auch während des Trocknungs- oder Granulationsprozesses hinzugefügt werden.

Ein weiterer Gegenstand der Erfindung ist ein Verfahren zur Herstellung eines festen Lysin-haltigen Produktes, wie es in seinen Grundzügen in der US 20050220933 beschrieben ist, und das die Aufarbeitung der unter Verwendung der erfindungsgemäßen Mikroorganismen erhaltenen Fermentationsbrühe in folgenden Schritten umfasst:
a) Filtration der Fermentationsbrühe, bevorzugt mit einem Membranfilter, so dass ein Biomasse-haltiger Schlamm und ein Filtrat erhalten wird,
b) Aufkonzentration des Filtrates, bevorzugt so, dass ein Feststoffgehalt von 48 bis 52 Gew.-% erhalten wird,
c) Granulation des in Schritt b) erhaltenen Konzentrates, bevorzugt bei einer Temperatur von 50°C bis 62°C, und
d) Beschichtung des in c) erhaltenen Granulates mit einem oder mehreren der Beschichtungsmittel (coating agent(s)).

Zur Beschichtung in Schritt d) werden bevorzugt Beschichtungsmittel verwendet, welche ausgewählt werden aus der Gruppe, bestehend aus
d1) der in Schritt a) erhaltenen Biomasse,
d2) einer L-Lysin-haltigen Verbindung, bevorzugt ausgewählt aus der Gruppe L-Lysinhydrochlorid oder L-Lysinsulfat,
d3) einem im wesentlichen L-Lysin-freien Stoff mit L-Lysingehalt < 1 Gew.-%, bevorzugt < 0,5 Gew.-%, bevorzugt ausgewählt aus der Gruppe Stärke, Karageenan, Agar, Kieselsäuren, Silikate, Schrote, Kleien und Mehle, und
d4) einem wasserabstoßenden Stoff, bevorzugt ausgewählt aus der Gruppe Öle, Polyethylenglykole und flüssige Paraffine.

Im Falle des Lysin wird bei der Herstellung Lysin-haltiger Produkte das Verhältnis der Ionen bevorzugt so eingestellt, dass das äquivalente Ionenverhältnis entsprechend nachstehender Formel

2x [SO₄²⁻] + [Cl⁻] - [NH₄⁺] - [Na⁺] - [K⁺] -2x [Mg²⁺] -2x[Ca²⁺] / [L-Lys]

0,68 bis 0,95, bevorzugt 0,68 bis 0,90 ergibt, so wie von Kushiki et al. in der US 20030152633 beschrieben (In den "[]" sind die molaren Konzentrationen anzugeben.).

Im Falle des Lysin hat das auf diese Weise hergestellte feste Produkt auf Fermentationsbrühebasis einen Lysingehalt (als Lysinbase) von 10 Gew.-% bis 70 Gew.-% oder 20 Gew.-% bis 70 Gew.-%, bevorzugt 30 Gew.-% bis 70 Gew.-% und ganz besonders bevorzugt von 40 Gew.-% bis 70 Gew.-% bezogen auf die Trockenmasse des Produkts. Maximale Gehalte an Lysinbase von 71 Gew.-% , 72 Gew.-%, 73 Gew.-% sind ebenfalls möglich.

Im Falle einer elektrisch neutralen Aminosäure wie dem L-Tryptophan hat das auf diese Weise hergestellte feste Produkt auf Fermentationsbrühebasis einen Aminosäuregehalt von mindestens 5 Gew.-%, 10 Gew.-%, 20 Gew.-%, 30 Gew.-% und maximal 50 Gew.-%, 60 Gew.-%, 70 Gew.-%, 80 Gew.-%, 90 Gew.-% oder bis 95 Gew.-%.

Der Wassergehalt des festen Produktes beträgt bis zu 5 Gew.-%, bevorzugt bis zu 4 Gew.-%, und besonders bevorzugt weniger als 3 Gew.-%.

Die Beschreibung offenbart daher auch ein L-Lysin haltiges Futtermitteladditiv auf Fermentationsbrühebasis, welches folgende Merkmale aufweist
a) einen Lysingehalt (als Base) von mindestens 10 Gew.-% bis maximal 73 Gew.-%,
b) einen Wassergehalt von höchstens 5 Gew.-%, und
c) einen Biomassegehalt ensprechend mindestens 0,1 % der in der Fermentationsbrühe enthaltenen Biomasse, wobei die gegebenenfalls inaktivierte Biomasse aus erfindungsgemäßen coryneformen Bakterien gebildet wird.

Die Beschreibung offenbart daher auch ein L-Tryptophan haltiges Futtermitteladditiv auf Fermentationsbrühebasis, welches folgende Merkmale aufweist
a) einen Tryptophangehalt von mindestens 5 Gew.-% bis maximal 95 Gew.-%,
b) einen Wassergehalt von höchstens 5 Gew.-%, und
c) einen Biomassegehalt ensprechend mindestens 0,1 % der in der Fermentationsbrühe enthaltenen Biomasse, wobei die gegebenenfalls inaktivierte Biomasse aus erfindungsgemäßen coryneformen Bakterien gebildet wird.

Der Stamm MH20-22B wurde am 28. Oktober 2004 bei der Deutschen Sammlung für Mikroorganismen und Zellkulturen (DSMZ, Braunschweig, Deutschland) als DSM 16835 hinterlegt.

Die erfindungsgemäße Mutante Corynebacterium glutamicum DM1915, die L-Leucin an Position 38 der Aminosäuresequenz des Rel-Polypeptids enthält, wurde am 15. Mai 2006 bei der Deutschen Sammlung für Mikroorganismen und Zellkulturen (DSMZ, Braunschweig, Deutschland) als DSM 18257 hinterlegt.

Die vorliegende Erfindung wird im Folgenden anhand von Ausführungsbeispielen näher erläutert.

### Beispiel 1

### Mutagenese des L-Lysin-produzierenden Stammes DM1797

Der Corynebacterium glutamicum Stamm DM1797 wurde als Ausgangsstamm für die Mutagenese mit N-Methyl-N'-Nitro-N-Nitrosoguanidin (MNNG) eingesetzt. Der Stamm DM1797 ist eine Aminoethylcystein-resistente Mutante von Corynebacterium glutamicum ATCC13032 und unter der Bezeichnung DSM16833 bei der Deutschen Sammlung für Mikroorganismen und Zellkulturen (DSMZ, Braunschweig, Deutschland) hinterlegt.

Der Stamm DM1797 wurde in 10 ml LB-Bouillon (Merck, Darmstadt, Germany), die in einem 100 ml Erlenmeyerkolben enthalten waren, für 24 Stunden bei 33°C und 200 rpm auf einem Rotations-Schüttler vom Typ Certomat BS-1 (B. Braun Biotech International, Melsungen, Deutschland) kultiviert. Anschließend wurde die Kultur abzentrifugiert, das Sediment in 10 ml 0,9% NaCl-Lösung resuspendiert, die erhaltene Suspension erneut abzentrifugiert und das erhaltene Sediment in 10 ml 0,9% NaCl-Lösung aufgenommen. 5 ml dieser Zellsuspension wurden mit 400µg/ml MNNG für 15 Minuten bei 30°C und 200 rpm auf einem Schüttler (siehe oben) behandelt. Anschließend wurde der Mutageneseansatz abzentrifugiert und das Sediment in 10 ml 2% Na-Thiosulfat in 0,9% NaCl-Puffer (pH = 6,0) aufgenommen. Die Zellsuspension wurde anschließend im Verhältnis 1:1000, 1:10000 und 1:100000 mit 0,9% NaCl-Lösung verdünnt, und Aliquots auf Hirn-Herz-Agar (Merck, Darmstadt, Deutschland) ausplattiert. Auf diese Weise wurden ungefähr 4500 Mutanten isoliert.

### Beispiel 2

### Leistungstest der Mutanten des Stammes DM1797

Die in Beispiel 1 erhaltenen Mutanten wurden in einem zur Produktion von Lysin geeigneten Nährmedium kultiviert und der Lysingehalt im Kulturüberstand bestimmt.

Dazu wurden die Klone zunächst auf Hirn-Herz-Agarplatten (Merck, Darmstadt, Deutschland) für 24 Stunden bei 33°C vermehrt. Ausgehend von diesen Agarplattenkulturen wurde je eine Vorkultur angeimpft (10 ml Medium im 100 ml Erlenmeyerkolben). Als Medium für die Vorkultur wurde das Medium MM verwendet. Die Vorkultur wurde 24 Stunden bei 33°C und 240 rpm auf einem Schüttler inkubiert. Von dieser Vorkultur wurde eine Hauptkultur angeimpft, so dass die Anfangs-OD (660 nm) der Hauptkultur 0,1 OD betrug. Für die Hauptkultur wurde ebenfalls das Medium MM verwendet.

| Medium MM | |
|---|---|
| CSL | 5 g/l |
| MOPS | 20 g/l |
| Glucose (getrennt autoklaviert) | 50 g/l |
| Salze: | |
| (NH₄)₂SO₄) | 25 g/l |
| KH₂PO₄ | 0,1 g/l |
| MgSO₄ * 7 H₂O | 1,0 g/l |
| CaCl₂ * 2 H₂O | 10 mg/l |
| FeSO₄ * 7 H₂O | 10 mg/l |
| MnSO₄ * H₂O | 5,0mg/l |
| Biotin (sterilfiltriert) | 0,3 mg/l |
| Thiamin * HCl (sterilfiltriert) | 0,2 mg/l |
| CaCO₃ | 25 g/l |

CSL (Corn Steep Liquor), MOPS (Morpholinopropansulfonsäure) und die Salzlösung wurden mit Ammoniakwasser auf pH 7 eingestellt und autoklaviert. Anschließend wurden die sterilen Substrat- und Vitaminlösungen sowie das trocken autoklavierte CaCO₃ zugesetzt.

Die Kultivierung erfolgte in Volumina von 10 ml, die in 100 ml Erlenmeyerkolben mit Schikanen enthalten waren. Die Temperatur betrug 33°C, die Umdrehungszahl 250 rpm und die Luftfeuchte 80%.

Nach 24 Stunden wurde die optische Dichte (OD) bei einer Meßwellenlänge von 660 nm mit dem Biomek 1000 (Beckmann Instruments GmbH, München, Deutschland) bestimmt. Die gebildete Lysinmenge wurde mit einem Aminosäureanalysator der Firma Eppendorf-BioTronik (Hamburg, Deutschland) durch Ionenaustauschchromatographie und Nachsäulenderivatisierung mit Ninhydrindetektion bestimmt. Eine Mutante, die sich durch eine erhöhte Lysinbildung auszeichnete, wurde als DM1915 bezeichnet.

**Tabelle 1**

| Stamm | OD(660) | Lysin-HCl (g/l) |
|---|---|---|
| DM1797 | 9,3 | 2,2 |
| DM1915 | 9,2 | 2,4 |

### Beispiel 3

### Sequenzierung des rel-Allels der Mutante DM1915

Aus dem Klon DM1915 wurde mit der Methode von Eikmanns et al. (Microbiology 140: 1817 - 1828 (1994)) chromosomale DNA isoliert. Mit Hilfe der Polymerase-Kettenreaktion wurde ein DNA-Abschnitt amplifiziert, welcher das rel-Gen bzw. Allel trägt. Aufgrund der für C. glutamicum bekannten Sequenz des rel-Gens (Sequenz Nr. 1824 aus EP1108790) wurden folgende Primer-Oligonukleotide für die PCR ausgewählt:
rel_XL_A1 (SEQ ID NO: 19):
   5' gcgaattcta tcggatggaa catgaccg 3'
rel_XL_A2 (SEQ ID NO: 20):
   5' gcgaattcat gcggatgcca acaagatc 3'

Die dargestellten Primer wurden von der Firma MWG Biotech (Ebersberg, Deutschland) synthetisiert und nach der Standard-PCR-Methode von Innis et al. (PCR Protocols. A Guide to Methods and Applications, 1990, Academic Press) die PCR Reaktion durchgeführt. Die Primer ermöglichen die Amplifizierung eines ca. 1,53 kb langen DNA-Abschnittes, welcher das rel-Gen bzw. Allel trägt. Außerdem enthalten die Primer die Sequenz für eine Schnittstelle der Restriktionsendonuklease EcoRI, die in der oben dargestellten Nukleotidabfolge durch Unterstreichen markiert ist.

Das amplifizierte DNA-Fragment von ca. 1,53 kb Länge, welches das rel-Allel des Stammes DM1915 trägt, wurde durch Elektrophorese in einem 0,8%igen Agarosegel identifiziert, aus dem Gel isoliert und mit den üblichen Methoden aufgereinigt (QIAquick Gel Extraction Kit, Qiagen, Hilden).

Die Nukleotidsequenz des amplifizierten DNA-Fragmentes bzw. PCR-Produktes wurde von der Firma Agowa (Berlin, Deutschland) durch Sequenzierung ermittelt. Die Sequenz des PCR-Produktes ist in der SEQ ID NO: 15 dargestellt. Die Sequenz der Kodierregion ist zusätzlich in der SEQ ID NO: 5 dargestellt. Die sich mit Hilfe des Programmes Patentin ergebende Aminosäuresequenz des dazugehörigen GTP-Pyrophosphatkinase-Proteins ist in der SEQ ID NO: 6 dargestellt.

An der Position 113 der Nukleotidsequenz der Kodierregion des rel-Allels von Stamm DM1915 befindet sich die Base Thymin (SEQ ID NO: 5). An der entsprechenden Position des Wildtypgens befindet sich die Base Cytosin (SEQ ID NO: 1).

An der Position 38 der Aminosäuresequenz des GTP-Pyrophosphatkinase-Proteins von Stamm DM1915 befindet sich die Aminosäure Leucin (SEQ ID NO: 6). An der entsprechenden Position des Wildtyp-Proteins befindet sich die Aminosäure Prolin (SEQ ID NO: 2).

Das rel-Allel, welches an der Position 113 der Kodierregion die Base Thymin enthält und dementsprechend für ein GTP-Pyrophosphatkinase-Protein kodiert, welches an Position 38 der Aminosäuresequenz die Aminosäure Leucin enthält, wird im Folgenden als rel_P38L-Allel bezeichnet. Bei der Bezeichnung "rel_P38L" steht P für L-Prolin, L für L-Leucin und 38 gibt die Position des Aminosäureaustausches an (Siehe SEQ ID NO: 2 und 6).

Die Corynebacterium glutamicum Mutante DM1915, die L-Leucin an Position 38 der Aminosäuresequenz des Rel-Polypeptids enthält, wurde am 15. Mai 2006 bei der Deutschen Sammlung für Mikroorganismen und Zellkulturen (DSMZ, Braunschweig, Deutschland) als DSM 18257 hinterlegt.

### Beispiel 4

### Austausch des rel-Wildtypgens von Stamm DM1797 gegen das rel_P38L-Allel

### 4.1 Konstruktion des Austauschvektors pK18mobsacB_rel_P38L

Das in Beispiel 3 beschriebene, mittels PCR hergestellte DNA-Fragment von ca. 1,53 kb Länge, welches das rel_P38L-Allel trägt, wurde mittels Austauschmutagenese unter Zuhilfenahme des bei Schäfer et al. (Gene, 14, 69-73 (1994)) beschriebenen sacB-Systems in das Chromosom des in Beispiel 1 beschriebenen C. glutamicum Stammes DM1797 eingebaut. Dieses System ermöglicht die Herstellung bzw. die Selektion von Allel-Austauschen, die sich durch homologe Rekombination vollziehen.

Dazu wurde das ca. 1,53 kb große rel_P38L-Fragment mit der Restriktionsendonuklease EcoRI gespalten, durch Elektrophorese in einem 0,8%igen Agarosegel identifiziert und anschließend aus dem Gel isoliert und mit den üblichen Methoden aufgereinigt (QIAquick Gel Extraction Kit, Qiagen, Hilden).

Der mobilisierbare Klonierungsvektor pK18mobsacB wurde mit dem Restriktionsenzym EcoRI verdaut und die Enden mit alkalischer Phosphatase (Alkaline Phosphatase, Boehringer Mannheim, Deutschland) dephosphoryliert. Der so vorbereitete Vektor wurde mit dem ca. 1,53 kb rel_P38L-Fragment gemischt und der Ansatz mit T4-DNA-Ligase (Amersham-Pharmacia, Freiburg, Deutschland) behandelt.

Anschließend wurde der E. coli Stamm S17-1 (Simon et al., Bio/Technology 1: 784-791, 1993) mit dem Ligationsansatz transformiert (Hanahan, In. DNA Cloning. A Practical Approach. Vol. 1, ILR-Press, Cold Spring Harbor, New York, 1989). Die Selektion der Plasmid-tragenden Zellen erfolgte durch Ausplattieren des Tansformationsansatztes auf LB-Agar (Sambrook et al., Molecular Cloning: A Laboratory Manual. 2nd Ed., Cold Spring Harbor, New York, 1989), der mit 25 mg/l Kanamycin supplementiert wurde.

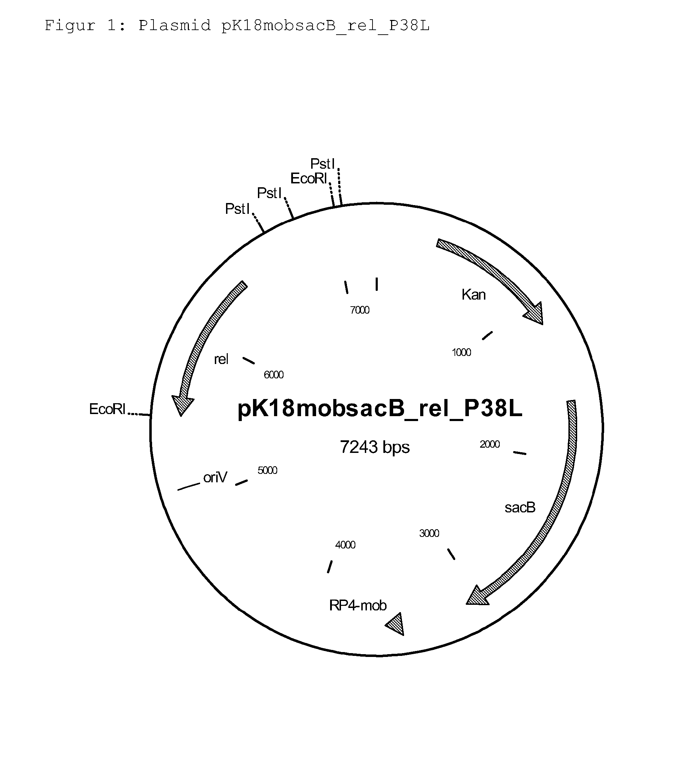

Plasmid-DNA wurde aus einer Transformante mit Hilfe des QIAprep Spin Miniprep Kit der Firma Qiagen isoliert und durch Restriktionsspaltung mit dem Enzym PstI und anschließender Agarosegel-Elektrophorese überprüft. Das Plasmid wird pK18mobsacB_rel_P38L genannt und ist in Figur 1 dargestellt.

### 4.2 Allelaustausch

Der in Beispiel 4.1 genannte Vektor pK18mobsacB_rel_P38L wurde nach einem Protokoll von Schäfer et al. (Journal of Microbiology 172: 1663-1666 (1990)) in den C. glutamicum Stamm DM1797 durch Konjugation transferiert. Der Vektor kann in DM1797 nicht selbständig replizieren und bleibt nur dann in der Zelle erhalten, wenn er als Folge eines Rekombinationsereignisses im Chromosom integriert vorliegt. Die Selektion von Transkonjuganten, d. h. von Klonen mit integriertem pK18mobsacB_rel_P38L erfolgte durch Ausplattieren des Konjugationsansatzes auf LB-Agar (Sambrook et al., Molecular Cloning: A Laboratory Manual. 2nd Ed. Cold Spring Harbor, New York, 1989), der mit 15 mg/l Kanamycin und 50 mg/l Nalidixinsäure supplementiert wurde. Kanamycin-resistente Transkonjuganten wurden auf LB-Agarplatten mit 25 mg/l Kanamycin ausgestrichen und für 24 Stunden bei 33°C inkubiert. Zur Selektion von Mutanten, bei denen als Folge eines zweiten Rekombinationsereignisses die Exzision des Plasmides stattgefunden hat, wurden die Klone 30 Stunden unselektiv in LB-Flüssigmedium kultiviert, anschließend auf LB-Agar mit 10% Sucrose ausgestrichen und 16 Stunden bebrütet.

Das Plasmid pK18mobsacB_rel_P38L enthält ebenso wie das Ausgangsplasmid pK18mobsacB neben dem Kanamycin-Resistenzgen eine Kopie des für die Levan-Sucrase aus Bacillus subtilis kodierenden sacB-Gens. Die durch Sucrose induzierbare Expression führt zur Bildung der Levan-Sucrase, die die Synthese des für C. glutamicum toxischen Produktes Levan katalysiert. Auf LB-Agar mit Sucrose wachsen daher nur solche Klone an, bei denen das integrierte pK18mobsacB_rel_P38L als Folge eines zweiten Rekombinationsereignisses exzisiert hat. In Abhängigkeit von der Lage des zweiten Rekombinationsereignisses in bezug auf den Mutationsort findet bei der Exzision der Allelaustausch bzw. der Einbau der Mutation statt oder es verbleibt die ursprüngliche Kopie im Chromosom des Wirtes.

Ungefähr 40 bis 50 Kolonien wurden auf den Phänotyp "Wachstum in Gegenwart von Sucrose" und "Nicht-Wachstum in Gegenwart von Kanamycin" geprüft. Bei 4 Kolonien, die den Phänotyp "Wachstum in Gegenwart von Sucrose" und "Nicht-Wachstum in Gegenwart von Kanamycin" aufwiesen, wurde ein die Mutation P38L überspannender Bereich des rel-Gens, ausgehend von dem Sequenzierprimer rel-2 (entspricht der Nukleotidsequenz Position 695-714 der stromaufwärts der CDS des rel-Gens gelegenen Sequenz aus SEQ ID NO: 3), von der Firma Agowa (Berlin, Deutschland) sequenziert, um nachzuweisen, dass die Mutation des rel_P38L-Allels im Chromosom vorliegt. Der verwendete Primer rel-2 wurde dazu von der Firma Agowa synthetisiert:
rel-2:
   5' ccg tca ttg tgg tca gag at 3'

Auf diese Weise wurde ein Klon identifiziert, der an der Position 113 der Kodierregion des rel-Gens die Base Thymin enthält und somit das rel_P38L-Allel besitzt. Dieser Klon wurde als Stamm DM1797rel_P38L bezeichnet.

### Beispiel 6

### Vergleich der Leistung des Stammes DM1797rel_P38L mit der des Ausgangsstammes DM1797

Der Leistungstest des in Beispiel 5 erhaltenen C. glutamicum Stammes DM1797rel_P38L wurde wie in Beispiel 2 beschrieben durchgeführt. Das Ergebnis des Versuchs ist in Tabelle 2 dargestellt.

**Tabelle 2**

| Stamm | OD (660 nm) | Lysin-HCl g/l |
|---|---|---|
| DM1797 | 9,3 | 2,2 |
| DM1797rel_P38L | 9,2 | 2,5 |

Kurze Beschreibung der Figur:
Figur 1: Karte des Plasmids pK18mobsacB_rel_P38L.

Die verwendeten Abkürzungen und Bezeichnungen haben folgende Bedeutung. Bei der Angabe der Basenpaarzahlen handelt es sich um Näherungswerte, die im Rahmen der Reproduzierbarkeit von Messungen erhalten werden.

| | |
|---|---|
| Kan: | Kanamycin Resistenz-Gen |
| EcoRI: | Schnittstelle des Restriktionsenzyms EcoRI |
| PstI: | Schnittstelle des Restriktionsenzyms PstI |
| rel: | rel_P38L-Allel |
| sacB: | sacB-Gen |
| RP4-mob: | mob-Region mit dem Replikationsursprung für den Transfer (oriT) |
| oriV: | Replikationsursprung V |

## Patentansprüche

1. Isolierte Mutanten coryneformer Bakterien, welche ein Gen enthalten, das für ein Polypeptid mit GTP-Pyrophosphatkinase Aktivität kodiert, **dadurch gekennzeichnet, dass** das Polypeptid eine Aminosäuresequenz umfasst, ausgewählt aus der Gruppe
a) Aminosäuresequenz gemäß SEQ ID NO:6, und
b) Aminosäuresequenz gemäß SEQ ID NO:6 einschließlich einer oder mehrerer bis max. 5 Insertionen oder Deletionen von Aminosäuren, welche an Position 38 bzw. an der zu Position 38 entsprechenden Position der Aminosäuresequenz L-Leucin enthält, wobei eine zu Position 38 entsprechende Position so zu verstehen ist, dass durch Insertion oder Deletion eines für eine Aminosäure kodierenden Kodons im N-terminalen Bereich bezogen auf die Position 38 von SEQ ID NO:6 des kodierten Polypeptids die Positionsangabe und Längenangabe im Falle einer Insertion formal um eine Einheit erhöht oder im Falle einer Deletion um eine Einheit verringert wird.
wobei gegebenenfalls an Position 262 L-Glutaminsäure enthalten ist.

2. Mutanten coryneformer Bakterien gemäß Anspruch 1, **dadurch gekennzeichnet, dass** es sich bei den coryneformen Bakterien um ein Bakterium ausgewählt aus der Gruppe Corynebacterium efficiens, Corynebacterium glutamicum, Corynebacterium thermoaminogenes und Corynebacterium aminogenes handelt.

3. Mutanten coryneformer Bakterien gemäß Anspruch 1 oder 2, **dadurch gekennzeichnet, dass** es sich um L-Aminosäure ausscheidende Bakterien bevorzugt um L-Lysin, L-Valin, L-Isoleucin, L-Tryptophan oder L-Homoserin ausscheidende Bakterien handelt.

4. Mutanten gemäß einem der Ansprüche 1 bis 3, **dadurch gekennzeichnet, dass** das Gen die Nukleotidsequenz von SEQ ID NO:5 oder SEQ ID NO:7 umfasst, wobei gegebenenfalls das Guanin an Position 785 von SEQ ID NO:5 oder das Guanin an Position 1535 von SEQ ID NO:7 durch Adenin ersetzt ist.

5. Isoliertes Polynukleotid, **dadurch gekennzeichnet, dass** das kodierte Protein eine Aminosäuresequenz ausgewählt aus der Gruppe
a) Aminosäuresequenz gemäß SEQ ID NO:6, und
b) Aminosäuresequenz gemäß SEQ ID NO:6, einschließlich einer oder mehrerer bis max. 5 Insertionen oder Deletionen von Aminosäuren, welche an Position 38 bzw. an der zu Position 38 entsprechenden Position der Aminosäuresequenz L-Leucin enthält, wobei eine zu Position 38 entsprechende Position so zu verstehen ist, dass durch Insertion oder Deletion eines für eine Aminosäure kodierenden Kodons im N-terminalen Bereich bezogen auf die Position 38 von SEQ ID NO:6 des kodierten Polypeptids die Positionsangabe und Längenangabe im Falle einer Insertion formal um eine Einheit erhöht oder im Falle einer Deletion um eine Einheit verringert wird.
umfasst, wobei gegebenenfalls an Position 262 L-Glutaminsäure enthalten ist.

6. Isoliertes Polynukleotid, gemäß Anspruch 5, **dadurch gekennzeichnet, dass** seine Nukleotidsequenz SEQ ID NO:5 umfasst, wobei gegebenenfalls an Position 785 Adenin enthalten ist.

7. Ein rekombinanter Mikroorganismus, der das isolierte Polynukleotid gemäß Anspruch 5 bis 6 enthält, bevorzugt
es sich um ein coryneformes Bakterium oder um ein Bakterium der Gattung Escherichia handelt,
besonders bevorzugt um die Gattung Corynebacterium,
ganz besonders bevorzugt um die Art Corynebacterium glutamicum.

8. Ein Vektor, der das isolierte Polynukleotid gemäß Anspruch 5 bis 6 enthält.

9. Ein rekombinanter Mikroorganismus, der den Vektor gemäß Anspruch 8 enthält, vorzugsweise
ein rekombinanter Mikroorganismus bei dem es sich um ein coryneformes Bakterium oder um ein Bakterium der Gattung Escherichia handelt,
besonders bevorzugt um die Gattung Corynebacterium,
ganz besonders bevorzugt um die Art Corynebacterium glutamicum.

10. Verfahren zur Herstellung einer L-Aminosäure **dadurch gekennzeichnet, dass** man
a) ein isoliertes coryneformes Bakterium in einem geeignetem Medium fermentiert, wobei das Bakterium mindestens eine Kopie ein für ein Polypeptid mit GTP-Pyrophosphatkinase-Enzymaktivität kodierendes Gen enthält, wobei das Polypeptid die Aminosäuresequenz von SEQ ID NO:6 oder die Aminosäuresequenz von SEQ ID NO:6 einschließlich einer bis max. 5 Insertionen oder Deletionen enthält, wobei an Position 38 bzw. an der zu Position 38 entsprechenden Position der Aminosäuresequenz L-Leucin enthalten ist und wobei eine zu Position 38 entsprechende Position so zu verstehen ist, dass durch Insertion oder Deletion eines für eine Aminosäure kodierenden Kodons im N-terminalen Bereich bezogen auf die Position 38 von SEQ ID NO:6 des kodierten Polypeptids die Positionsangabe und Längenangabe im Falle einer Insertion formal um eine Einheit erhöht oder im Falle einer Deletion um eine Einheit verringert wird, und
b) die L-Aminosäure in der Fermentationsbrühe oder in den Zellen des Bakteriums anreichert.

11. Verfahren gemäß Anspruch 10, **dadurch gekennzeichnet, dass** es sich bei dem isolierten coryneformen Bakterium um eine Mutante gemäß Anspruch 1 bis 4 handelt, oder um ein rekombinantes coryneformes Bakterium, das ein isoliertes Polynukleotid gemäß Anspruch 5 bis 6 enthält oder unter Verwendung desselben hergestellt wurde.

12. Verfahren nach Anspruch 10 oder 11, **dadurch gekennzeichnet, dass** man die L-Aminosäure isoliert oder sammelt, und vorzugsweise reinigt oder,
dass man die L-Aminosäure gemeinsam mit Bestandteilen aus der Fermentationsbrühe und/oder der Biomasse (> 0 bis 100 %) isoliert oder sammelt.

13. Verfahren nach einem der Ansprüche 10 bis 12, **dadurch gekennzeichnet, dass** man
a) aus der in Schritt b) von Anspruch 12 erhaltenen Fermentationsbrühe die gebildete Biomasse in einer Menge von 0 bis 100 % entfernt, und
b) aus der in Schritt a) erhaltenen Brühe ein im wesentlichen trockenes und geformtes Produkt durch eine Methode ausgewählt aus der Gruppe Granulation, Kompaktierung, Sprühtrocknung und Extrusion herstellt.

14. Verfahren nach Anspruch 13, **dadurch gekennzeichnet, dass** man im Fall der Aminosäure L-Lysin der Fermentationsbrühe vor oder nach Schritt a) eine Säure ausgewählt aus der Gruppe Schwefelsäure, Salzsäure und Phosphorsäure hinzufügt oder,
dass man aus der vor oder nach in Schritt a) erhaltenen Brühe Wasser entfernt und/oder dass man das in oder während Schritt b) erhaltene geformte Produkt mit einem Öl besprüht oder,
dass man folgende Schritte durchführt
a) Filtration der Fermentationsbrühe, bevorzugt mit einem Membranfilter, so dass ein Biomassehaltiger Schlamm und ein Filtrat erhalten wird,
b) Aufkonzentration des Filtrates, bevorzugt so, dass ein Feststoffgehalt von 48 bis 52 Gew.-% erhalten wird,
c) Granulation des in Schritt b) erhaltenen Konzentrates, bevorzugt bei einer Temperatur von 50°C bis 62°C, und
d) Beschichtung des in c) erhaltenen Granulates mit einem oder mehreren der Beschichtungsmittel (coating agent(s)).

## Claims

1. Isolated mutants of coryneform bacteria, which comprise a gene coding for a polypeptide having GTP-pyrophosphate kinase activity, **characterized in that** said polypeptide comprises an amino acid sequence selected from the group consisting of
a) amino acid sequence according to SEQ ID NO: 6, and
b) amino acid sequence according to SEQ ID NO: 6 including one or more, to a maximum of 5, insertions or deletions of amino acids, which comprises L-leucine in position 38 or in the position of the amino acid sequence that corresponds to position 38, wherein a position that corresponds to position 38 is to be understood as meaning that insertion or deletion of a codon coding for an amino acid in the N-terminal region based on position 38 of SEQ ID NO: 6 of the encoded polypeptide formally increases, in the case of an insertion, or decreases, in the case of a deletion, the indicated position and indicated length, in each case by one unit,
with L-glutamic acid optionally being present in position 262.

2. Mutants of coryneform bacteria according to Claim 1, **characterized in that** the coryneform bacteria are a bacterium selected from the group consisting of Corynebacterium efficiens, Corynebacterium glutamicum, Corynebacterium thermoaminogenes and Corynebacterium aminogenes.

3. Mutants of coryneform bacteria according to Claim 1 or 2, **characterized in that** they are L-amino acid-secreting bacteria, preferably L-lysine-, L-valine-, L-isoleucine-, L-tryptophan- or L-homoserine-secreting bacteria.

4. Mutants according to one of Claims 1 to 3, **characterized in that** the gene comprises the nucleotide sequence of SEQ ID NO:5 or SEQ ID NO:7, the guanine in position 785 of SEQ ID NO:5 or the guanine in position 1535 of SEQ ID NO:7 optionally being replaced by adenine.

5. Isolated polynucleotide, **characterized in that** the encoded protein comprises an amino acid sequence selected from the group consisting of
a) an amino acid sequence according to SEQ ID NO:6, and
b) an amino acid sequence according to SEQ ID NO:6, including one or more, to a maximum of 5, amino acid insertions or deletions, which comprises L-leucine in position 38 or in the position of the amino acid sequence that corresponds to position 38, wherein a position that corresponds to position 38 is to be understood as meaning that insertion or deletion of a codon coding for an amino acid in the N-terminal region based on position 38 of SEQ ID NO:6 of the encoded polypeptide formally increases, in the case of an insertion, or decreases, in the case of a deletion, the indicated position and indicated length, in each case by one unit,
L-glutamic acid being optionally present in position 262.

6. Isolated polynucleotide according to Claim 5, **characterized in that** the nucleotide sequence thereof comprises SEQ ID NO:5, adenine optionally being present in position 785.

7. Recombinant microorganism comprising the isolated polynucleotide according to Claim 5 or 6,
which is preferably a coryneform bacterium or a bacterium of the genus Escherichia,
particularly preferably the genus Corynebacterium, very particularly preferably the species Corynebacterium glutamicum.

8. Vector comprising the isolated polynucleotide according to Claim 5 or 6.

9. Recombinant microorganism comprising the vector according to Claim 8,
preferably a recombinant microorganism which is a coryneform bacterium or a bacterium of the genus Escherichia,
particularly preferably the genus Corynebacterium, very particularly preferably the species Corynebacterium glutamicum.

10. Process for preparing an L-amino acid, which comprises
a) fermenting an isolated coryneform bacterium in a suitable medium, said bacterium harboring at least one copy of a gene encoding a polypeptide having GTP-pyrophosphate kinase enzyme activity, wherein the polypeptide comprises the amino acid sequence according to SEQ ID NO: 6, or the amino acid sequence according to SEQ ID NO: 6 including one to a maximum of 5 insertions or deletions, with L-leucine being present in position 38 or in the position of the amino acid sequence that corresponds to position 38, and wherein a position that corresponds to position 38 is to be understood as meaning that insertion or deletion of a codon coding for an amino acid in the N-terminal region based on position 38 of SEQ ID NO:6 of the encoded polypeptide formally increases, in the case of an insertion, or decreases, in the case of a deletion, the indicated position and indicated length, in each case by one unit, and
b) concentrating the L-amino acid in the fermentation broth or in the cells of said bacterium.

11. Process according to Claim 10, **characterized in that** the isolated coryneform bacterium is a mutant according to Claims 1 to 4, or a recombinant coryneform bacterium which harbors an isolated polynucleotide according to Claim 5 or 6 or which has been prepared using the latter.

12. Process according to Claim 10 or 11, **characterized in that** the L-amino acid is isolated or collected and preferably purified or
**in that** the L-amino acid is isolated or collected together with components of the fermentation broth and/or the biomass (> 0 to 100%).

13. Process according to one of Claims 10 to 12, **characterized in that** it comprises
a) removing an amount of from 0 to 100% of the biomass produced from the fermentation broth obtained in step b) of Claim 12, and
b) preparing from the broth obtained in step a) an essentially dry and shaped product by a method selected from the group consisting of granulation, compacting, spray drying and extrusion.

14. Process according to Claim 13, **characterized in that**, in the case of the amino acid L-lysine, an acid selected from the group consisting of sulfuric acid, hydrochloric acid and phosphoric acid is added to the fermentation broth prior to or after step a) or
**in that** water is removed from the broth obtained prior to or after step a) and/or **in that** the shaped product obtained in or during step b) is sprayed with an oil or
**in that** the following steps are carried out
a) filtering the fermentation broth, preferably by using a membrane filter, so as to obtain a biomass-containing sludge and a filtrate,
b) concentrating said filtrate, preferably so as to obtain a solids content of from 48 to 52% by weight,
c) granulating the concentrate obtained in step b), preferably at a temperature of from 50°C to 62°C, and
d) coating the granules obtained in c) with one or more of the coating agents.

## Revendications

1. Mutants isolés de bactéries corynéformes, qui contiennent un gène qui code pour un polypeptide ayant une activité de GTP-pyrophosphate kinase, **caractérisés en ce que** le polypeptide comprend une séquence d'acides aminés choisie dans le groupe consistant en
a) la séquence d'acides aminés selon SEQ ID NO:6, et
b) la séquence d'acides aminés selon SEQ ID NO:6, y compris une ou plusieurs, jusqu'à un maximum de 5 insertions ou délétions d'acides aminés, qui en position 38, ou sur la position de la séquence d'acides aminés correspondant à la position 38, contient une L-leucine, une position correspondant à la position 38 devant être comprise par le fait que, sous l'effet d'une insertion ou d'une délétion d'un codon codant pour un acide aminé dans le domaine N-terminal, l'indication de position et l'indication de longueur subissent, par rapport à la position 38 de la séquence SEQ ID NO:6 du peptide codant, une augmentation formelle d'une unité dans le cas d'une insertion, ou une diminution d'une unité dans le cas d'une délétion,
un acide L-glutamique étant éventuellement présent en position 262.

2. Mutants de bactéries corynéformes selon la revendication 1, **caractérisés en ce que**, pour ce qui concerne les bactéries corynéformes, il s'agit d'une bactérie choisie dans le groupe consistant en Corynebacterium efficiens, Corynebacterium glutamicum, Corynebacterium thermoaminogenes et Corynebacterium aminogenes.

3. Mutants de bactéries corynéformes selon la revendication 1 ou 2, **caractérisés en ce qu'**il s'agit de bactéries sécrétant un acide L-aminé, de préférence de bactéries sécrétant la L-lysine, la L-valine, la L-isoleucine, le L-tryptophane ou la L-homosérine.

4. Mutants selon l'une des revendications 1 à 3, **caractérisés en ce que** le gène comprend la séquence nucléotidique SEQ ID NO:5 ou SEQ ID NO:7, la guanine en position 785 de SEQ ID NO:5 ou la guanine en position 1535 de SEQ ID NO:7 étant éventuellement remplacée par une adénine.

5. Polynucléotide isolé, **caractérisé en ce que** la protéine codée comprend une séquence d'acides aminés choisie dans le groupe consistant en
a) la séquence d'acides aminés selon SEQ ID NO:6, et
b) la séquence d'acides aminés selon SEQ ID NO:6, y compris une ou plusieurs, jusqu'à un maximum de 5 insertions ou délétions d'acides aminés, qui en position 38, ou sur la position de la séquence d'acides aminés correspondant à la position 38, contient une L-leucine, une position correspondant à la position 38 devant être comprise par le fait que, sous l'effet d'une insertion ou d'une délétion d'un codon codant pour un acide aminé dans le domaine N-terminal, l'indication de position et l'indication de longueur subissent, par rapport à la position 38 de la séquence SEQ ID NO:6 du peptide codant, une augmentation formelle d'une unité dans le cas d'une insertion, ou une diminution d'une unité dans le cas d'une délétion,
un acide L-glutamique étant éventuellement présent en position 262.

6. Polynucléotide isolé selon la revendication 5, **caractérisé en ce que** sa séquence nucléotidique comprend SEQ ID NO:5, une adénine étant éventuellement présente en position 785.

7. Microorganisme recombinant qui contient le polynucléotide isolé selon les revendications 5 à 6, pour ce qui concerne lequel il s'agit de préférence d'une bactérie corynéforme ou d'une bactérie du genre Escherichia, d'une manière particulièrement préférée du genre Corynebacterium, d'une manière tout particulièrement préférée de l'espèce Corynebacterium glutamicum.

8. Vecteur qui contient le polynucléotide isolé selon les revendications 5 à 6.

9. Microorganisme recombinant qui contient le vecteur selon la revendication 8,
de préférence microorganisme recombinant pour ce qui concerne lequel il s'agit d'une bactérie corynéforme ou d'une bactérie du genre Escherichia,
d'une manière particulièrement préférée du genre Corynebacterium,
d'une manière tout particulièrement préférée de l'espèce Corynebacterium glutamicum.

10. Procédé de préparation d'un acide L-aminé, **caractérisé en ce que**
a) on fermente une bactérie corynéforme isolée dans un milieu approprié, la bactérie contenant au moins une copie d'un gène codant pour un polypeptide ayant l'activité de l'enzyme GTP-pyrophosphate kinase, le polypeptide contenant la séquence d'acides aminés SEQ ID NO:6 ou la séquence d'acides aminés SEQ ID NO:6 y compris une à un maximum de 5 insertions ou délétions, et une L-leucine étant présente en position 38 ou sur la position de la séquence d'acides aminés correspondant à la position 38, une position correspondant à la position 38 devant être comprise par le fait que, sous l'effet d'une insertion ou d'une délétion d'un codon codant pour un acide aminé dans le domaine N-terminal, l'indication de position et l'indication de longueur subissent, par rapport à la position 38 de la séquence SEQ ID NO:6 du peptide codant, une augmentation formelle d'une unité dans le cas d'une insertion, ou une diminution d'une unité dans le cas d'une délétion, et
b) l'acide L-aminé s'enrichit dans le bouillon de fermentation ou dans des cellules de la bactérie.

11. Procédé selon la revendication 10, **caractérisé en ce que**, pour ce qui concerne la bactérie corynéforme isolée, il s'agit d'un mutant selon les revendications 1 à 4, ou d'une bactérie qui contient un polynucléotide isolé selon les revendications 5 à 6 ou qui a été fabriquée par utilisation de ce dernier.

12. Procédé selon la revendication 10 ou 11, **caractérisé en ce qu'**on isole ou on recueille l'acide L-aminé et de préférence on le purifie, ou
**en ce qu'**on isole ou recueille l'acide L-aminé en même temps que des constituants du bouillon de fermentation et/ou de la biomasse (> 0 à 100 %).

13. Procédé selon l'une des revendications 10 à 12, **caractérisé en ce que**
a) on élimine du bouillon de fermentation obtenu dans l'étape b) de la revendication 12 la biomasse formée, en une quantité de 0 à 100 %, et
b) à partir du bouillon obtenu dans l'étape a), on fabrique un produit pour l'essentiel sec et façonné, par une méthode choisie dans le groupe consistant en une granulation, un compactage, un séchage par atomisation et une extrusion.

14. Procédé selon la revendication 13, **caractérisé en ce que**, dans le cas de l'acide aminé L-lysine, on ajoute au bouillon de fermentation, avant ou après l'étape a), un acide choisi dans le groupe consistant en l'acide sulfurique, l'acide chlorhydrique et l'acide phosphorique, ou
**en ce qu'**on élimine l'eau du bouillon obtenu avant ou après l'étape a) et/ou **en ce qu'**on pulvérise une huile sur le produit façonné obtenu dans ou pendant l'étape b),
**en ce qu'**on met en oeuvre les étapes suivante
a) filtration du bouillon de fermentation, de préférence avec une membrane filtrante, de façon à obtenir une boue contenant une biomasse, et un filtrat,
b) concentration du filtrat, de préférence de façon à réaliser une teneur en extrait sec de 48 à 52 % en poids,
c) granulation du concentré obtenu dans l'étape b), de préférence à une température de 50°C à 62°C, et
d) application, sur le granulat obtenu en c), d'un ou plusieurs agents de revêtement (coating agent(s)).
